# EUROPEAN PATENT SPECIFICATION

(11) **EP 4 178 580 B1**
(45) Date of publication and mention of the grant of the patent: **05.11.2025**
(21) Application number: 21836755.5
(22) Date of filing: 08.07.2021
(51) Int. Cl.: A61K 31/5025, C07D 487/04, A61P 35/00, A61P 29/00

(54) **BF 844 FOR USE IN THE TREATMENT OF CANCER**
BF 844 ZUR VERWENDUNG BEI DER BEHANDLUNG VON KREBS
BF 844 POUR L'UTILISATION DANS LE TRAITEMENT DU CANCER

(30) Priority: 09.07.2020 US 202063049948 P
(43) Date of publication of application: 17.05.2023
(73) Proprietor: Usher III Initiative, Inc., Chicago, Illinois 60606 (US)
(72) Inventor: FARHAN, Mahdi, Chicago, Illinois 60606 (US); HAMBY, James M., Chicago, Illinois 60606 (US); FLETCHER, Tracey L., Chicago, Illinois 60606 (US)
(74) Representative: Cooley (UK) LLP
(86) International application number: PCT/US2021/040810
(87) International publication number: WO 2022/011091

(56) References cited:
- WO-A1-03/080616
- WO-A1-2017/098367
- US-A1- 2017 143 718
- US-A1- 2018 099 974
- US-A1- 2018 099 974

## Description

### CROSS-REFERENCE TO RELATED APPLICATIONS

This application claims the benefit of U.S. Provisional Application No. 63/049,948, filed July 9, 2020.

### BACKGROUND OF THE INVENTION

Cancer is one of the leading causes of death worldwide. In the United States alone, it is estimated that in 2020, more than 1.8 million new cancer cases will be diagnosed and more than 600,000 lives will be lost due to cancer. Cancer affects a large portion of the population - about 40% of people in the US will develop cancer in their lifetime. *See* "Cancer Costs and Figures" by the American Cancer Society. Inflammatory diseases and autoimmune diseases also afflict millions of people worldwide and remain a significant threat to people's health. While substantial progress in the treatment of these diseases have been made in recent years, there are still needs for identifying new methods for treating, preventing or reducing risk of developing cancer, inflammatory disease or autoimmune disease. US 2018/099974 A1 (SAPERSTEIN DAV) discloses the compound of the present invention used in a method of treating proteopathy, medullary thyroid carcinoma and cancer. However, this document makes no reference to BRCA nor to colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

### SUMMARY OF THE INVENTION

The present invention relates to a compound having the structure: 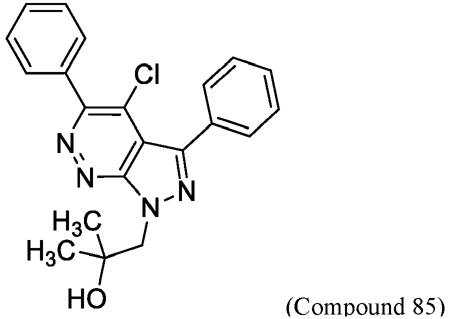
or a pharmaceutically acceptable salt thereof, for use in a method of treating cancer, preventing cancer, or reducing risk of developing cancer,
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

In some embodiments, the cancer has one or more disease-associated mutations in BRCA1 or BRCA2. In other embodiments, the cancer has one or more disease-associated mutations in BRCA1 but harbors no disease-associated mutations in BRCA2.

In some embodiments, the method further comprises administering an anti-cancer therapy to the subject. In some such embodiments, the anti-cancer therapy is neoadjuvant therapy or an adjuvant therapy.

In some embodiments, the anti-cancer therapy is chemotherapy, targeted therapy, hormone therapy, immunotherapy, T-cell therapy, or stem cell therapy.

In some embodiments, the chemotherapy is an alkylating agent, an antimetabolite, an anthracycline antibiotic, a non-anthracycline antibiotic, a topoisomerase inhibitor, a mitotic inhibitor, or a corticosteroid. In some embodiments, the immunotherapy is an anti-PD-L1 antibody, an anti-PD-1 antibody, or an anti-CTLA4 antibody.

Also disclosed herein are compounds of Formula I, II, III, IV, V, VI, VII, XIII, XIV or XV; Compounds 1-35, 37-39, 42, 43, 44, 45, 46, 47-84, 86-97, 98-123, 124a, 124b, 125-213, Va-Vz, Vaa-Vii, VIa-VIy, VIIa-VIId, XIIIa-XIIIz, XIVa or XVa-XVm; or a pharmaceutically acceptable salt of any of the foregoing. Each of these compounds are referred to herein as a "compound of the disclosure".

Also disclosed herein are methods for treating cancer, preventing cancer, or reducing risk of developing cancer, comprising administering to a subject in need thereof an effective amount of a compound of Formula II: 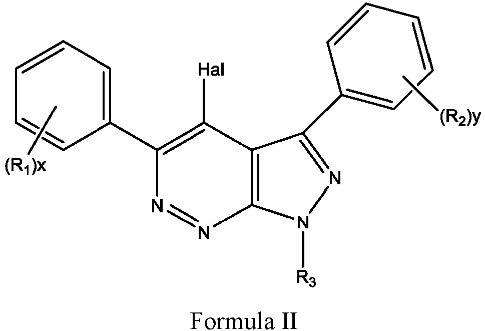 or a pharmaceutically acceptable salt thereof, wherein
Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 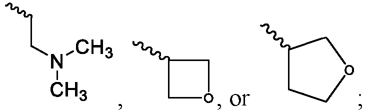
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 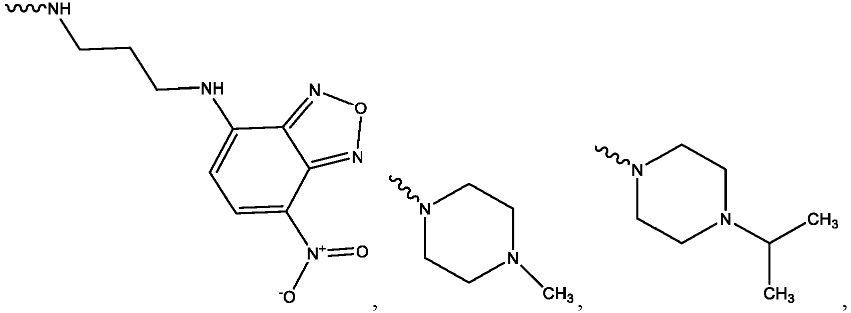 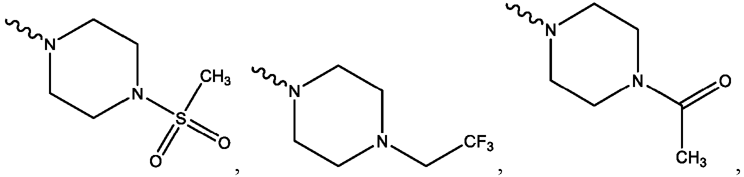 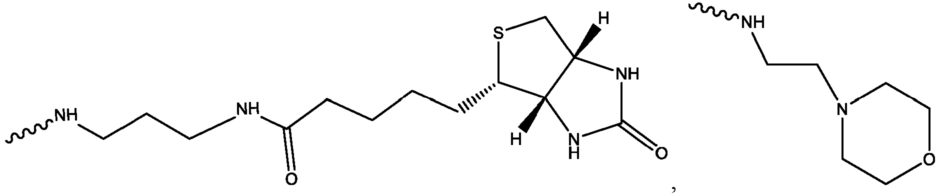 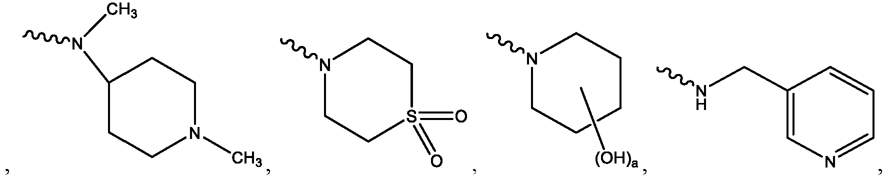 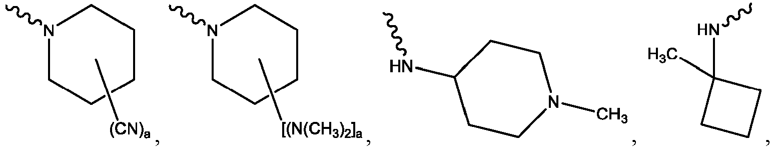 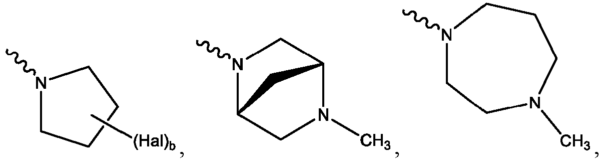 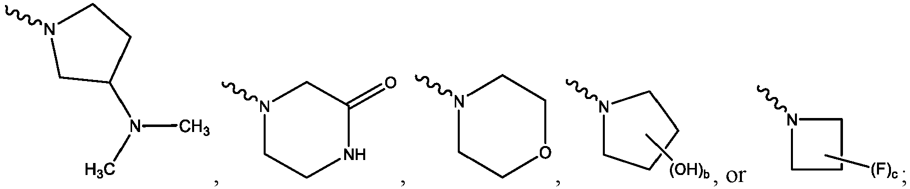
a is an integer ranging from 0 to 10;
b is an integer ranging from 0 to 8;
c is an integer ranging from 0 to 6; and
R₅ is 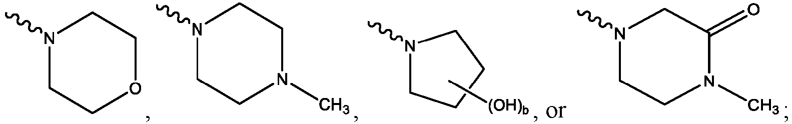
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula III: 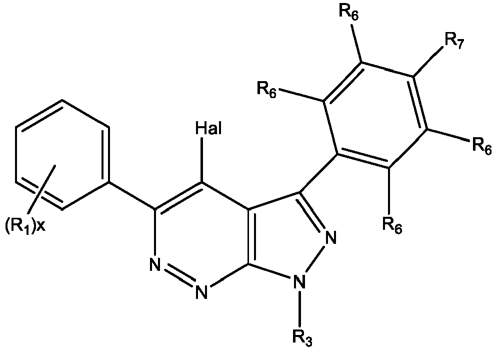 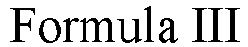 or a pharmaceutically acceptable salt thereof, wherein:
Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 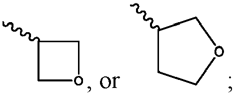
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 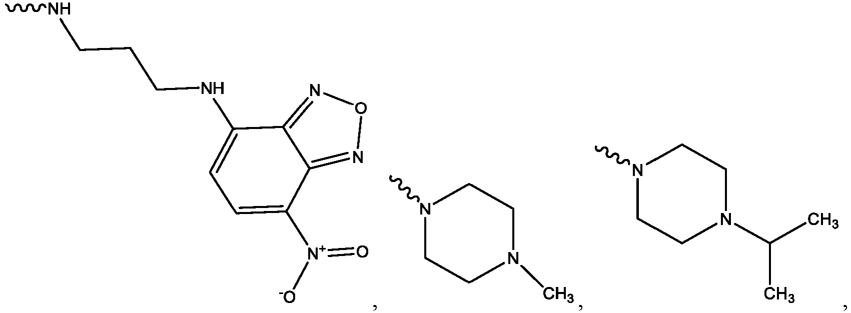 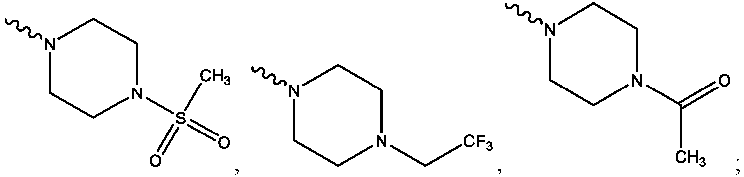 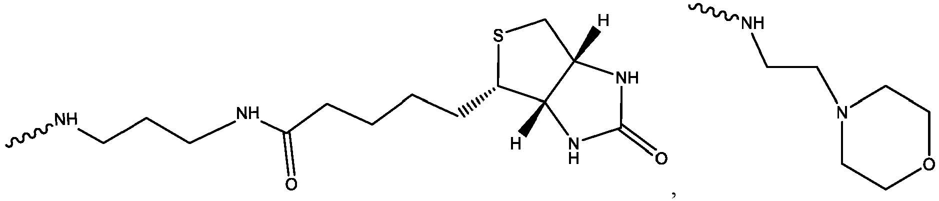 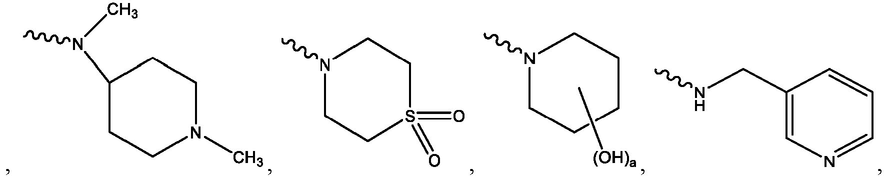 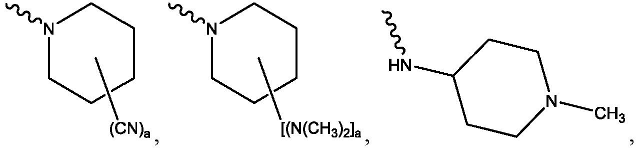 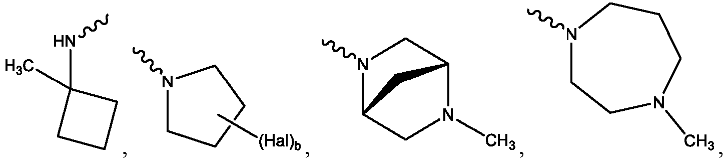 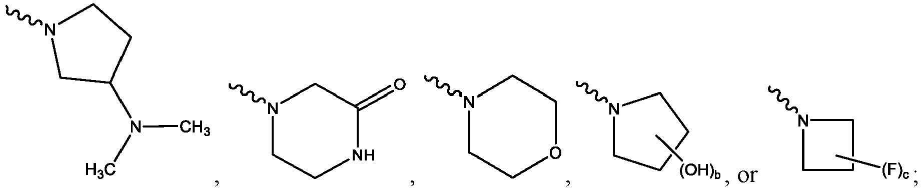
a is an integer ranging from 0 to 10;
b is an integer ranging from 0 to 8;
c is an integer ranging from 0 to 6;
R₅ is 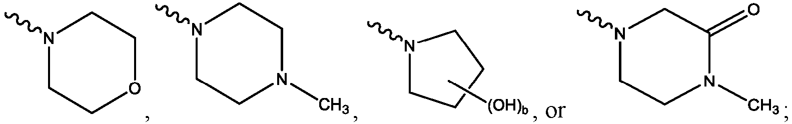 and
each R₆ and R₇ is independently -H or -I, wherein at least one of R₆ and R₇ is -I, and wherein when R₃ is -C₁-C₃ alkyl, then R₇ is -H;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of Compound 46, or a pharmaceutically acceptable salt thereof;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula IV: 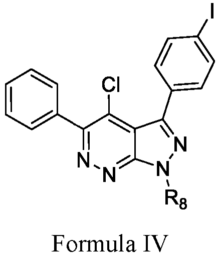 or a pharmaceutically acceptable salt thereof;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula V: 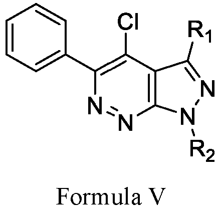 or a pharmaceutically acceptable salt thereof, wherein:
R₁ is: 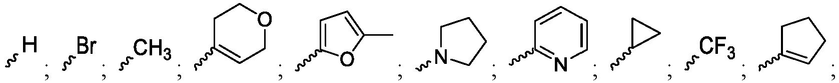 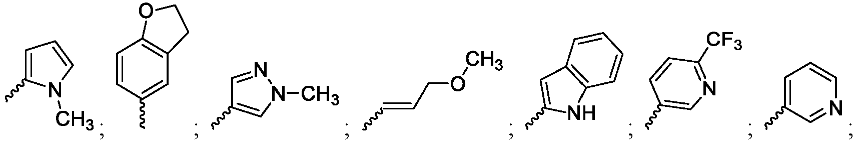 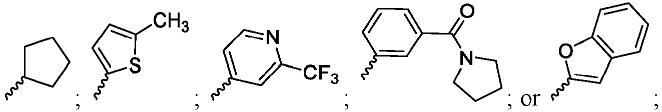
R₂ is: 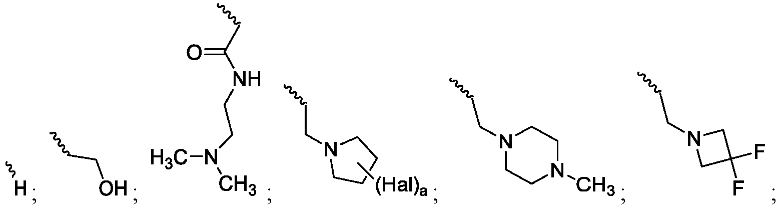 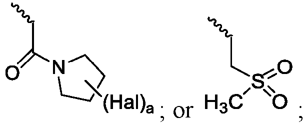
Hal is -Cl, -F, -I, or -Br; and
a is 0, 1, or 2;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula VI: 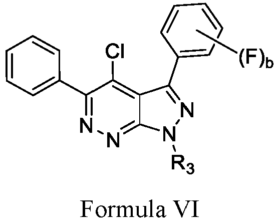 or a pharmaceutically acceptable salt thereof, wherein:
R₃ is: 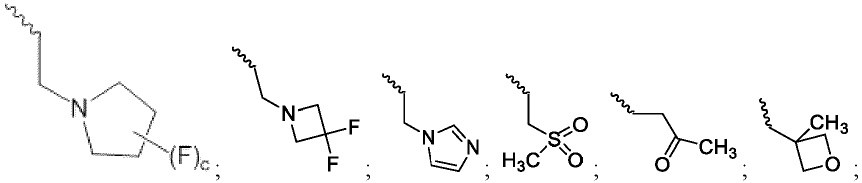 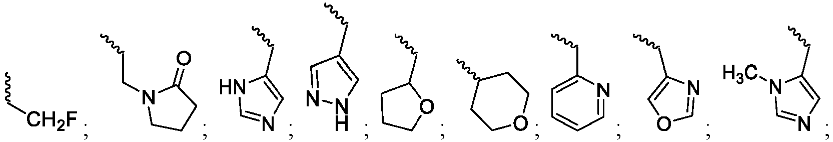 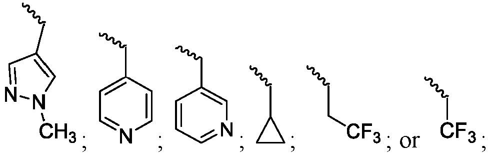
b is 0 or 1; and
c is 1 or 2;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula VII: 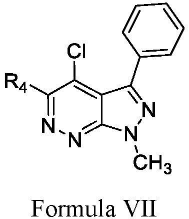 or a pharmaceutically acceptable salt thereof, wherein:
R₄ is -I; 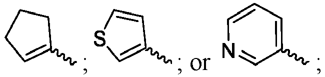 and
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula XIII: 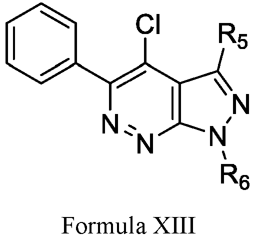 or a pharmaceutically acceptable salt thereof,
wherein R₅ is: 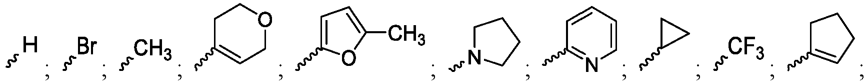 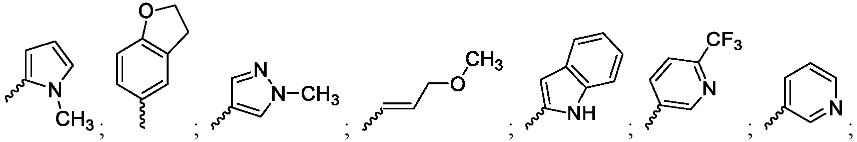 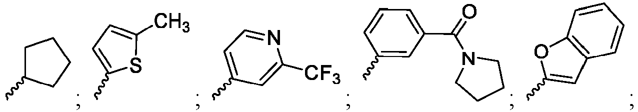 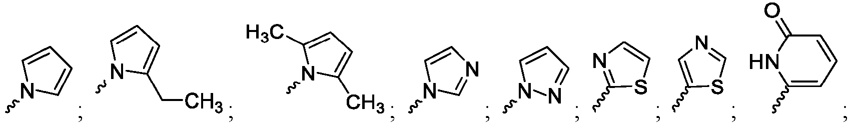 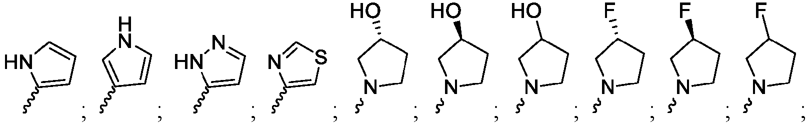 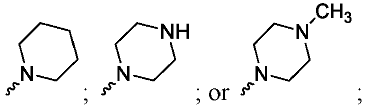
R₆ is: 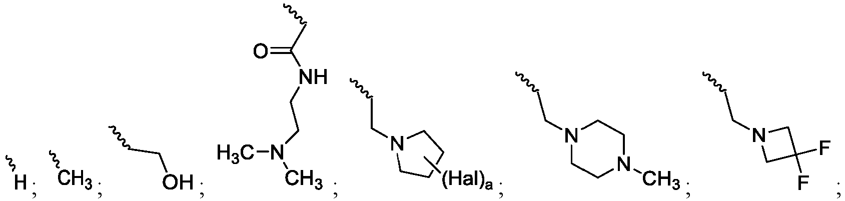 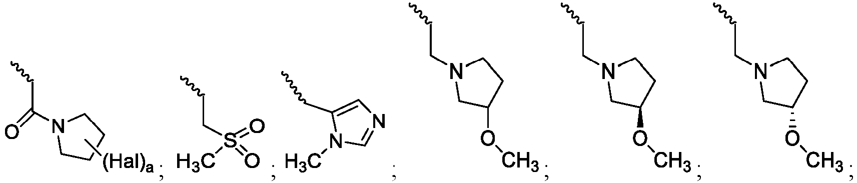 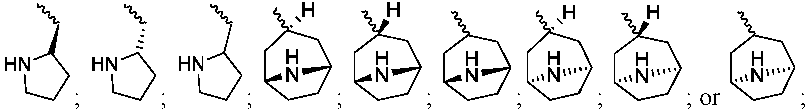
Hal is -Cl, -F, -I, or -Br; and
a is 0, 1, or 2;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula XIV: 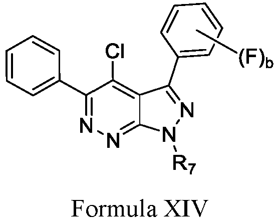 or a pharmaceutically acceptable salt thereof, wherein:
R₇ is: 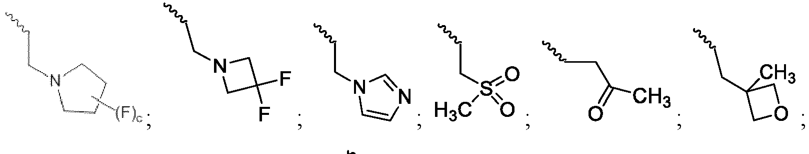 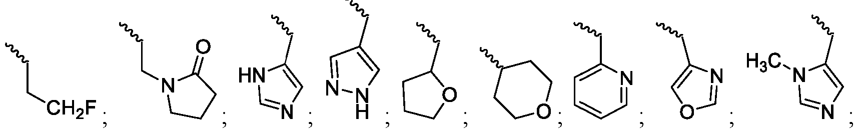 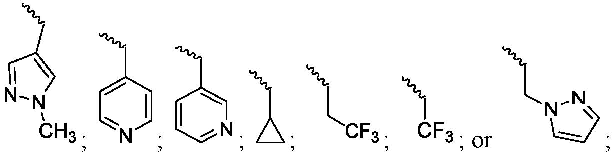
b is 0 or 1; and
c is 1 or 2;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of a compound of Formula XV: 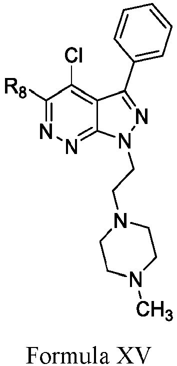 or a pharmaceutically acceptable salt thereof, wherein:
R₈ is: 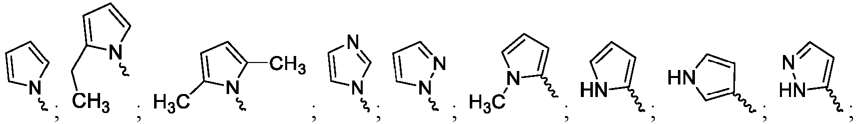 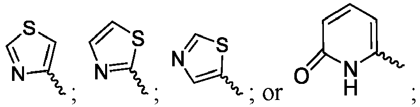 and
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of any of Compounds 44, 112, 113, and 116-123, or a pharmaceutically acceptable salt thereof;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating cancer, preventing cancer, or reducing risk of developing cancer, comprising administering to a subject in need thereof an effective amount of Compound 114 or 115, or a pharmaceutically acceptable salt thereof;
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating or preventing an inflammatory disease or an autoimmune disease, or reducing risk of developing an inflammatory disease or an autoimmune disease, comprising administering to a subject in need thereof an effective amount of a compound of Formula II: 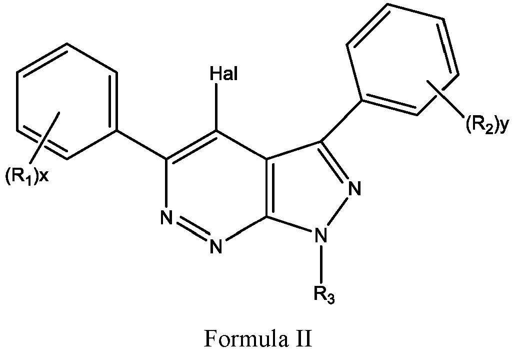 or a pharmaceutically acceptable salt thereof, wherein
Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 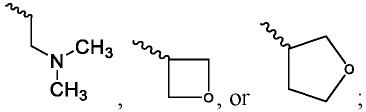
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 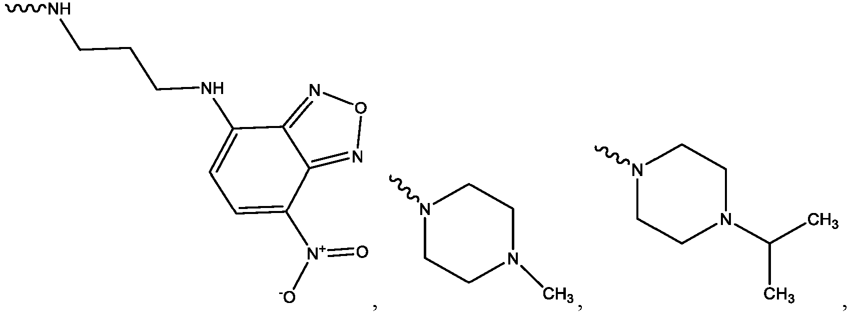 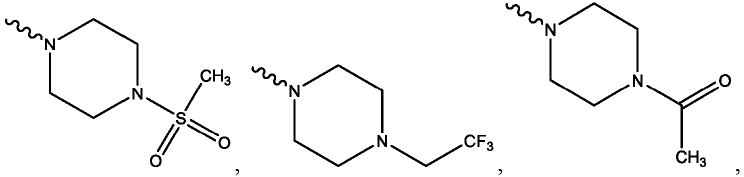 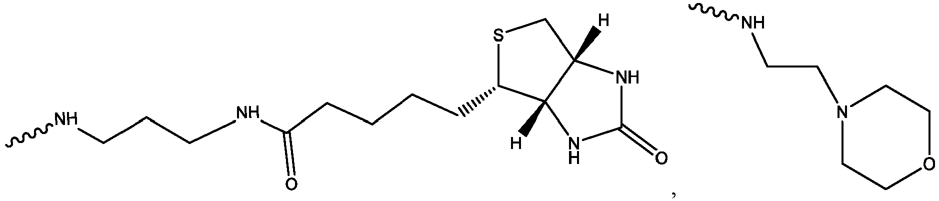 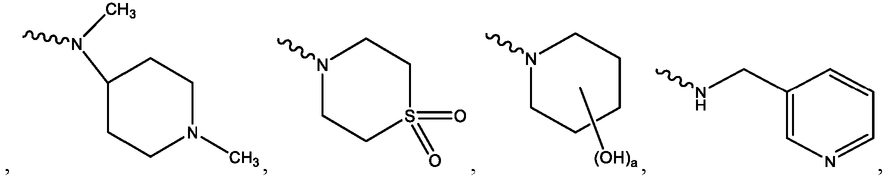 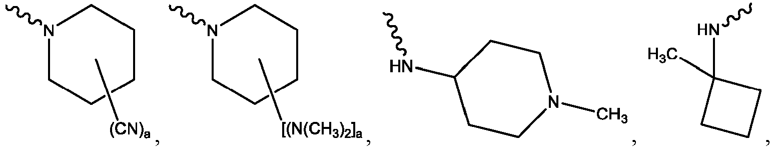 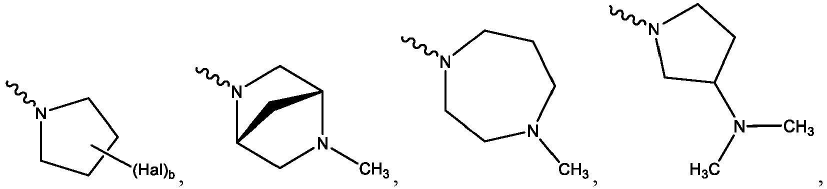 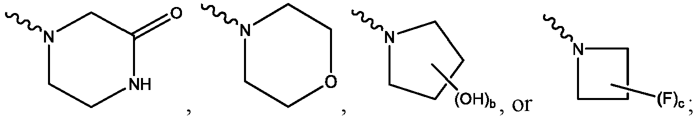
a is an integer ranging from 0 to 10;
b is an integer ranging from 0 to 8;
c is an integer ranging from 0 to 6; and
R₅ is

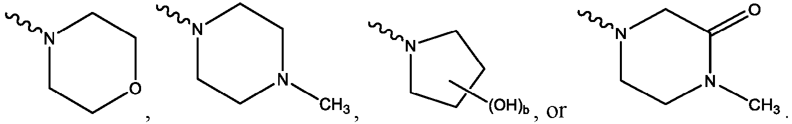

Also disclosed herein are methods for treating or preventing an inflammatory disease or an autoimmune disease, or reducing risk of developing an inflammatory disease or an autoimmune disease, comprising administering to a subject in need thereof an effective amount of a compound of Formula III: 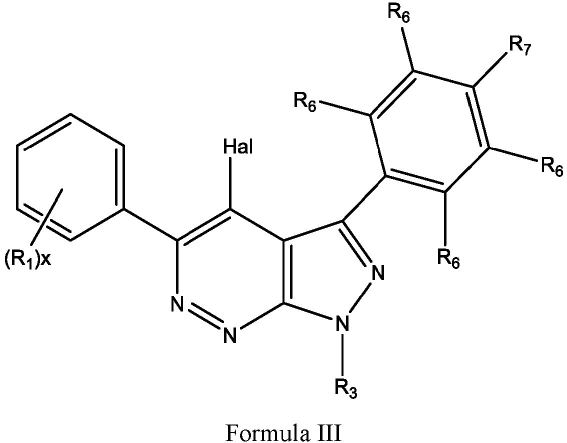 or a pharmaceutically acceptable salt thereof, wherein:
Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 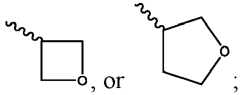
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂), -NH(C₁-C₆ alkylene)-O-(C₁-C₆ alkyl), 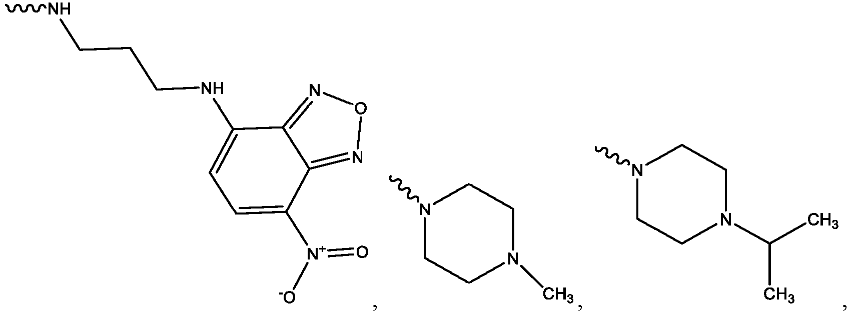 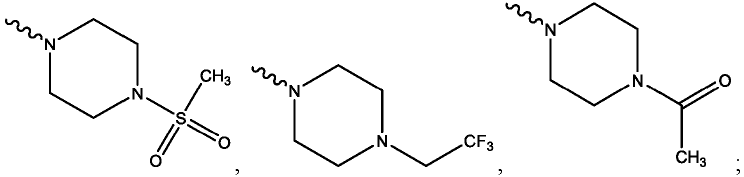 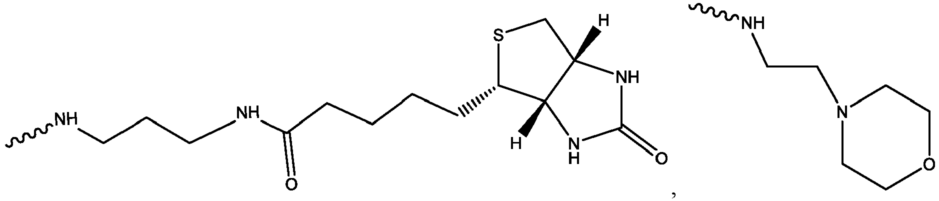 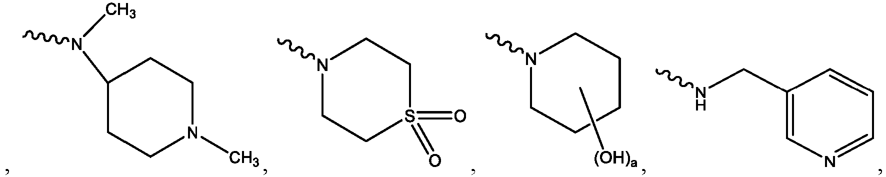 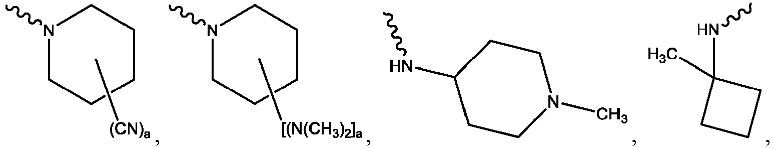 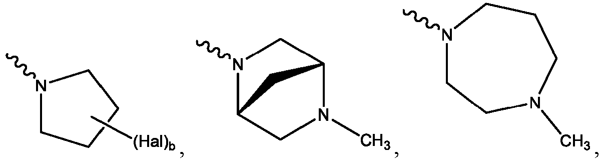 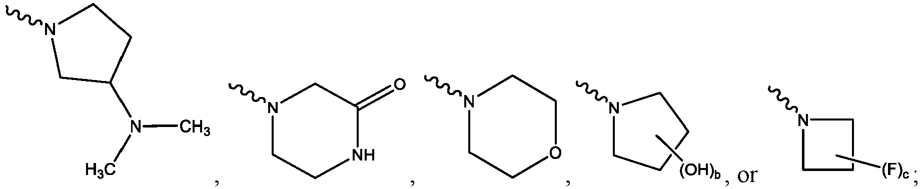
a is an integer ranging from 0 to 10;
b is an integer ranging from 0 to 8;
c is an integer ranging from 0 to 6;
R₅ is 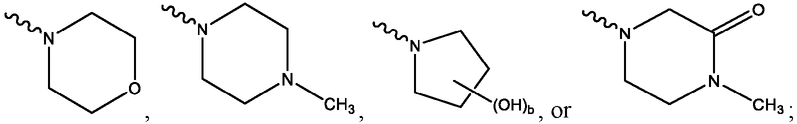 and
each R₆ and R₇ is independently -H or -I, wherein at least one of R₆ and R₇ is -I, and wherein when R₃ is -C₁-C₃ alkyl, then R₇ is -H.

Also disclosed herein are methods for treating or preventing an inflammatory disease or an autoimmune disease, or reducing risk of developing an inflammatory or an autoimmune disease, comprising administering to a subject in need thereof an effective amount of Compound 46, or a pharmaceutically acceptable salt thereof.

Also disclosed herein are methods for treating or preventing an inflammatory or an autoimmune disease, or reducing risk of developing an inflammatory or an autoimmune disease, comprising administering to a subject in need thereof an effective amount of a compound of Formula IV: 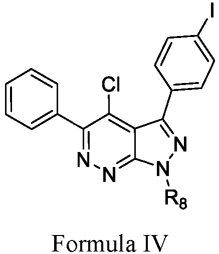 or a pharmaceutically acceptable salt thereof.

Also disclosed herein are methods for treating or preventing an inflammatory or an autoimmune disease, or reducing risk of developing an inflammatory or an autoimmune disease, comprising administering to a subject in need thereof an effective amount of a compound of Formula V: 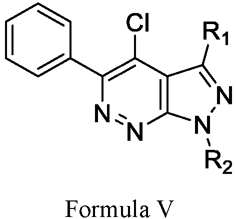 or a pharmaceutically acceptable salt thereof, wherein:
R₁ is: 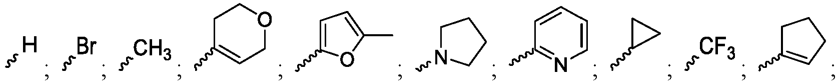 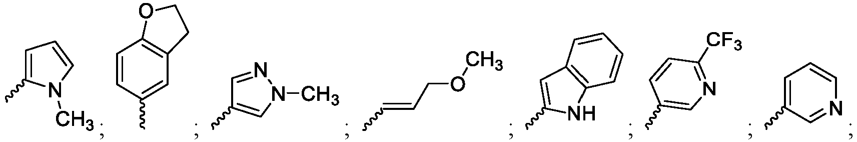 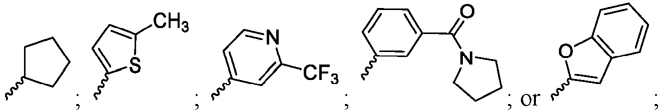
R₂ is: 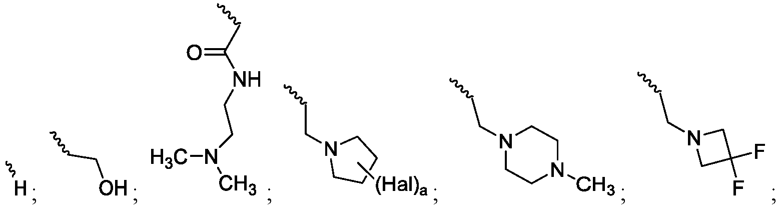 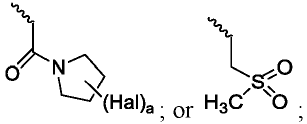
Hal is -Cl, -F, -I, or -Br; and
a is 0, 1, or 2.

Also disclosed herein are methods for treating or preventing an inflammatory or an autoimmune disease, or reducing risk of developing an inflammatory or an autoimmune disease, comprising administering to a subject in need thereof an effective amount of a compound of Formula VI: 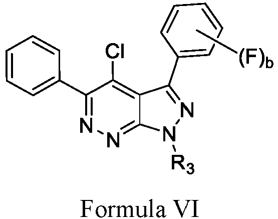 or a pharmaceutically acceptable salt thereof, wherein:
R₃ is: 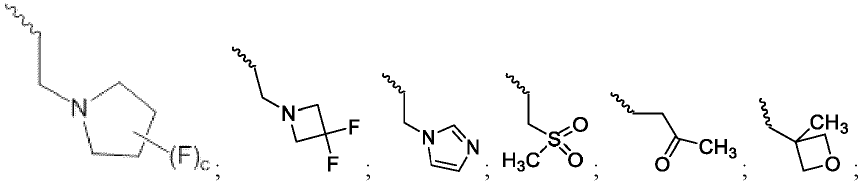 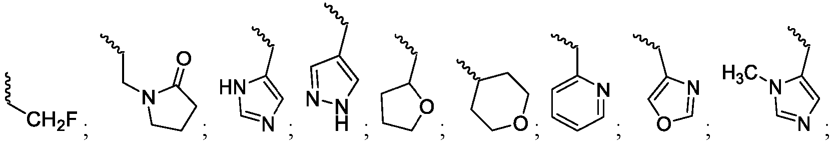 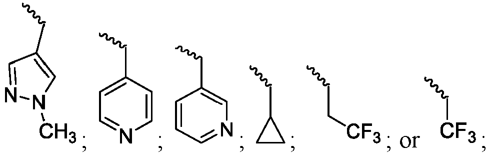
b is 0 or 1; and
c is 1 or 2.

Also disclosed herein are methods for treating or preventing an inflammatory or an autoimmune disease, or reducing risk of developing an inflammatory or an autoimmune disease, comprising administering to a subject in need thereof an effective amount of a compound of Formula VII: 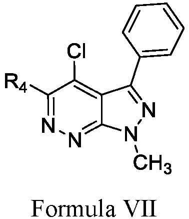 or a pharmaceutically acceptable salt thereof, wherein:
R₄ is -I;

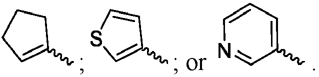

Also disclosed herein are methods for treating or preventing an inflammatory disease or an autoimmune disease, or reducing risk of developing an inflammatory disease or an autoimmune disease, comprising administering to a subject in need thereof an effective amount of a compound of Formula XIII: 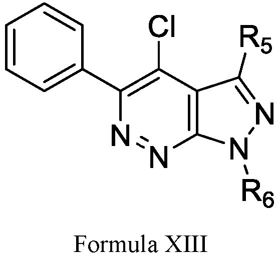 or a pharmaceutically acceptable salt thereof,
wherein R₅ is: 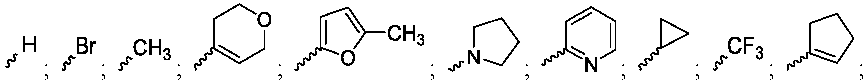 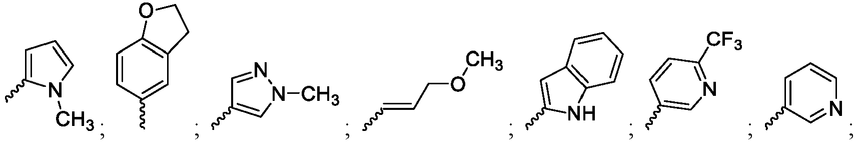 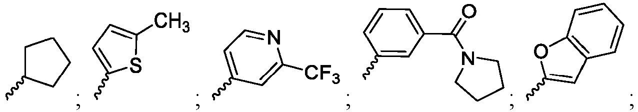 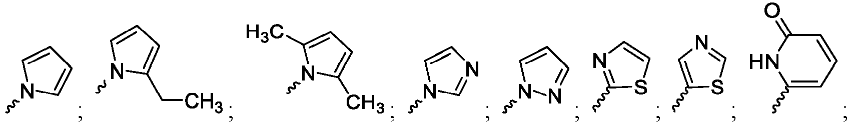 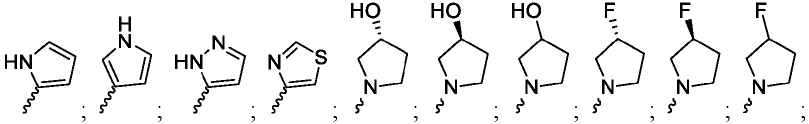 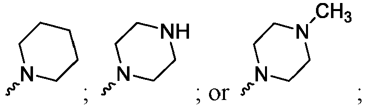
R₆ is: 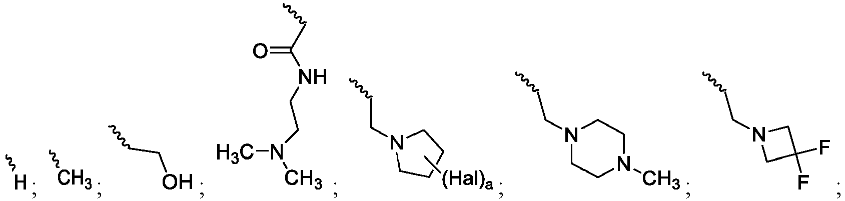 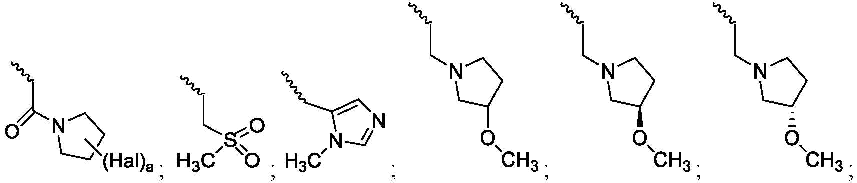 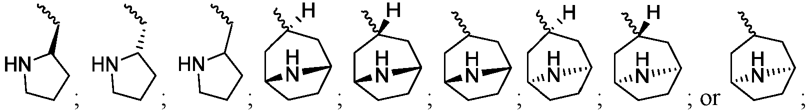
Hal is -Cl, -F, -I, or -Br; and
a is 0, 1, or 2.

Also disclosed herein are methods for treating or preventing an inflammatory disease or an autoimmune disease, or reducing risk of developing an inflammatory disease or an autoimmune disease, comprising administering to a subject in need thereof an effective amount of a compound of Formula XIV: 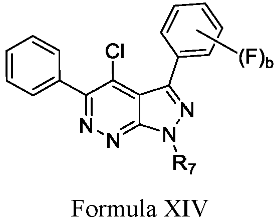 or a pharmaceutically acceptable salt thereof, wherein:
R₇ is: 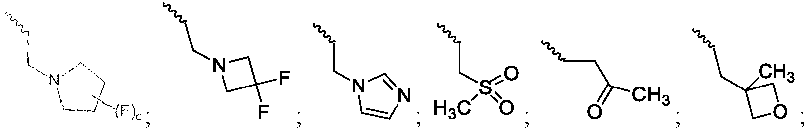 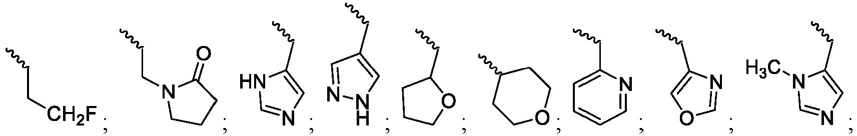 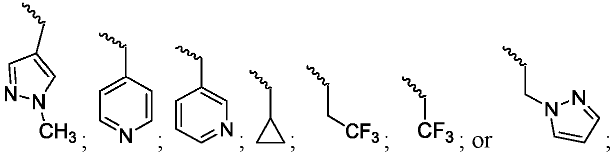
b is 0 or 1; and
c is 1 or 2.

Also disclosed herein are methods for treating or preventing an inflammatory disease or an autoimmune disease, or reducing risk of developing an inflammatory disease or an autoimmune disease, comprising administering to a subject in need thereof an effective amount of a compound of Formula XV: 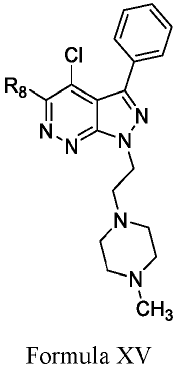 or a pharmaceutically acceptable salt thereof, wherein:
R₈ is:

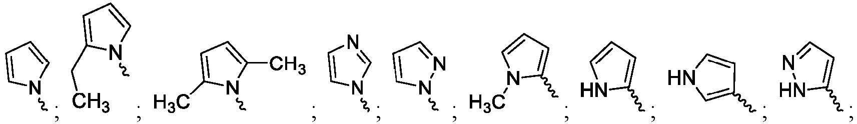

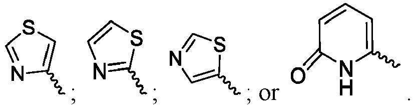

Also disclosed herein are methods for treating or preventing an inflammatory disease or an autoimmune disease, or reducing risk of developing an inflammatory or an autoimmune disease, comprising administering to a subject in need thereof an effective amount of any of Compounds 44, 112, 113, and 116-123, or a pharmaceutically acceptable salt thereof.

Also disclosed herein are methods for treating or preventing an inflammatory disease or an autoimmune disease, or reducing risk of developing an inflammatory disease or an autoimmune disease, comprising administering to a subject in need thereof an effective amount of Compound 114 or Compound 115, or a pharmaceutically acceptable salt thereof.

### DETAILED DESCRIPTION OF THE INVENTION

The invention provides a compound having the structure: 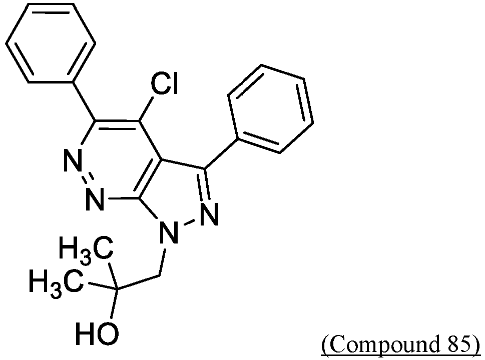 or a pharmaceutically acceptable salt thereof, for use in a method of treating cancer, preventing cancer, or reducing risk of developing cancer, wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

Also disclosed herein are methods for treating or preventing an inflammatory disease or an autoimmune disease, or reducing risk of developing an inflammatory disease or an autoimmune disease, comprising administering to a subject in need thereof an effective amount of a compound of the disclosure. Also disclosed herein are methods for treating or preventing an inflammatory disease or an autoimmune disease, or reducing risk of developing an inflammatory disease or an autoimmune disease, comprising administering to a subject in need thereof an effective amount of Compound 85 or a pharmaceutically acceptable salt thereof.

### Definitions

The term "alkyl" refers to a straight or branched saturated hydrocarbon group. Illustrative alkyl groups include -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH(CH₃)CH₂CH₂CH₃, -CH₂CH₂CH(CH₃)₂, -CH₂C(CH₃)₃, -CH₂CH₂CH₂CH₂CH₃, -CH(CH₃)CH₂CH₂CH₃, -CH₂CH₂CH(CH₃)₂ and -CH(CH₃)C(CH₃)₃ groups.

The term "alkylene" refers to an alkyl group bonded to another atom or group. Illustrative alkylene groups include -CH₂-, -CH₂CH₂-, -CH₂CH₂CH₂-, -C(CH₃)₂-, -CH(CH₃), -CH₂CH₂CH₂CH₂-, -CH(CH₃)CH₂CH₂-, -CH₂C(CH₃)₂-, -C(CH₃)₂CH₂-, -CH₂CH₂CH₂CH₂CH₂-, -CH(CH₃)CH₂CH₂CH₂-, -CH₂CH₂C(CH₃)₂-, -CH₂CH(CH₃)CH₂CH₂, -CH₂CH₂CH(CH₃)CH₂-, -CH₂CH₂CH₂CH₂CH₂CH₂-, -CH(CH₃)CH₂CH₂CH₂CH₂-, -CH₂CH₂CH₂C(CH₃)₂-, -CH₂CH(CH₃)CH₂CH₂CH₂-, -CH₂CH₂CH₂CH(CH₃)CH₂- and -C(CH₃)₂C(CH₃)₂ - groups.

The term "alkenyl" refers to a straight or branched hydrocarbon group having one or more double bonds. Illustrative alkenyl groups include -CH=CH₂, -CH₂CH=CH₂, *cis* - CH=CHCH₃, *trans* -CH=CHCH₃, -C(CH₃)=CH₂, *cis* -CH=CHCH₂CH₃, *trans -* CH=CHCH₂CH₃, *cis* -CH₂CH=CHCH₃, *trans* -CH₂CH=CHCH₃, -CH₂CH₂CH=CH₂, *cis -* CH=CHCH₂CH₂CH₃, *trans* -CH=CHCH₂CH₂CH₃, *cis* -CH₂CH₂CH=CHCH₃, *trans -* CH₂CH₂CH=CHCH₃, -CH₂CH₂CH₂CH=CH₂, -CH₂CH=C(CH₃)₂, *cis -* CH=CHCH₂CH₂CH₂CH₃, *trans* -CH=CHCH₂CH₂CH₂CH₃, *cis* -CH₂CH₂CH₂CH=CHCH₃, *trans* -CH₂CH₂CH₂CH=CHCH₃, -CH₂CH₂CH₂CH₂CH=CH₂, and -CH₂CH₂CH=C(CH₃)₂, groups.

The word "about" when immediately preceding a numerical value means a range of plus or minus 10% of that value, e.g., "about 100 mg" means 90 mg to 110 mg, "about 300 mg" means 270 mg to 330 mg, etc.

### Abbreviations:

- DCM: dichloromethane
- DEAD: diethyl azodicarboxylate
- DIPEA: diisopropylethylamine
- DMF: dimethylformamide
- DMSO: Dimethyl sulfoxide
- ESI: Electrospray ionization
- ESI-TOF: Electrospray ionization-Time-of-flight
- HATU: 2-(7-Aza-1H-benzotriazole-1-yl)-1,1,3,3-tetramethyluronium hexafluorophosphate
- HPLC: High-performance liquid chromatography
- LCMS: Liquid chromatography-mass spectrometry
- LDA: lithium diisopropyl amide
- m/z: Mass-to-charge ratio
- MS: Mass spectrometry
- Rt: Retention time
- TFA: trifluoroacetic acid
- THF: tetrahydrofuran

The term "effective amount" means an amount of a compound that is effective to treat or prevent cancer, an inflammatory disease or an autoimmune disease, or to lower risk of developing cancer, an inflammatory disease or an autoimmune disease. In some embodiments, where another therapeutic or prophylactic agent is administered prior to, subsequent to or concurrently with administration of a compound of the disclosure, the "effective amount" is the total amount of (i) the compound and (ii) the other therapeutic or prophylactic agent that is effective to treat or prevent cancer, an inflammatory disease or an autoimmune disease, or to lower risk developing of cancer, an inflammatory disease or an autoimmune disease.

A "subject" is a mammal, including a species-rich order, e.g., a primate, such as a human; a Rodentia species, such as a mouse, a rat or a guinea pig; a Carnivora species such as a *Canis sp.,* e.g., a dog, *Felis sp.,* e.g., a cat, weasel, bear or seal; a non-human primate, such as a monkey, chimpanzee, baboon or rhesus; a Chiroptera species, such as a bat; a Soricomorpha species, such as a shrew, mole or solenodon; and a Cetartiodactyla species, such as a whale. In one embodiment, the subject is a human. In another embodiment, the human is a human fetus.

### Compounds of the Disclosure

### Compounds of Formula I

Disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula I: 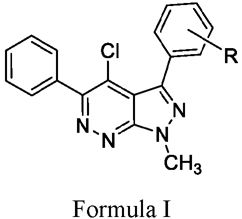
or a pharmaceutically acceptable salt thereof,
wherein R is fluoro, chloro, iodo, methyl, methoxy, cyano, trifluoromethyl, or
-(CO)NH(CH₃).

R of Formula I may be in the para position relative to the pyrazolopyridazino ring system. Alternatively, R of Formula I may be in the meta position relative to the pyrazolopyridazino ring system. Alternatively, R of Formula I may be in the ortho position relative to the pyrazolopyridazino ring system.

### Compounds of Formula II

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula II: 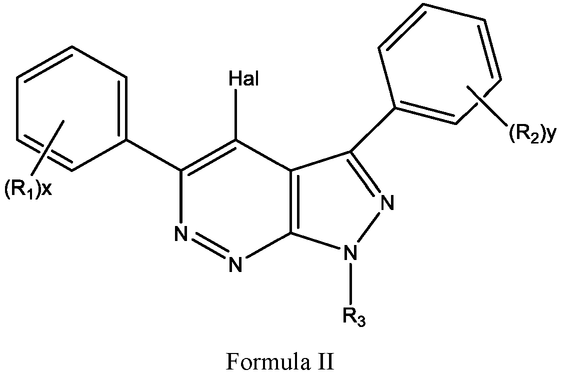 or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
y is an integer ranging from 0 to 5;
each R₂ is independently -Cl, -F, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅, 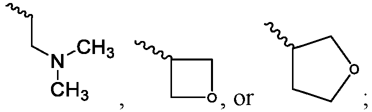
R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂),
-NH(C₁-C₆ alkylene)-O-(C₁-C₆ alkyl), 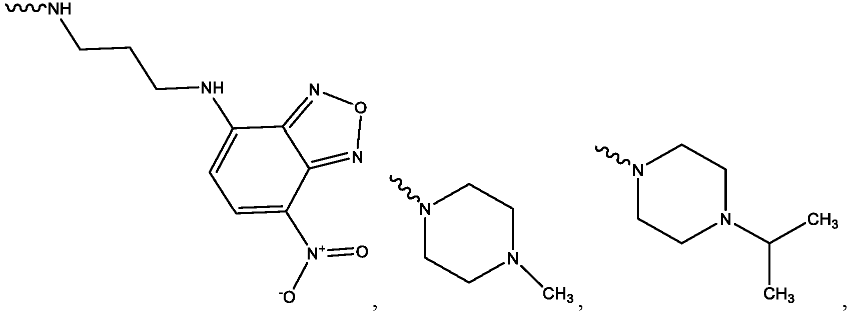 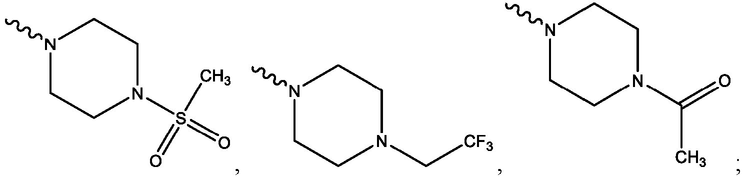 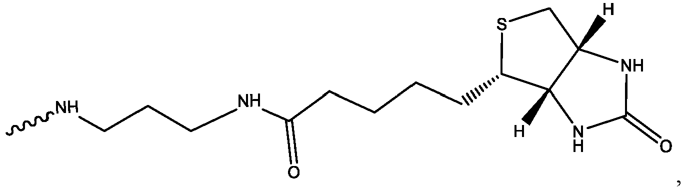 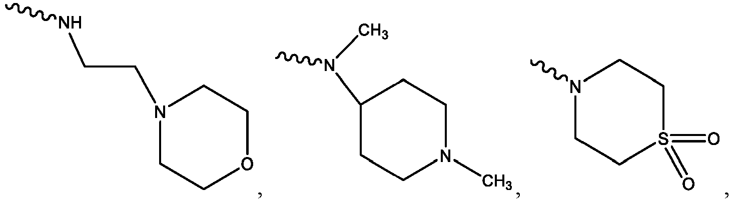 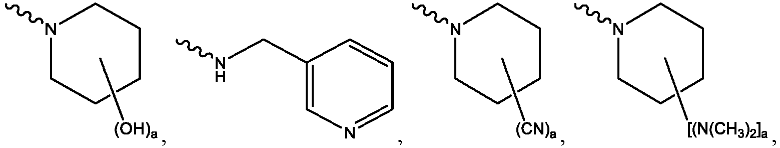 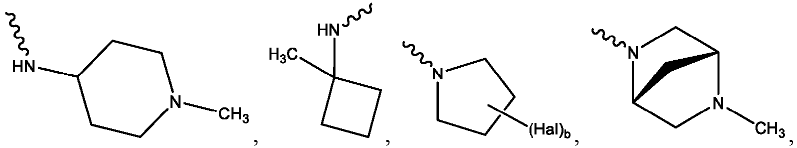 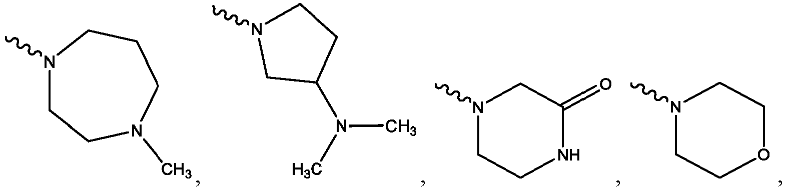 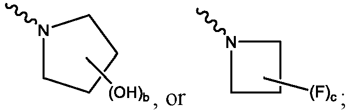
a is an integer ranging from 0 to 10;
b is an integer ranging from 0 to 8;
c is an integer ranging from 0 to 6; and
R₅ is

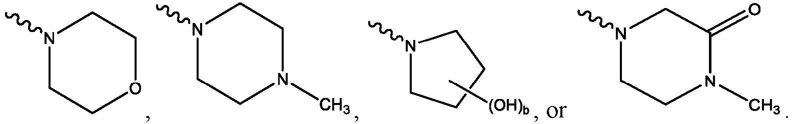

In certain examples, Hal is -Cl. In yet another example, x and y are 0.

In certain examples, x and y are 0, x is 0 and y is 1, x is 1 and y is 2, x is 1 and y is 0, x is 1 and y is 1, x is 1 and y is 2, x is 2 and y is 0, x is 2 and y is 1, or x is 2 and y is 2.

In certain examples, Hal is -Cl and: x and y are 0, x is 0 and y is 1, x is 1 and y is 2, x is 1 and y is 0, x is 1 and y is 1, x is 1 and y is 2, x is 2 and y is 0, x is 2 and y is 1, or x is 2 and y is 2.

In particular examples, x is 1 and R₁ is in the ortho position relative to the pyrazolopyridazino ring system. In certain examples, x is 1 and R₁ is in the para position relative to the pyrazolopyridazino ring system. In further examples, x is 1 and R₁ is in the meta position relative to thepyrazolopyridazino ring system.

In particular examples, y is 1 and R₂ is in the ortho position relative to the pyrazolopyridazino ring system. In certain examples, y is 1 and R₂ is in the para position relative to the pyrazolopyridazino ring system. In further examples, y is 1 and R₂ is in the meta position relative to the pyrazolopyridazino ring system.

In particular examples, x is 2 and R₁ is in the ortho and meta position relative to the pyrazolopyridazino ring system. In certain examples, x is 2 and R₁ is in the ortho and para position relative to the pyrazolopyridazino ring system. In further examples, x is 2 and R₁ is in the para and meta position relative to the pyrazolopyridazino ring system.

In particular examples, y is 2 and R₂ is in the ortho and meta position relative to the pyrazolopyridazino ring system. In certain examples, y is 2 and R₂ is in the ortho and para position relative to the pyrazolopyridazino ring system. In further examples, y is 2 and R₂ is in the para and meta position relative to the pyrazolopyridazino ring system.

In yet other examples, R₁ is chloro. In certain examples, R₁ is fluoro. In certain examples, R₁ is iodo. In other examples, R₁ is -Br. In further examples, R₁ is -OCH₃. In other examples, R₁ is -CH₃. In yet other examples, R₁ is -C(O)N(H)CH₃. In certain examples, R₁ is -CF₃. In further examples, R₁ is -CN. In additional examples, R₁ is -C≡CCH₂OH.

In yet other examples, x is 1 or 2, and R₁ is -Cl, -F, -1, -Br, -OCH₃, -CH₃, -C(O)N(H)CH₃, -CF₃, -CN or -C≡CCH₂OH.

In yet other examples, Hal is -Cl, x is 1 or 2, and R₁ is -Cl, -F, -I, -Br, -OCH₃, -CH₃, -C(O)N(H)CH₃, -CF₃, -CN or -C≡CCH₂OH.

In yet other examples, R₂ is -Cl. In certain examples, R₂ is -F. In other examples, R₂ is -Br. In further examples, R₂ is -OCH₃. In other examples, R₂ is
-CH₃. In yet other examples, R₂ is -C(O)N(H)CH₃. In certain examples, R₂ is -CF₃. In further examples, R₂ is -CN. In additional examples, R₂ is -C≡CCH₂OH.

In yet other examples, y is 1 or 2, and R₂ is -Cl, -F, -Br, -OCH₃, -CH₃, -C(O)N(H)CH₃, -CF₃, -CN or -C≡CCH₂OH.

In yet other examples, Hal is -Cl, y is 1 or 2, and R₂ is -Cl, -F, -Br, -OCH₃,
-CH₃, -C(O)N(H)CH₃, -CF₃, -CN or -C≡CCH₂OH.

In particular examples, R₃ is -H. In certain examples, R₃ is -CH₃. In further examples, R₃ is -CH₂CH₃. In still further examples, R₃ is -CHCH₂. In other examples, R₃ is - CH₂CH₂OH. In particular examples, R₃ is -(CH₂)₂C₆H₅. In other examples, R₃ is - CH₂C(O)OH. In yet other examples, R₃ is -CH₂C(O)N(H)CH₃. In certain examples, R₃ is - CH₂C(O)N(H)((CH₂)₂N(CH₃)₂).

In yet other examples, R₃ is -CH₂C(O)N(H)((CH₂)₃N(CH₃)₂). In other examples, R₃ is -CH₂C(O)N(CH₃)CH₂CN. In particular examples, R₃ is -CH₂C(O)NH₂. In certain examples, R₃ is -CH₂C(O)N(H)((CH₂)₂OH). In other examples, R₃ is -CH₂C(O)N(H)((CH₂)₂OCH₃). In still further examples, R₃ is -CH₂C(CH₃)₂OH. In yet other examples, R₃ is -CH₂C(O)OCH₃. In further examples, R₃ is - CH₂CH(OH)CH₃. In still further examples, R₃ is -CH₂CH₂OH. In particular examples, R₃ is -CH(CH₃)CH₂OH.

In further examples, R₃ is -CH₂C(O)R₄ and R₄ is 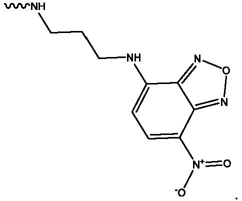 In other examples, R₃ is -CH₂C(O)R₄ and R₄ is 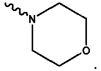 In particular examples, R₃ is -CH₂C(O)R₄ and R₄ is 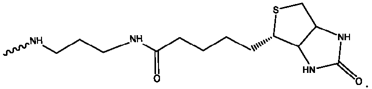 In yet other examples, R₃ is - CH₂C(O)R₄ and R₄ is 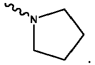 In certain examples, R₃ is -CH₂C(O)R₄ and R₄ is 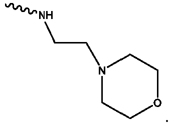 In other examples, R₃ is -CH₂C(O)R₄ and R₄ is 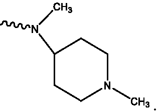 In particular examples, R₃ is -CH₂C(O)R₄ and R₄ is 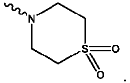 In yet other examples, R₃ is - CH₂C(O)R₄ and R₄ is 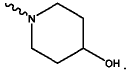 In certain examples, R₃ is -CH₂C(O)R₄ and R₄ is 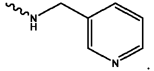 In other examples, R₃ is -CH₂C(O)R₄ and R₄ is 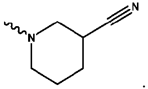 In particular examples, R₃ is -CH₂C(O)R₄ and R₄ is 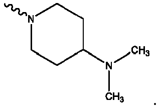 In yet other examples, R₃ is - CH₂C(O)R₄ and R₄ is 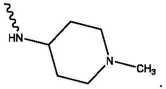 In certain examples, R₃ is -CH₂C(O)R₄ and R₄ is 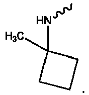 In other examples, R₃ is -CH₂C(O)R₄ and R₄ is 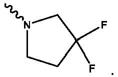 In particular examples, R₃ is -CH₂C(O)R₄ and R₄ is 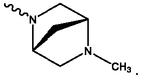 In yet other examples, R₃ is -CH₂C(O)R₄ and R₄ is 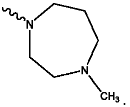 In certain examples, R₃ is -CH₂C(O)R₄ and R₄ is 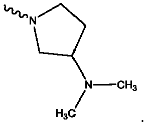 In other examples, R₃ is -CH₂C(O)R₄ and R₄ is 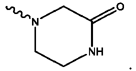 In particular examples, R₃ is -CH₂C(O)R₄ and R₄ is 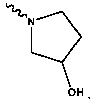 In yet other examples, R₃ is -CH₂C(O)R₄ and R₄ is 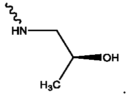 In certain examples, R₃ is -CH₂C(O)R₄ and R₄ is 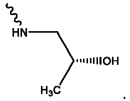 In other examples, R₃ is -CH₂C(O)R₄ and R₄ is 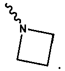 In particular examples, R₃ is -CH₂C(O)R₄ and R₄ is 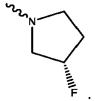 In yet other examples, R₃ is - CH₂C(O)R₄ and R₄ is 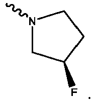 In certain examples, R₃ is -CH₂C(O)R₄ and R₄ is 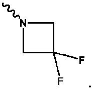 In other examples, R₃ is -CH₂C(O)R₄ and R₄ is 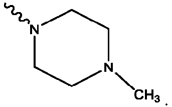 In further examples of the invention, R₃ is -CH₂C(O)R₄ and R₄ is 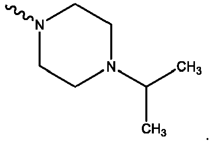 In certain examples of the invention, R₃ is -CH₂C(O)R₄ and R₄ is 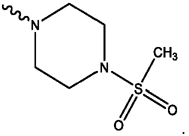 In other examples, R₃ is - CH₂C(O)R₄ and R₄ is 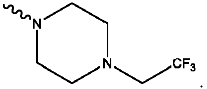 In further examples, R₃ is -CH₂C(O)R₄ and R₄ is 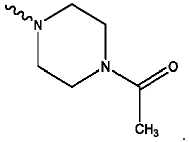 In further examples, R₃ is -CH₂C(O)R₄ and R₄ is

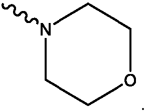

In particular examples, R₃ is -(CH₂)₂R₅ and R₅ is 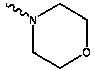 In yet other examples, R₃ is -(CH₂)₂R₅ and R₅ is 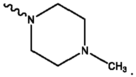 In certain examples, R₃ is -(CH₂)₂R₅ and R₅ is 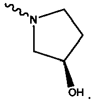 In other examples, invention, R₃ is -(CH₂)₂R₅ and R₅ is

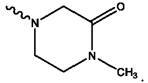

In some examples, a is an integer ranging from 0 to 5. In some examples, b is an integer ranging from 0 to 4. In some examples, c is an integer ranging from 0 to 6.

### Illustrative Compounds of Formula II

The compound of Formula II may have the structure: 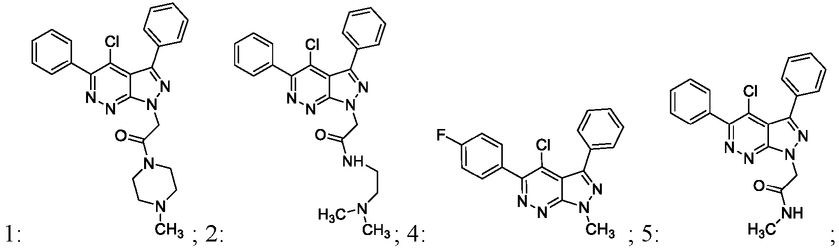 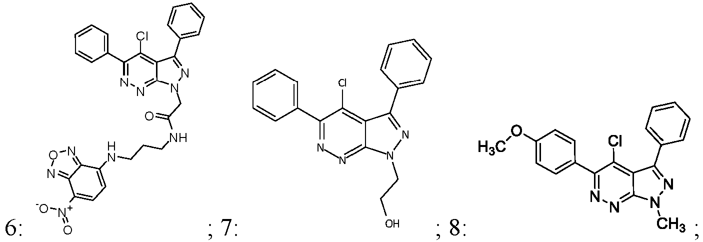 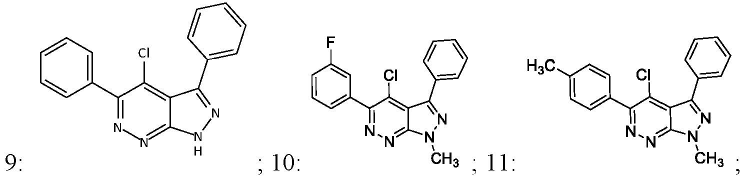 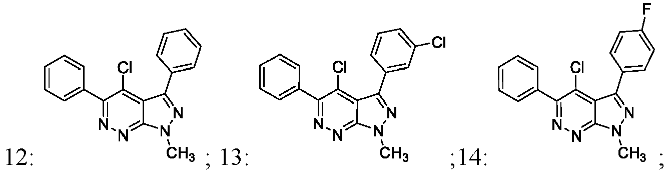 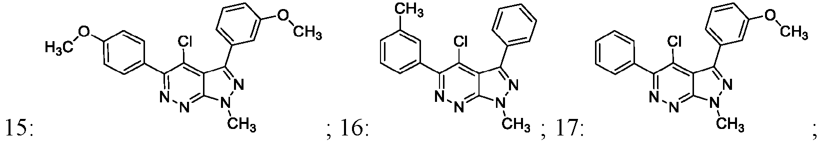 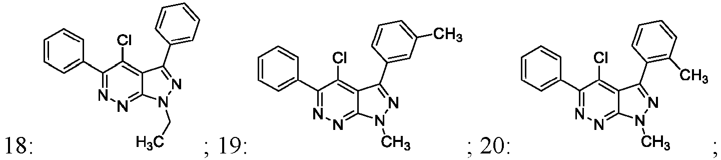 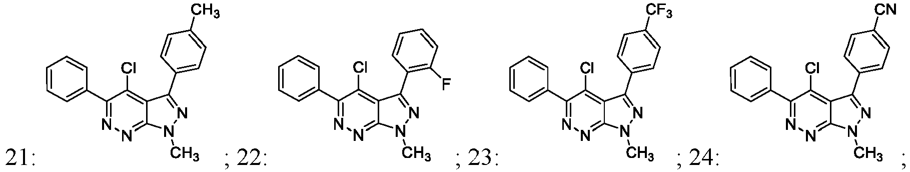 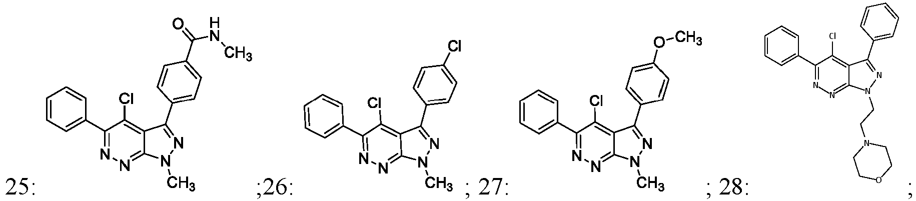 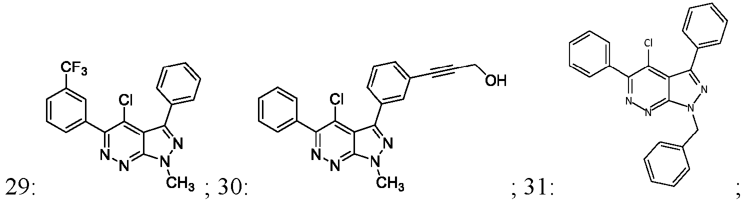 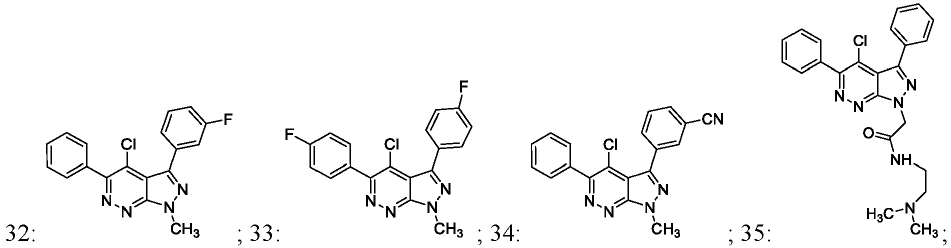 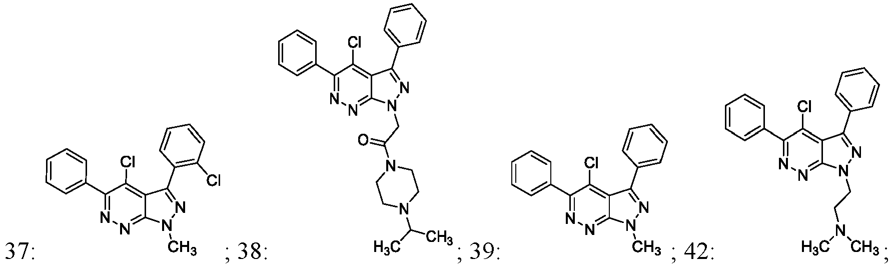 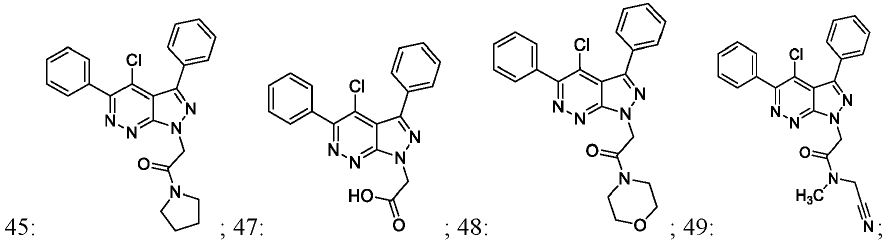 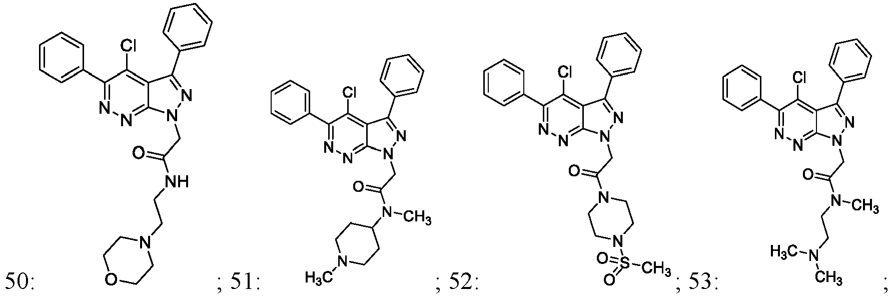 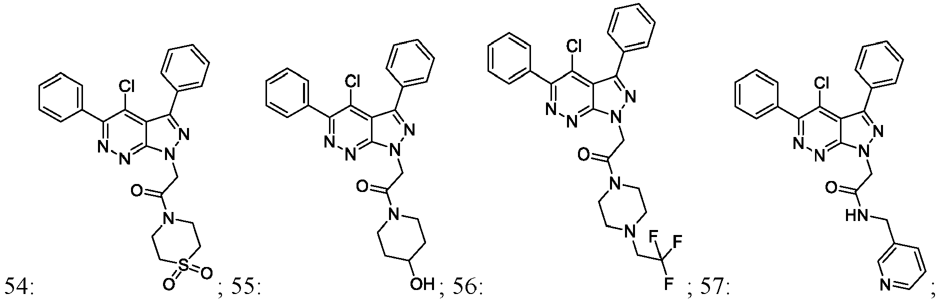 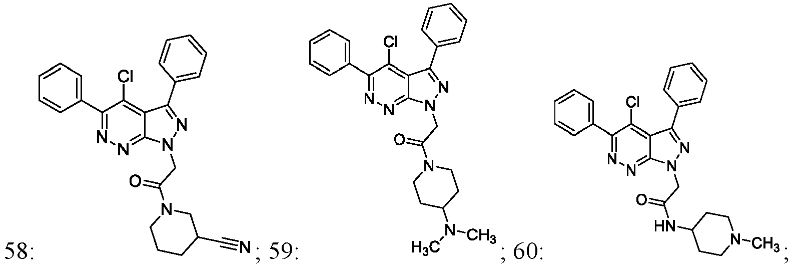 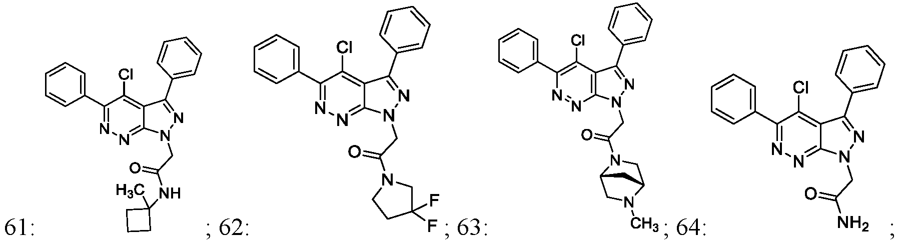 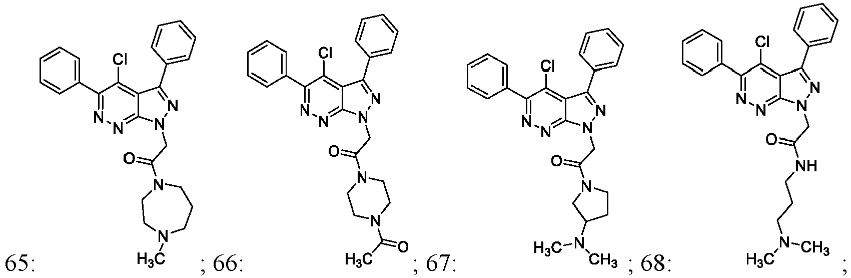 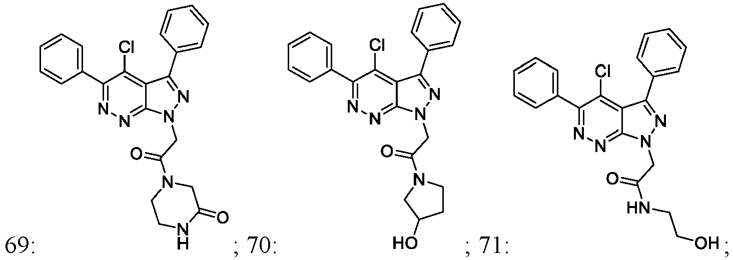 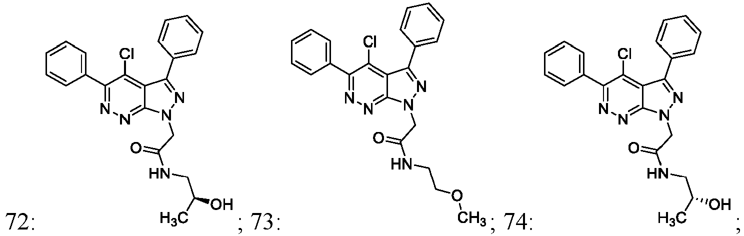 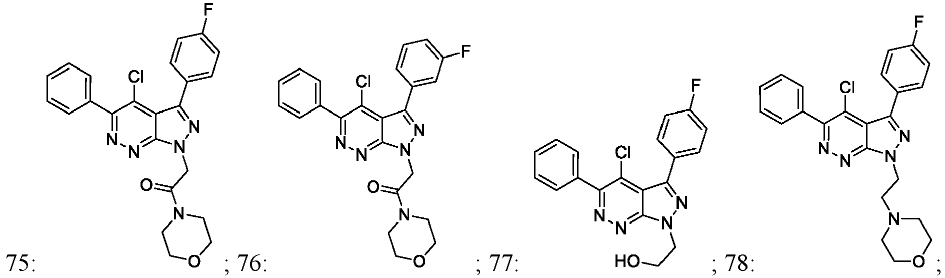 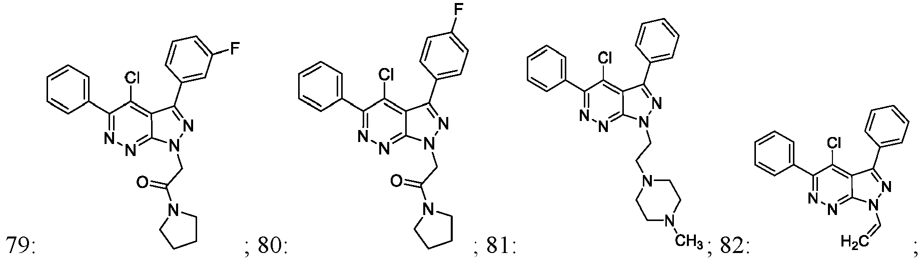 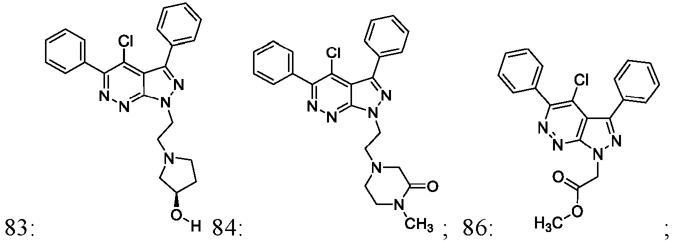 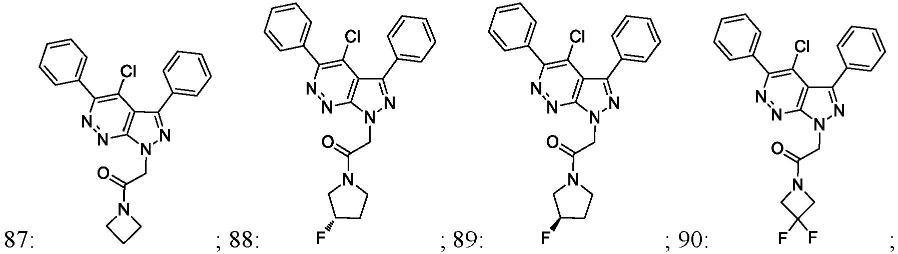 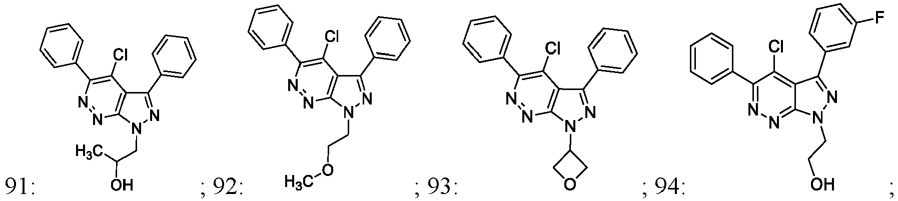 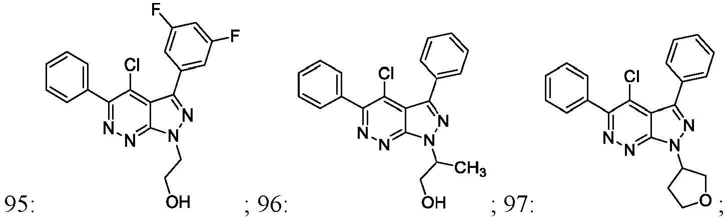 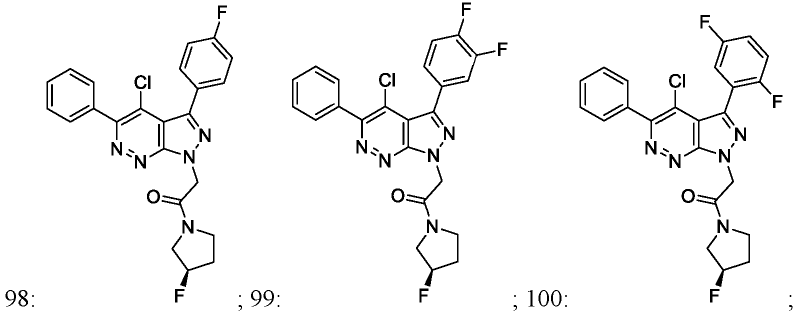 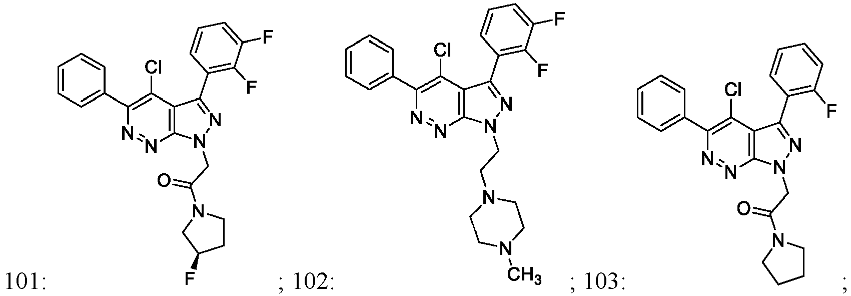 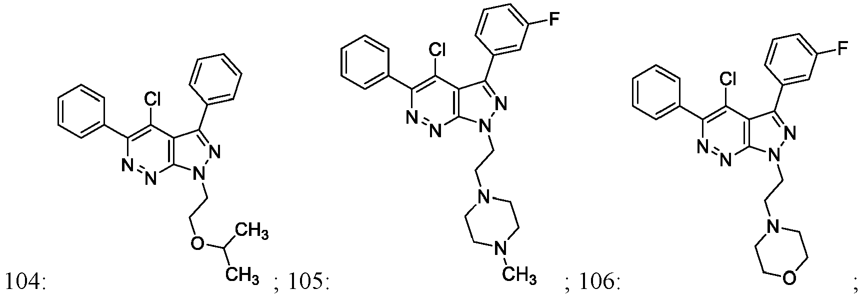 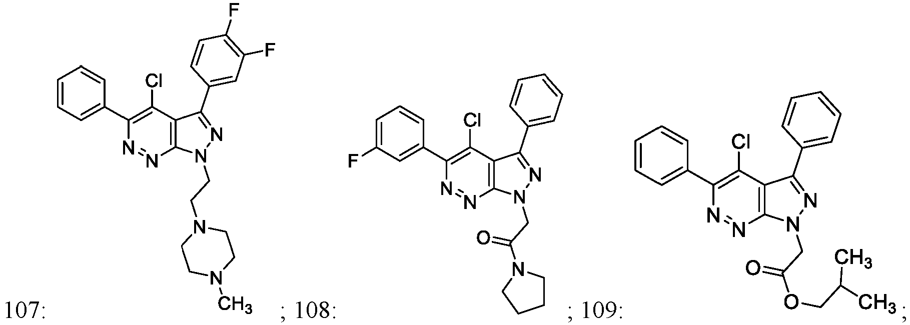 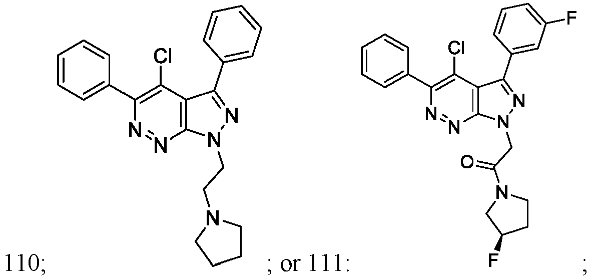 or a pharmaceutically acceptable salt thereof.

### Compounds of Formula III

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula III: 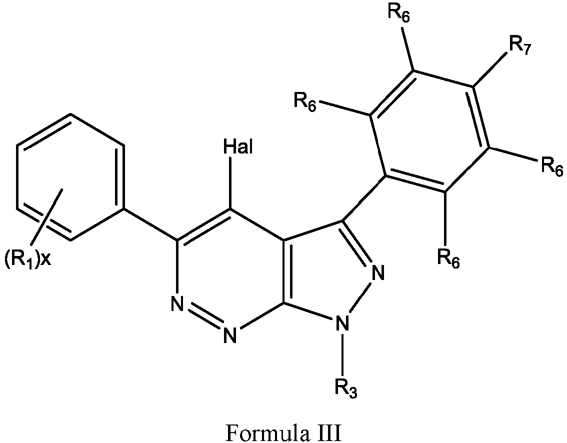 or a pharmaceutically acceptable salt thereof,
wherein Hal is -Cl, -F, -I, or -Br;
x is an integer ranging from 0 to 5;
each R₁ is independently -Cl, -F, -I, -Br, -C₁-C₃ alkyl, -O- C₁-C₃ alkyl, -CN, -CF₃, -C(O)NH(CH₃), or -C≡CCH₂OH;
R₃ is -H, -C₁-C₆ alkyl, -(C₁-C₆ alkylene)-OH, -(C₁-C₆ alkylene)-phenyl, -(C₁-C₆ alkylene)-O-(C₁-C₆ alkyl), -C₂-C₆ alkenyl, -(C₁-C₆ alkylene)-C(O)R₄, -(C₁-C₆ alkylene)-R₅ 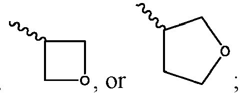 R₄ is -OH, -O-(C₁-C₆ alkyl), -NH₂, -NH(C₁-C₆ alkyl), -NH((C₁-C₆ alkylene)-OH), -NH((C₁-C₆ alkylene)N(C₁-C₆ alkyl)₂), -N(C₁-C₆ alkyl)((C₁-C₆ alkylene)-CN), -N(C₁-C₆ alkyl)((C₁-C₆ alkylene)N(C₁-C₆ alkyl)₂),
-NH(C₁-C₆ alkylene)-O-(C₁-C₆ alkyl), 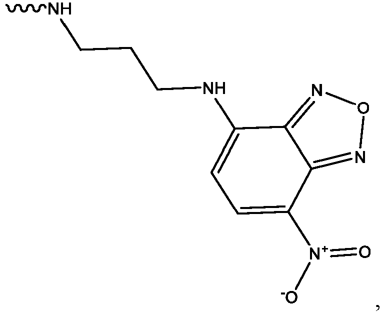 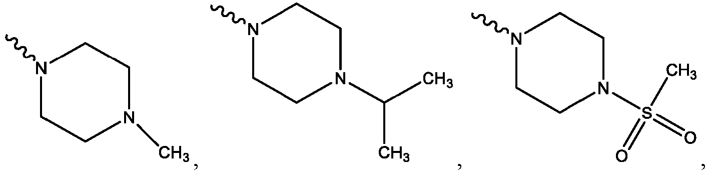 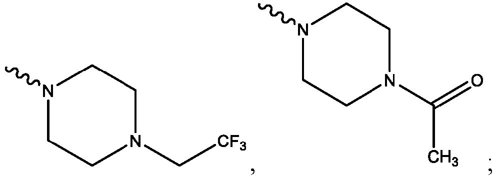 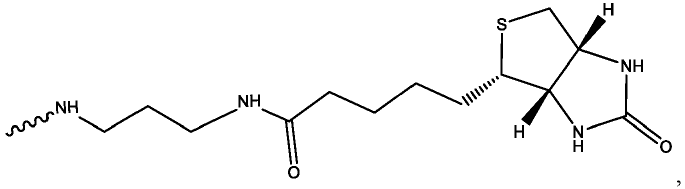 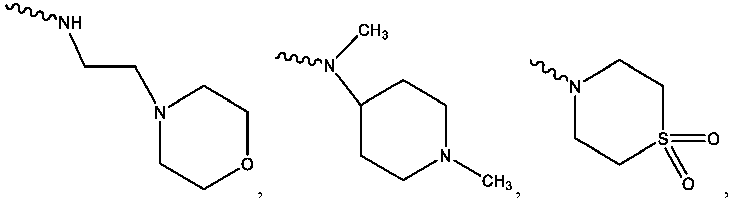 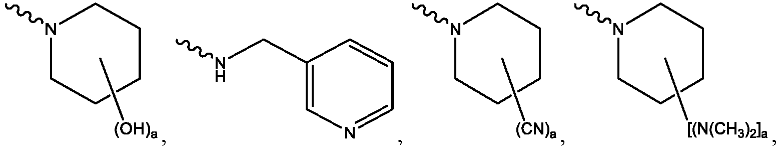 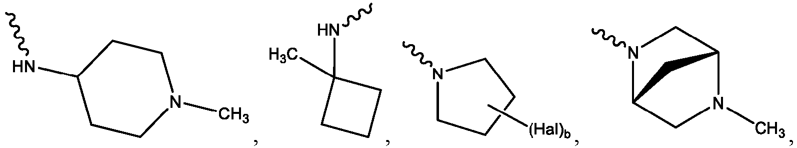 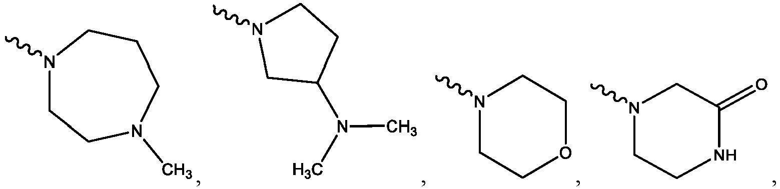 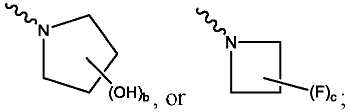
a is an integer ranging from 0 to 10;
b is an integer ranging from 0 to 8;
c is an integer ranging from 0 to 6;
R₅ is 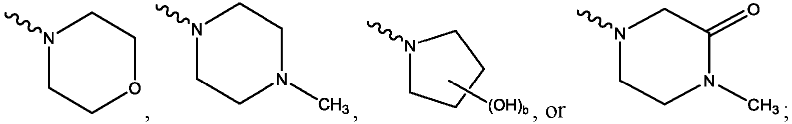
wherein each R₆ and R₇ is independently -H or -I, wherein at least one of R₆ and
R₇ is -I, and
wherein when R₃ is -C₁-C₃ alkyl, R₇ is -H.

In certain examples, one R₆ in the ortho position relative to the pyrazolopyridazino ring system is iodo and the remaining R₆ and R₇ groups are hydrogen. In other examples, one R₆ in the para position relative to the pyrazolopyridazino ring system is iodo and the remaining R₆ and R₇ groups are hydrogen. In further examples, one R₆ in the ortho position relative to the pyrazolopyridazino ring system and one R₆ in the para position relative to the pyrazolopyridazino ring system are iodo and the remaining R₆ and R₇ groups are hydrogen. In further examples, the two R₆ groups in the ortho positions relative to the pyrazolopyridazino ring system and one R₆ in the para position relative to the pyrazolopyridazino ring system are iodo and the remaining R₆ and R₇ groups are hydrogen. In further examples, the two R₆ groups in the para positions relative to the pyrazolopyridazino ring system and one R₆ in the ortho position relative to the pyrazolopyridazino ring system are iodo and the remaining R₆ and R₇ are hydrogen. In certain examples, all R₆ groups are iodo and R₇ is hydrogen. In yet further examples, R₇ is iodo and the R₆ groups are hydrogen.

In a particular example, one R₆ in the para position relative to the pyrazolopyridazino ring system is iodo and R₃ is -CH₃.

In certain examples, Hal is -Cl. In yet another example, x is 0. In another example, x is 1. In a certain examples, x is 2.

In particular examples, x is 1 and R₁ is in the ortho position relative to the pyrazolopyridazino ring system. In certain examples, x is 1 and R₁ is in the para position relative to the pyrazolopyridazino ring system. In further examples, x is 1 and R₁ is in the meta position relative to the pyrazolopyridazino ring system.

In particular examples, x is 2 and R₁ is in the ortho and meta position relative to the pyrazolopyridazino ring system. In certain examples, x is 2 and R₁ is in the ortho and para position relative to the pyrazolopyridazino ring system. In further examples, x is 2 and R₁ is in the para and meta position relative to the pyrazolopyridazino ring system.

In yet other examples, R₁ is -Cl. In certain examples, R₁ is -F. In certain examples, R₁ is -I. In further examples, R₁ is -OCH₃. In other examples, R₁ is -CH₃. In yet other examples, R₁ is -C(O)N(H)CH₃. In certain examples, R₁ is -CF₃. In further examples, R₁ is -CN. In additional examples, R₁ is -C≡CCH₂OH.

In yet other examples, x is 1 or 2, and R₁ is -Cl, -F, -Br, -I, -OCH₃, -CH₃, -C(O)N(H)CH₃, -CF₃, -CN or -C≡CCH₂OH.

In yet other examples, Hal is -Cl, x is 1 or 2, and R₁ is -Cl, -F, -Br, -I, -OCH₃, -CH₃, -C(O)N(H)CH₃, -CF₃, -CN or -C≡CCH₂OH.

In particular examples, R₃ is -H. In certain examples, R₃ is -CH₃. In further examples, R₃ is -CH₂CH₃. In still further examples, R₃ is -CHCH₂. In other examples, R₃ is - CH₂CH₂OH. In particular examples, R₃ is -(CH₂)₂C₆H₅. In other examples, R₃ is - CH₂C(O)OH. In yet other examples, R₃ is -CH₂C(O)N(H)CH₃. In certain examples, R₃ is - CH₂C(O)N(H)((CH₂)₂N(CH₃)₂). In yet other examples, R₃ is - CH₂C(O)N(H)((CH₂)₃N(CH₃)₂). In other examples, R₃ is-CH₂C(O)N(CH₃)CH₂CN. In particular examples, R₃ is -CH₂C(O)NH₂. In certain examples, R₃ is -CH₂C(O)N(H)((CH₂)₂OH). In other examples, R₃ is -CH₂C(O)N(H)((CH₂)₂OCH₃). In still further examples, R₃ is -CH₂C(CH₃)₂OH. In yet other examples, R₃ is -CH₂C(O)OCH₃. In further examples, R₃ is -CH₂CH(OH)CH₃. In still further examples, R₃ is -CH₂CH₂OH. In particular examples, R₃ is -CH(CH₃)CH₂OH.

In further examples, R₃ is -CH₂C(O)R₄ and R₄ is 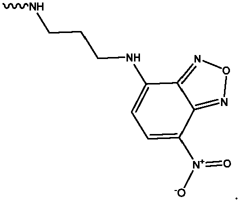 In other examples, R₃ is -CH₂C(O)R₄ and R₄ is 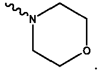 In particular examples, R₃ is -CH₂C(O)R₄ and R₄ is 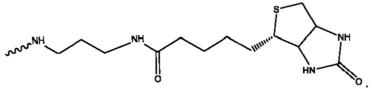 In yet other examples, R₃ is - CH₂C(O)R₄ and R₄ is 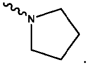 In certain examples, R₃ is -CH₂C(O)R₄ and R₄ is 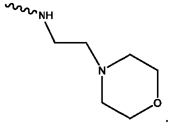 In other examples, R₃ is -CH₂C(O)R₄ and R₄ is 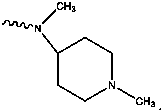 In particular examples, R₃ is -CH₂C(O)R₄ and R₄ is 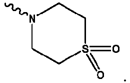 In yet other examples, R₃ is - CH₂C(O)R₄ and R₄ is 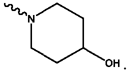 In certain examples, R₃ is -CH₂C(O)R₄ and R₄ is 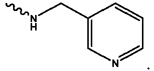 In other examples, R₃ is -CH₂C(O)R₄ and R₄ is 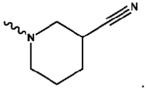 In particular examples, R₃ is -CH₂C(O)R₄ and R₄ is 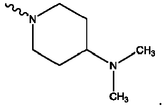 In yet other examples, R₃ is - CH₂C(O)R₄ and R₄ is 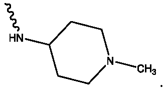 In certain examples, R₃ is -CH₂C(O)R₄ and R₄ is 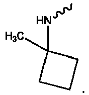 In other examples, R₃ is -CH₂C(O)R₄ and R₄ is 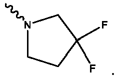 In particular examples, R₃ is -CH₂C(O)R₄ and R₄ is 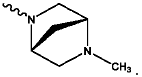 In yet other examples, R₃ is -CH₂C(O)R₄ and R₄ is 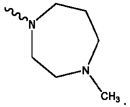 In certain examples, R₃ is -CH₂C(O)R₄ and R₄ is 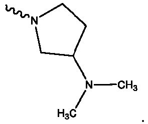 In other examples, R₃ is -CH₂C(O)R₄ and R₄ is 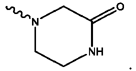 In particular examples, R₃ is -CH₂C(O)R₄ and R₄ is 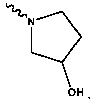 In yet other examples, R₃ is -CH₂C(O)R₄ and R₄ is 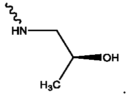 In certain examples, R₃ is -CH₂C(O)R₄ and R₄ is 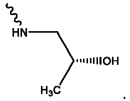 In other examples, R₃ is -CH₂C(O)R₄ and R₄ is 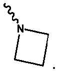 In particular examples, R₃ is -CH₂C(O)R₄ and R₄ is 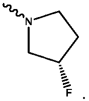 In yet other examples, R₃ is - CH₂C(O)R₄ and R₄ is 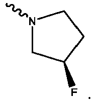 In certain examples, R₃ is -CH₂C(O)R₄ and R₄ is 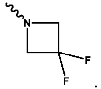 In other examples, R₃ is -CH₂C(O)R₄ and R₄ is 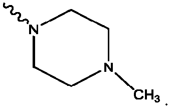 In further examples of the invention, R₃ is -CH₂C(O)R₄ and R₄ is 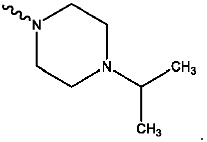 In certain examples of the invention, R₃ is -CH₂C(O)R₄ and R₄ is 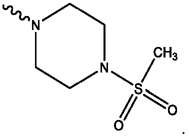 In other examples, R₃ is - CH₂C(O)R₄ and R₄ is 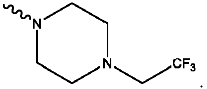 In further examples, R₃ is -CH₂C(O)R₄ and R₄ is 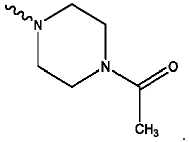 In further examples, R₃ is -CH₂C(O)R₄ and R₄ is

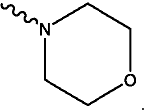

In particular examples, R₃ is -(CH₂)₂R₅ and R₅ is 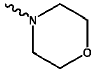 In yet other examples, R₃ is -(CH₂)₂R₅ and R₅ is 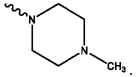 In certain examples, R₃ is -(CH₂)₂R₅ and R₅ is 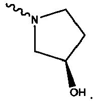 In other examples, invention, R₃ is -(CH₂)₂R₅ and R₅ is

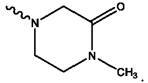

In some examples, a is an integer ranging from 0 to 5. In some examples, b is an integer ranging from 0 to 4. In some examples, c is an integer ranging from 0 to 6.

In certain examples, the compound of Formula III is Compound 3, which has the structure: 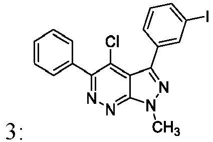 or a pharmaceutically acceptable salt thereof.

### Compounds of Formula IV

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula IV: 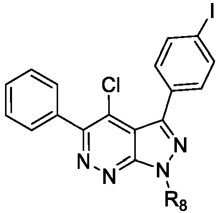 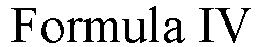 or a pharmaceutically acceptable salt thereof,
wherein R₈ is -C₁-C₃ alkyl.

In certain examples of the invention, R₈ is -CH₃, in yet further examples of the invention, R₈ is -CH₂CH₃. In other examples of the invention, R₈ is -CH₂CH₂CH₃. In other examples of the invention, R₈ is -CH(CH₃)₂.

In certain examples, the compound of Formula IV is Compound 43, which has the structure: 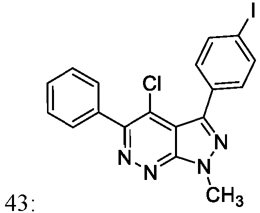 or a pharmaceutically acceptable salt thereof.

### Compounds of Formula V

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula V: 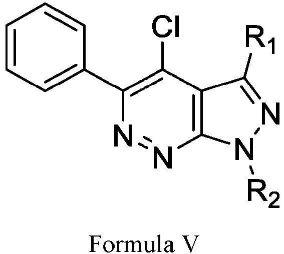 or a pharmaceutically acceptable salt thereof,
wherein R₁ is: 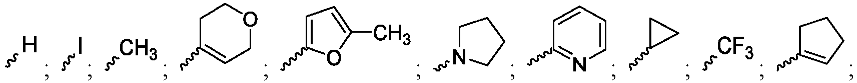 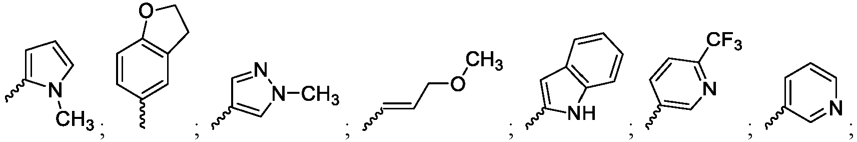 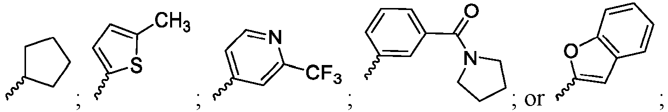
R₂ is: 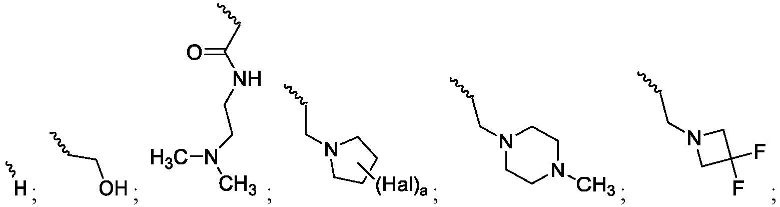 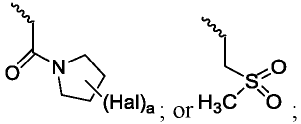
Hal is -Cl, -F, -I, or -Br; and
a is 0, 1, or 2.

In particular examples, R₁ is -I. In other examples, R₁ is -H. In yet other examples, R₁ is -CH₃. In certain examples, R₁ is -CF₃.

In yet other examples, R₁ is 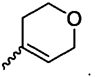 In certain examples, R₁ is 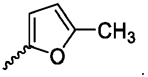 In still further examples, R₁ is 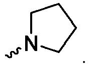 In particular examples, R₁ is 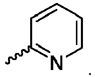 In other examples, R₁ is 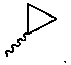 In yet other examples, R₁ is 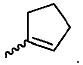 In certain examples, R₁ is 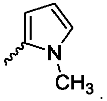 In particular examples, R₁ is 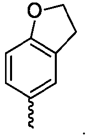 In certain examples, R₁ is 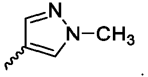 In still further examples, R₁ is 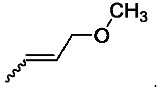 In other examples, R₁ is

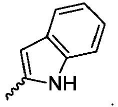

In yet other examples, R₁ is 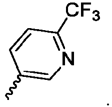 In certain examples, R₁ is 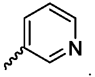 In still further examples, R₁ is 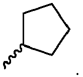 In other examples, R₁ is 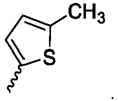 In particular examples, R₁ is 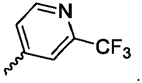 In further examples, R₁ is 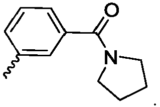 In still further examples, R₁ is

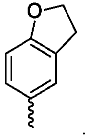

In certain examples, R₂ is -H. In yet other examples, R₂ is 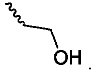 In particular examples, R₂ is 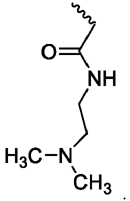 In yet other examples, R₂ is 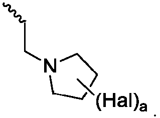 In further examples, R₂ is 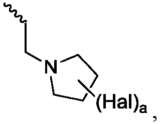 a = 1, and Hal is -F.

In certain examples, R₂ is 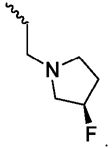 In still further examples, R₂ is 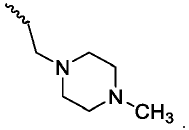 In particular examples, R₂ is 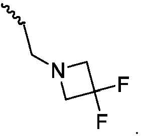 In other examples, R₂ is 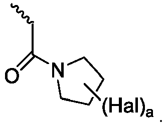 In yet other examples, R₂ is 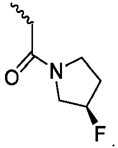 In certain examples, R₂ is

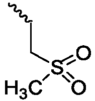

In further examples, when a is 2, each Hal is the same or different.

In certain examples the compound of Formula V has the structure: 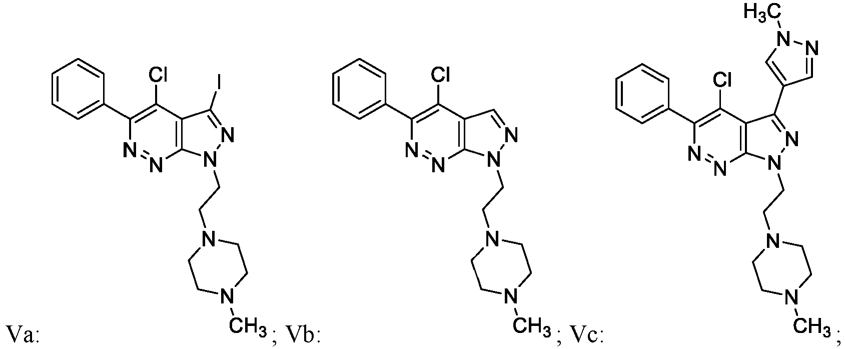 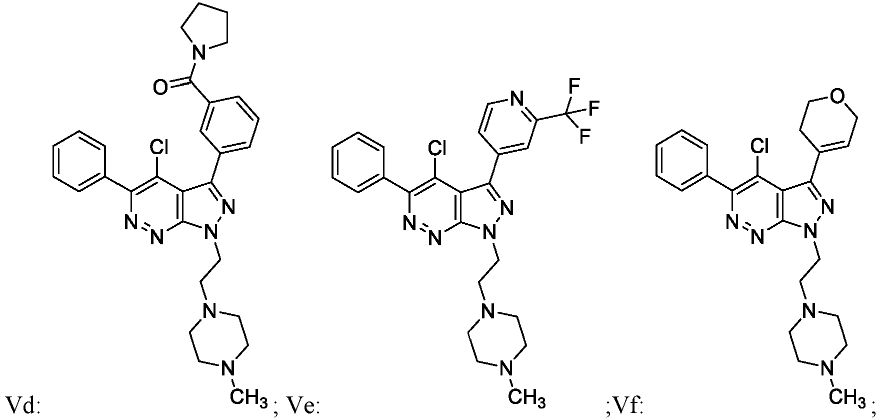 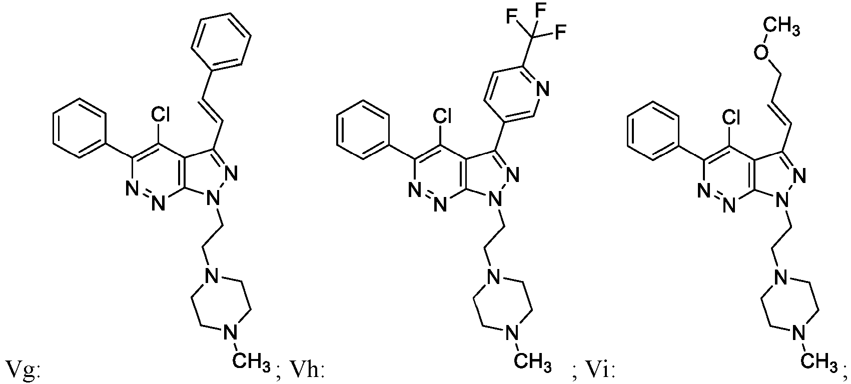 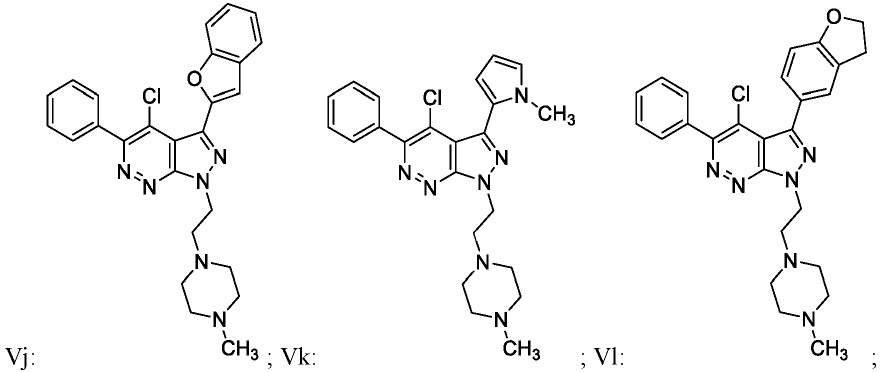 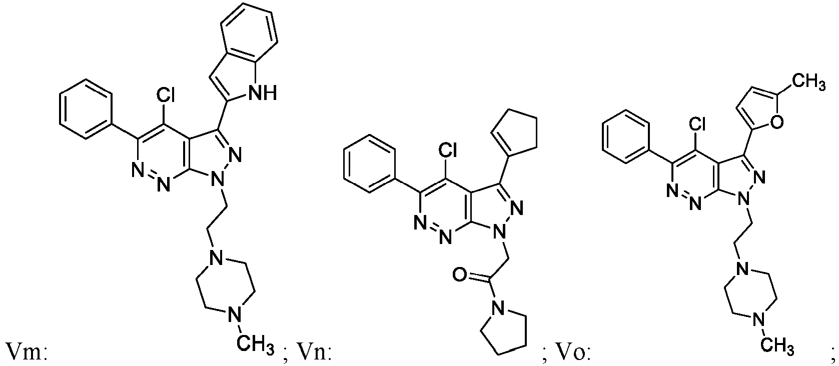 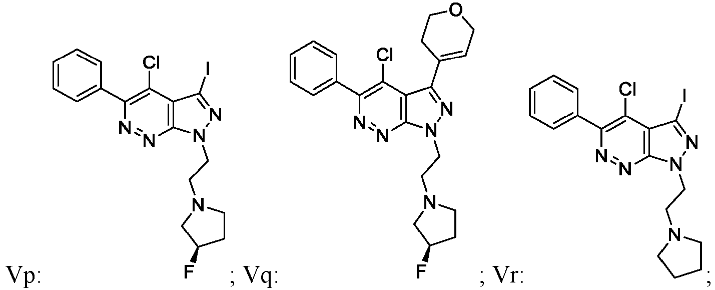 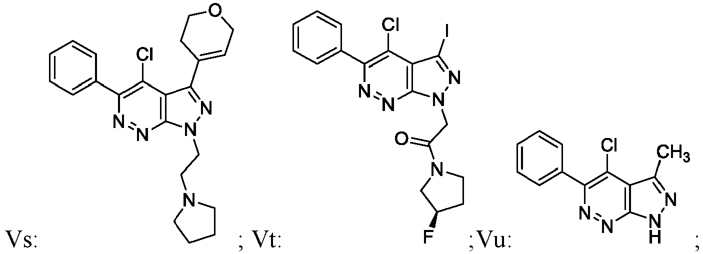 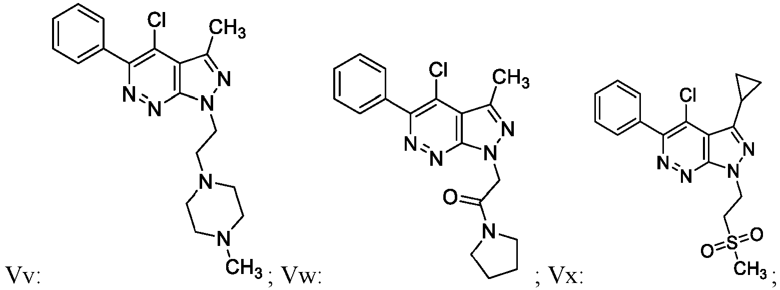 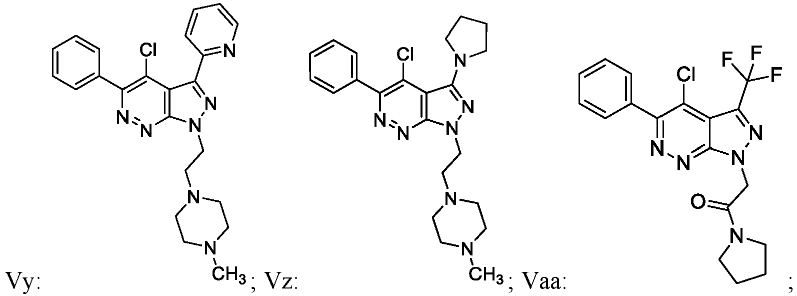 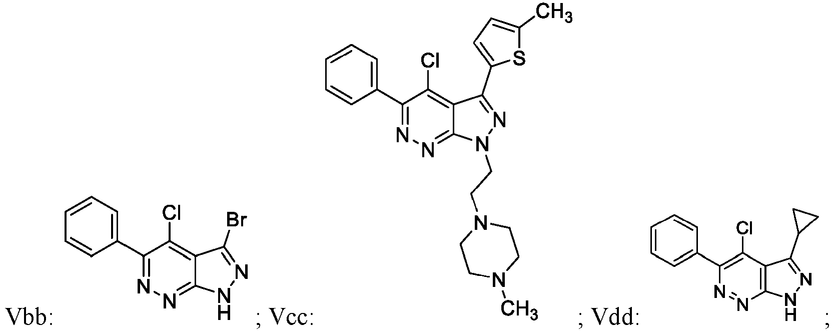 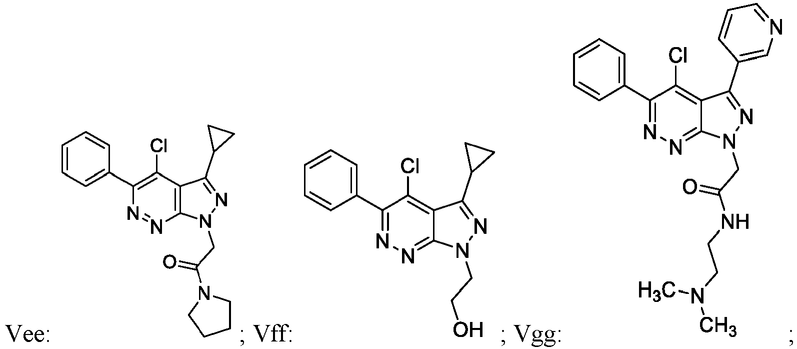 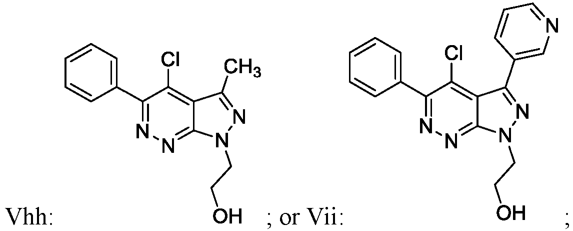 or a pharmaceutically acceptable salt thereof.

### Compounds of Formula VI

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula VI: 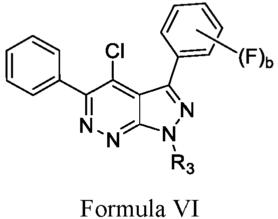 or a pharmaceutically acceptable salt thereof,
wherein R₃ is: 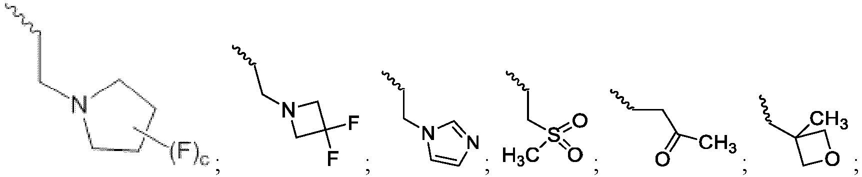 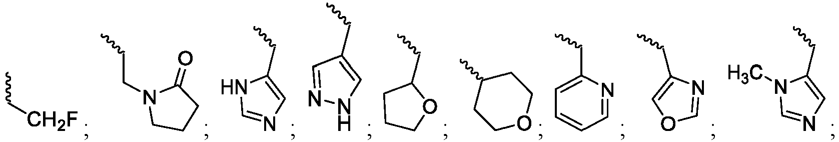 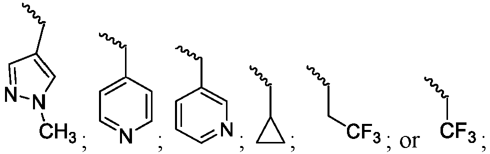
b is 0 or 1; and
c is 1 or 2.

In particular examples, b is 0. In other examples b is 1 and the -F is in the meta position relative to the pyrazolopyridazino ring system. In yet other examples b is 1 and the - F is in the para position relative to the pyrazolopyridazino ring system.

In particular examples R₃ is -CF₃. In certain examples R₃ is 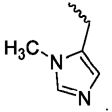 In other examples R₃ is 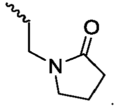 In yet other examples R₃ is 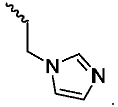 In further examples R₃ is 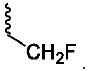 In still further examples R₃ is 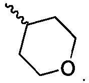 In particular examples R₃ is

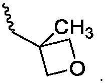

In other examples R₃ is 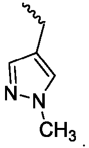 In yet other examples R₃ is 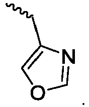 In certain examples R₃ is 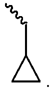 In further examples R₃ is 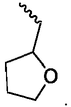 In further examples R₃ is

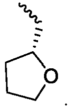

In certain examples R₃ is 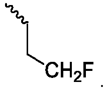 In other examples R₃ is 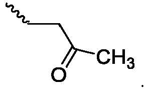 In yet other examples R₃ is 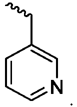 In further examples R₃ is 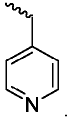 In still further examples R₃ is 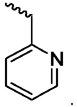 In particular examples R₃ is

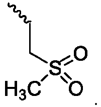

In certain examples R₃ is 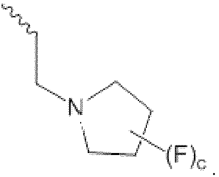 In further examples R₃ is 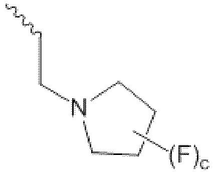 and c = 1. In still further examples R₃ is 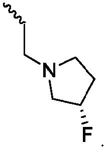 In particular examples R₃ is 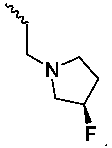 In other examples R₃ is 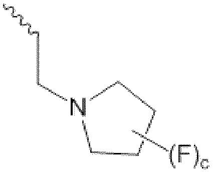 and c = 2. In yet other examples R₃ is

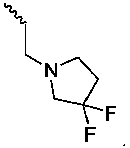

In certain examples R₃ is 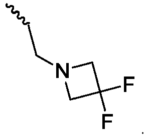 In other examples R₃ is 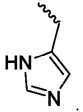 In yet other examples R₃ is

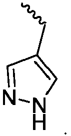

In certain examples the compound of Formula VI has the structure: 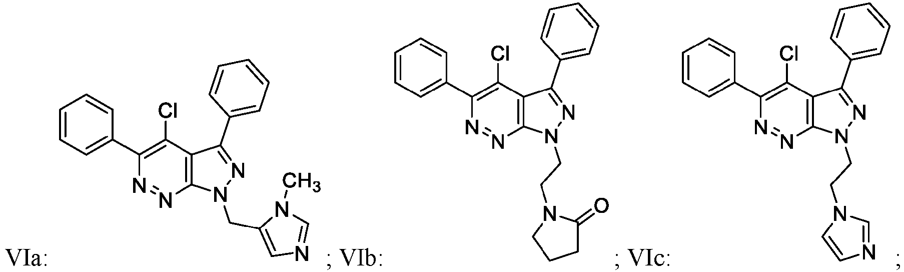 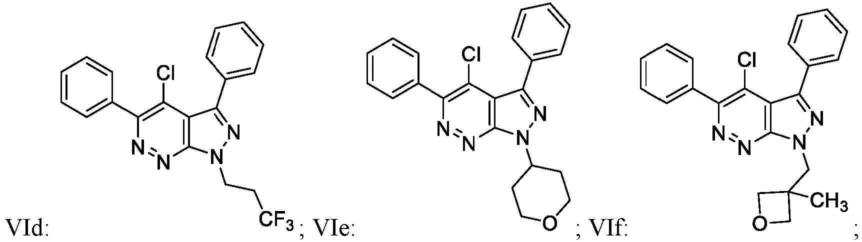 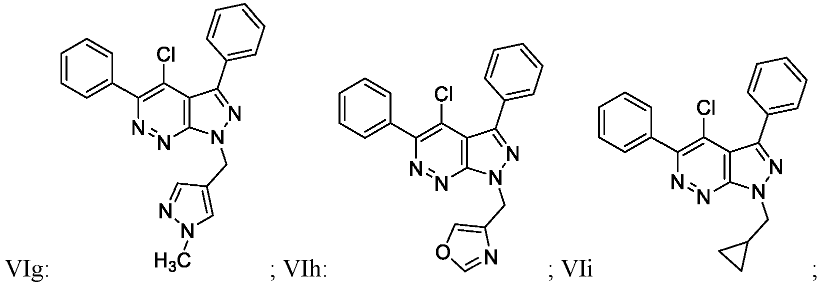 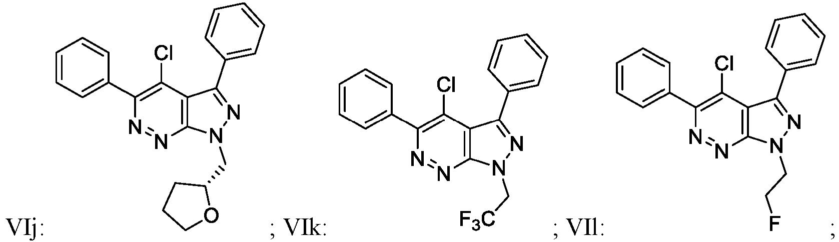 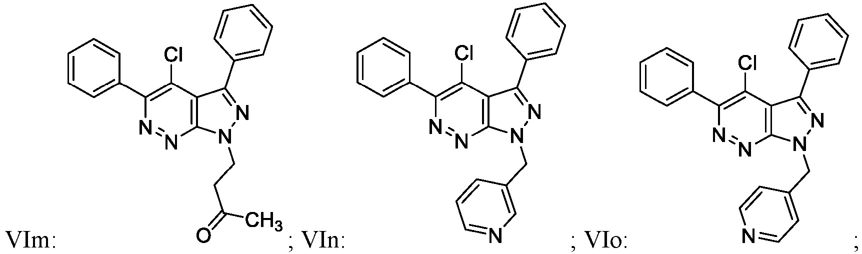 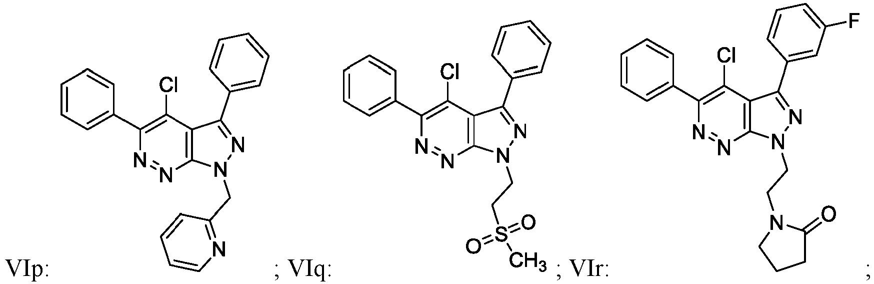 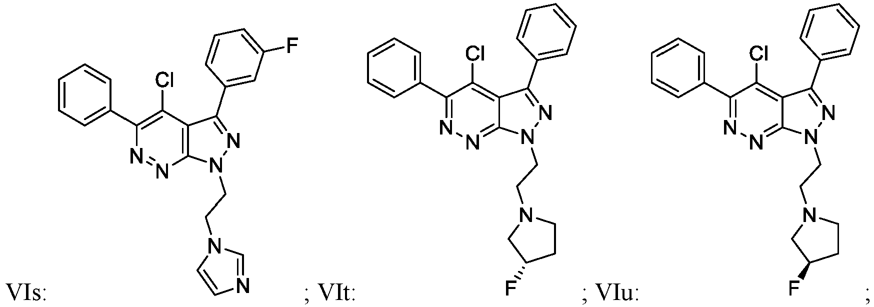 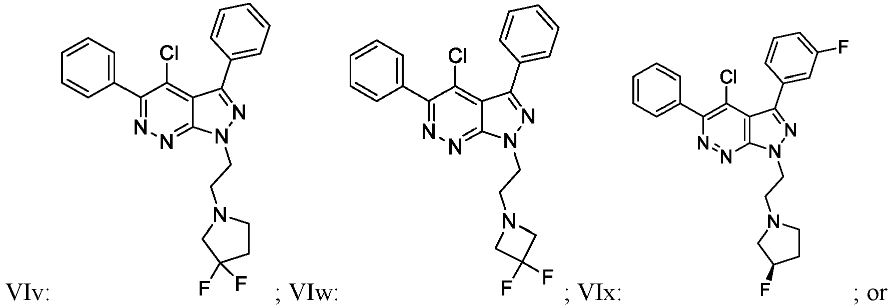 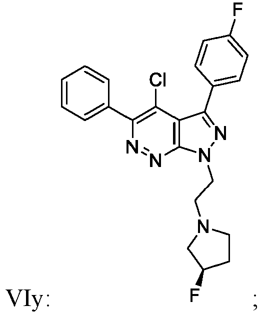 or a pharmaceutically acceptable salt thereof.

### Compounds of Formula VII

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula VII: 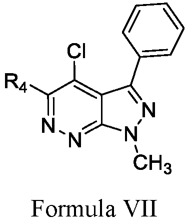 or a pharmaceutically acceptable salt thereof,
wherein R₄ is -I;

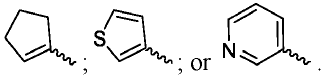

In certain examples R₄ is -I (iodo). In particular examples R₄ is 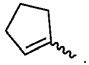 In other examples R₄ is 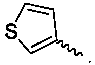 In yet other examples R₄ is

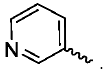

In certain examples the compound of Formula VII has the structure: 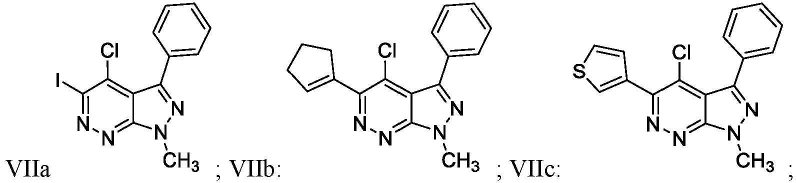 or 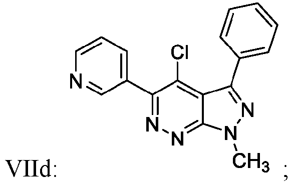 or a pharmaceutically acceptable salt thereof.

### Compounds of Formula XIII

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula XIII: 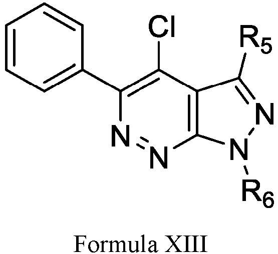 or a pharmaceutically acceptable salt thereof,
wherein R₅ is: 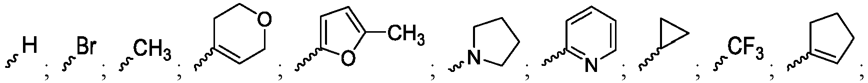 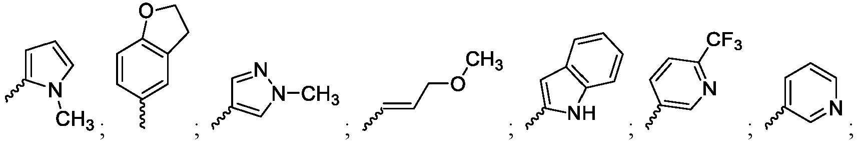 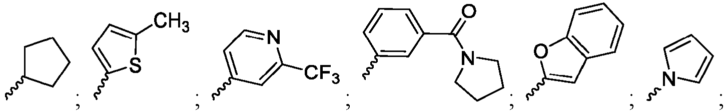 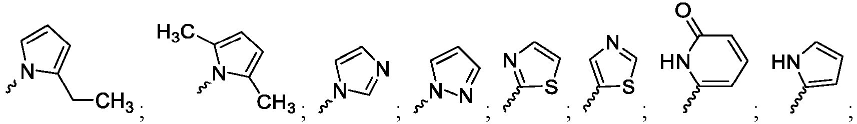 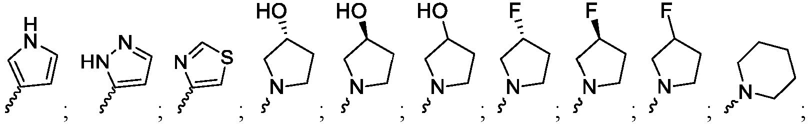 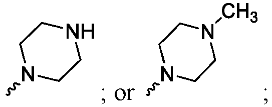
R₆ is: 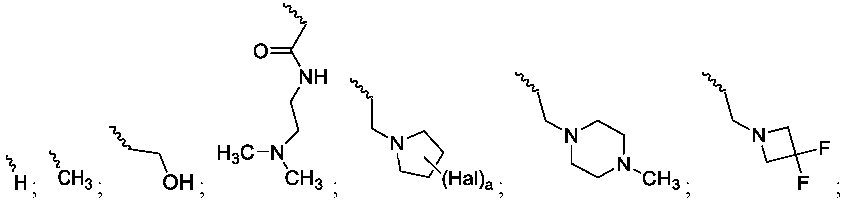 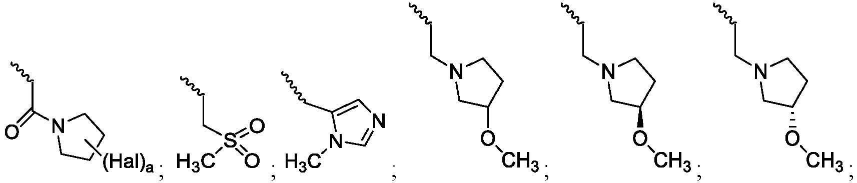 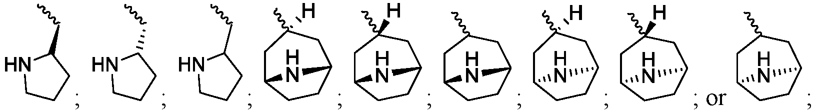
Hal is -Cl, -F, -I, or -Br; and
a is 0, 1, or 2.

In particular examples, R₅ is -I. In other examples, R₅ is -H. In yet other examples, R₅ is -CH₃. In certain examples, R₅ is -CF₃.

In yet other examples, R₅ is 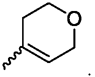 In certain examples, R₅ is 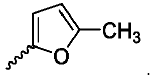 In still further examples, R₅ is 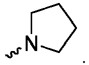 In particular examples, R₅ is 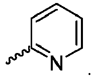 In other examples, R₅ is 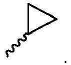 In yet other examples, R₅ is 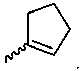 In certain examples, R₅ is 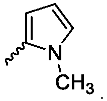 In particular examples, R₅ is 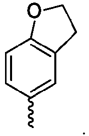 In certain examples, R₅ is 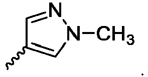 In still further examples, R₅ is 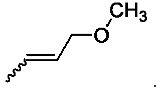 In other examples, R₅ is

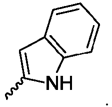

In yet other examples, R₅ is 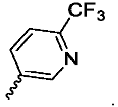 In certain examples, R₅ is 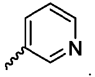 In still further examples, R₅ is 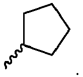 In other examples, R₅ is 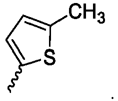 In particular examples, R₅ is 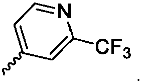 In further examples, R₅ is 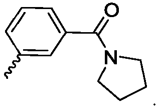 In still further examples, R₅ is

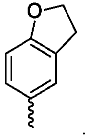

In certain examples, R₅ is 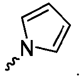 In further examples, R₅ is 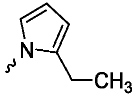 In further examples, R₅ is 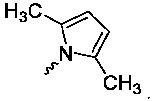 In other examples, R₅ is 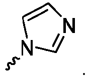 In yet other examples, R₅ is 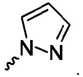 In particular examples, R₅ is

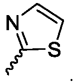

In further examples, R₅ is 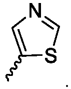 In still further examples, R₅ is

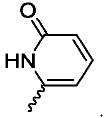

In certain examples, R₅ is 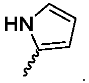 In other examples, R₅ is 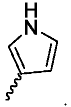 In yet other examples, R₅ is 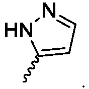 In particular examples, R₅ is

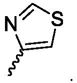

In further examples, R₅ is 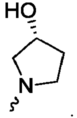 In still further examples, R₅ is

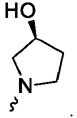

In certain examples, R₅ is 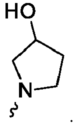 In other examples, R₅ is 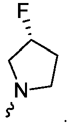 In yet other examples, R₅ is 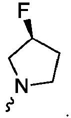 In particular examples, R₅ is

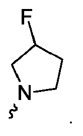

In further examples, R₅ is 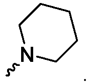 In still further examples, R₅ is

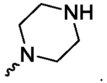

In certain examples, R₅ is

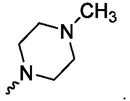

In certain examples, R₆ is 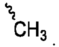 In further examples, R₆ is 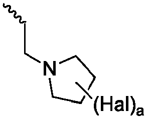 and a = 0. In other examples, the compound of Formula XIII is a pharmaceutically acceptable salt and R₆ is 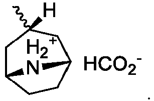 In yet other examples, the compound of Formula XIII is a pharmaceutically acceptable salt and R₆ is 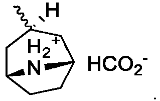 In particular examples, the compound of Formula XIII is a pharmaceutically acceptable salt and R₆ is 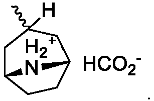 In certain examples, the compound of Formula XIII is a pharmaceutically acceptable salt and R₆ is 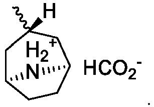 In further examples, the compound of Formula XIII is a pharmaceutically acceptable salt and R₆ is 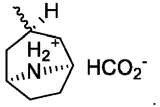 In other examples, the compound of Formula XIII is a pharmaceutically acceptable salt and R₆ is 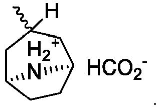 In certain examples, R₆ is 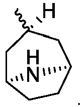 In further examples, R₆ is 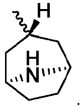 In particular examples, R₆ is

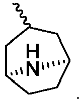

In further examples, R₆ is 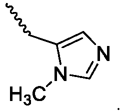 In still further examples, R₆ is 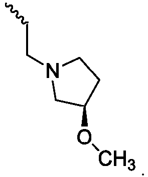 In other examples, R₆ is 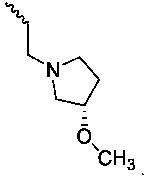 In certain examples, R₆ is 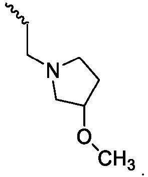 In yet other examples, R₆ is 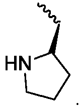 In particular examples, R₆ is 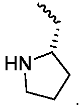 In further examples, R₆ is 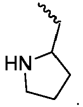 In still further examples, R₆ is

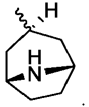

In certain examples, R₆ is 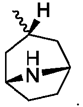 In other examples, R₆ is

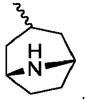

In further examples, when a is 2, each Hal is the same or different.

In certain examples the compound of Formula XIII has the structure: 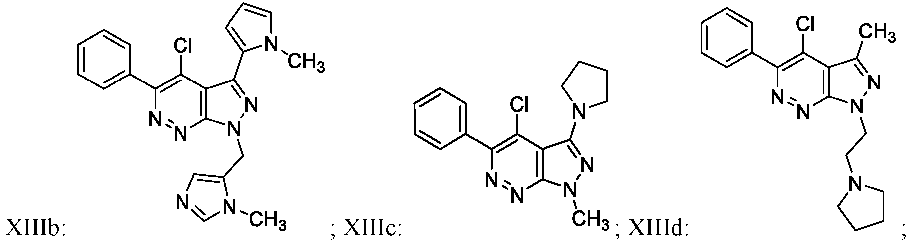 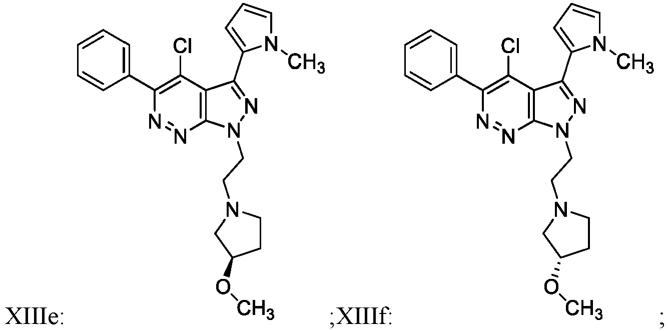 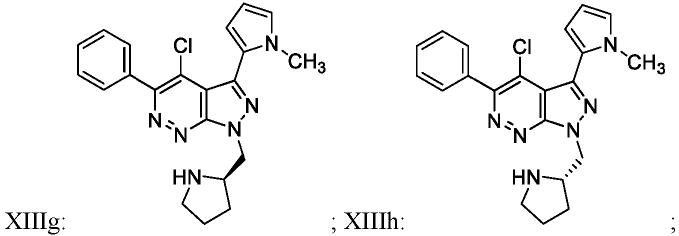 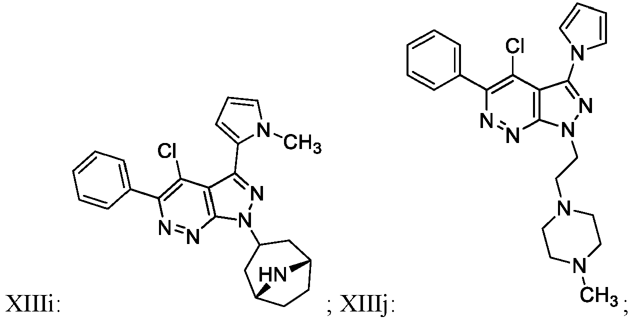 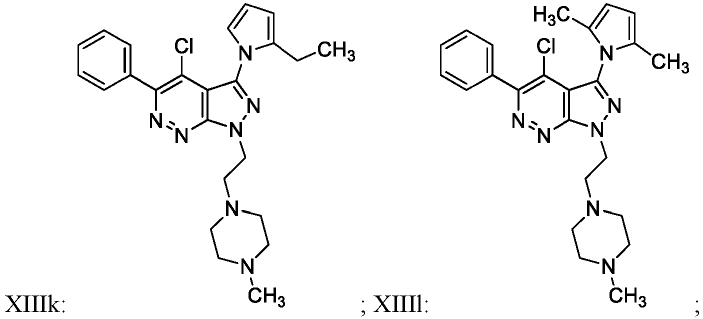 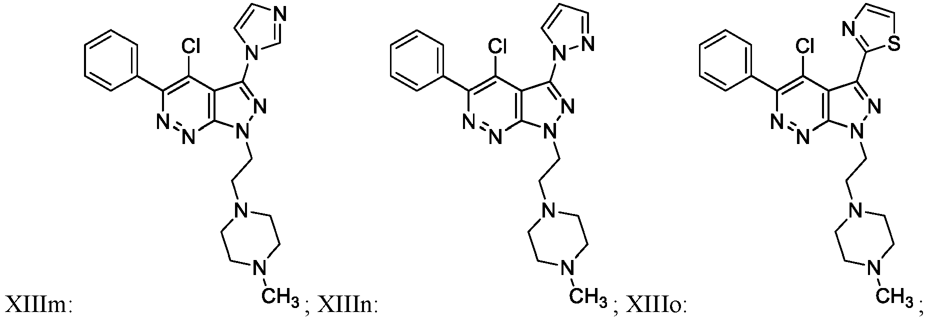 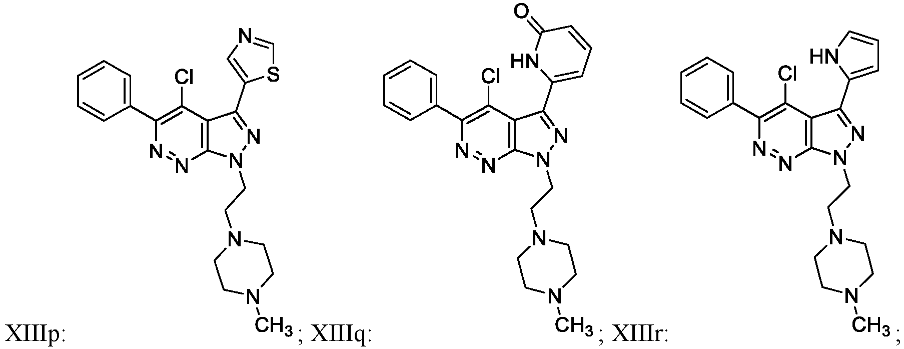 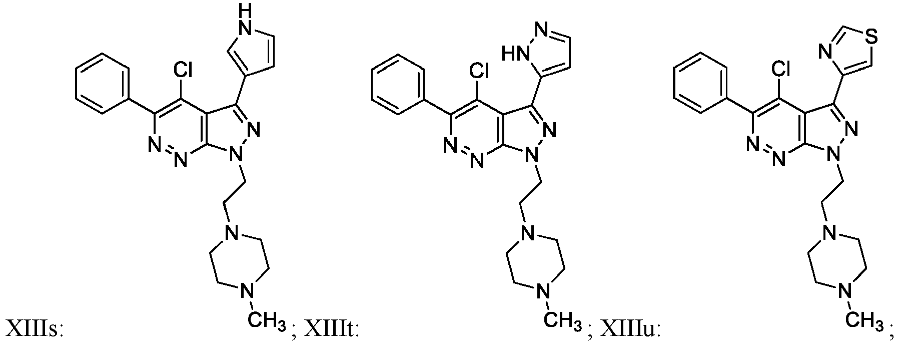 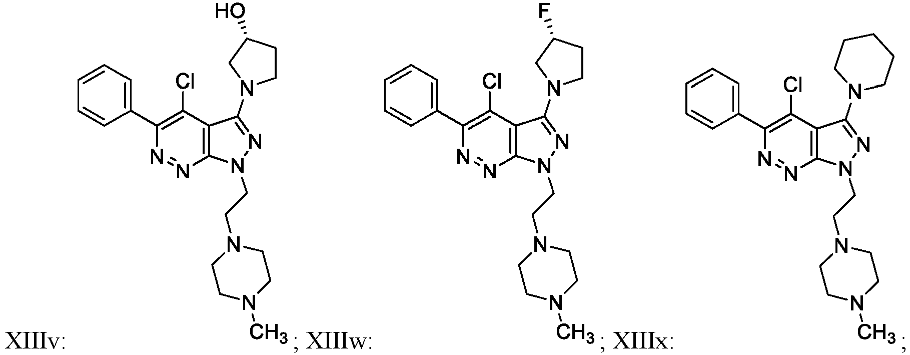 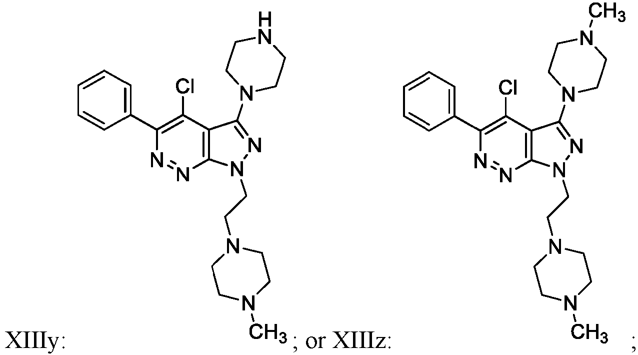 or a pharmaceutically acceptable salt thereof.

In other examples, the compound of Formula XIII is a pharmaceutically acceptable salt and has the structure:

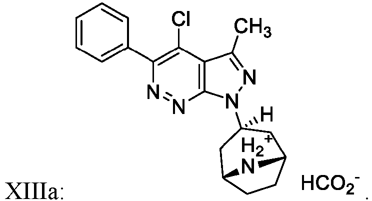

### Compounds of Formula XIV

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula XIV: 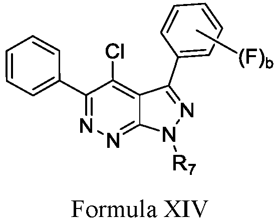 or a pharmaceutically acceptable salt thereof,
wherein R₇ is: 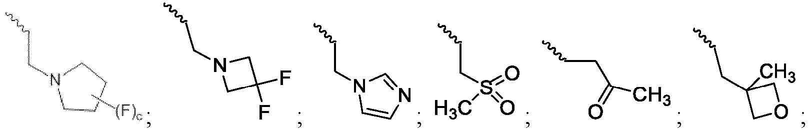 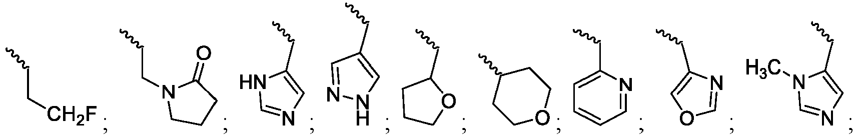 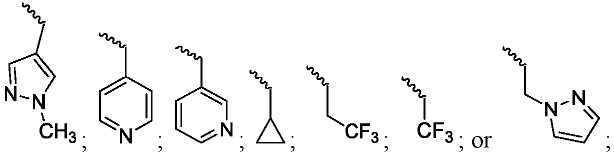
b is 0 or 1; and
c is 1 or 2.

In particular examples, b is 0. In other examples b is 1 and the -F is in the meta position relative to the pyrazolopyridazino ring system. In yet other examples b is 1 and the - F is in the para position relative to the pyrazolopyridazino ring system.

In particular examples R₇ is -CF₃. In certain examples R₇ is 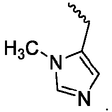 In other examples R₇ is 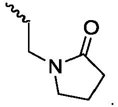 In yet other examples R₇ is 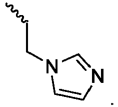 In further examples R₇ is 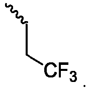 In still further examples R₇ is 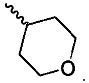 In particular examples R₇ is

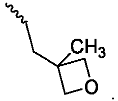

In other examples R₇ is 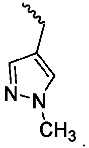 In yet other examples R₇ is 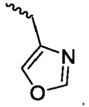 In certain examples R₇ is 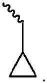 In further examples R₇ is 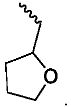 In further examples R₇ is

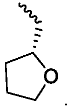

In certain examples R₇ is 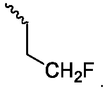 In other examples R₇ is 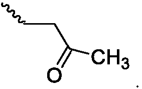 In yet other examples R₇ is 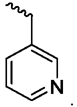 In further examples R₇ is 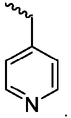 In still further examples R₇ is 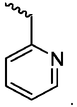 In particular examples R₇ is

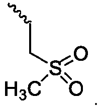

In certain examples R₇ is 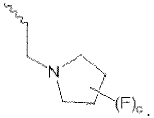 In further examples R₇ is 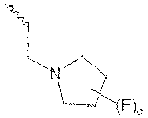 and c = 1. In still further examples R₇ is 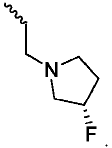 In particular examples R₇ is 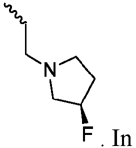 other examples R₇ is 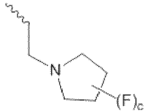 and c = 2. In yet other examples R₇ is

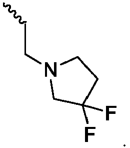

In certain examples R₇ is 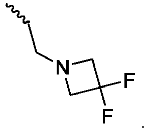 In other examples R₇ is 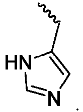 In yet other examples R₇ is 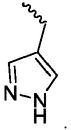 In yet other examples R₇ is

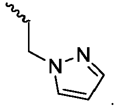

In certain examples the compound of Formula XIV has the structure: 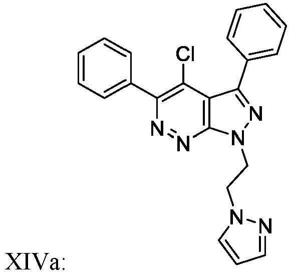 or a pharmaceutically acceptable salt thereof.

### Compounds of Formula XV

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound of Formula XV: 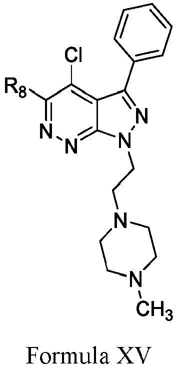 or a pharmaceutically acceptable salt thereof,
wherein R₈ is:

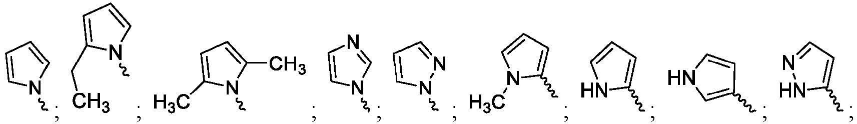

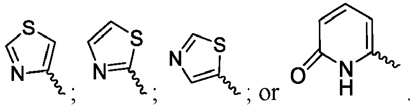

In particular examples R₈ is 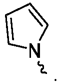 In certain examples R₈ is 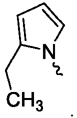 In other examples R₈ is 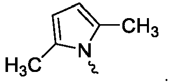 In yet other examples R₈ is 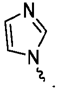 In further examples R₈ is 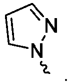 In still further examples R₈ is 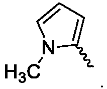 In particular examples R₈ is

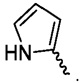

In particular examples R₈ is 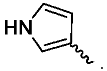 In certain examples R₈ is 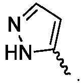 In other examples R₈ is 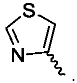 In yet other examples R₈ is 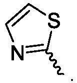 In further examples R₈ is 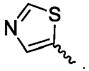 In still further examples R₈ is

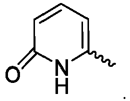

In certain examples the compound of Formula XV has the structure: 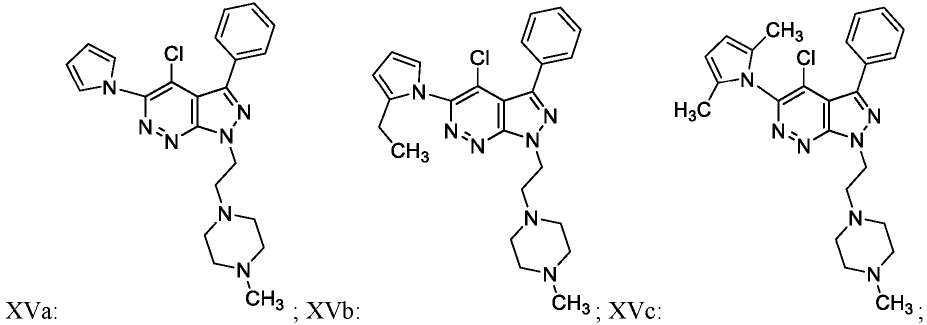 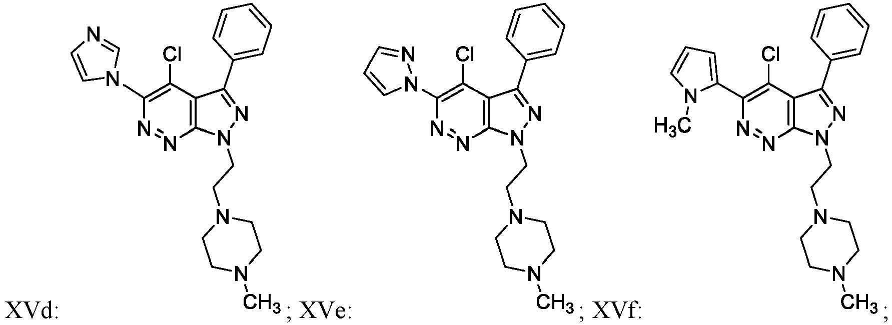 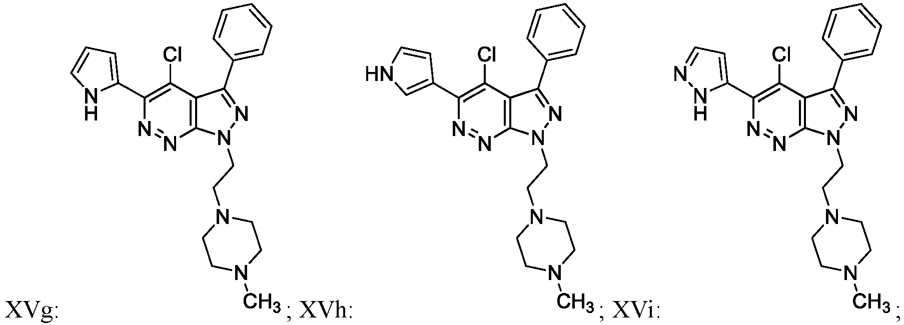 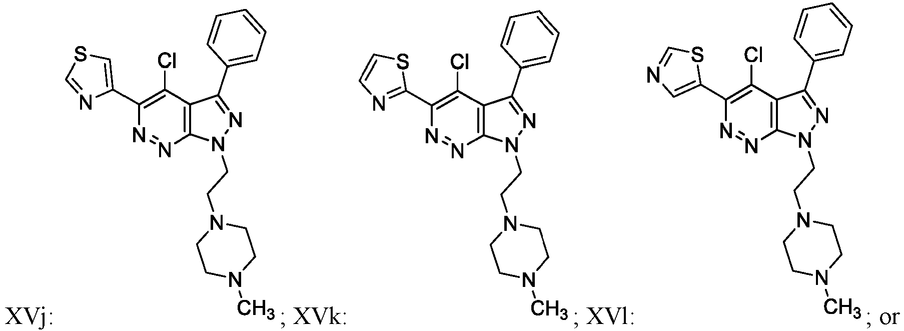 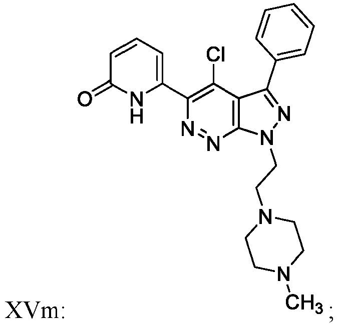 or a pharmaceutically acceptable salt thereof.

### Other Compounds

Also disclosed herein are methods for treating or preventing cancer, an inflammatory disease or an autoimmune disease, or for reducing risk of developing cancer, an inflammatory disease or an autoimmune disease, comprising administering to a subject in need thereof an effective amount of a compound having the structure: 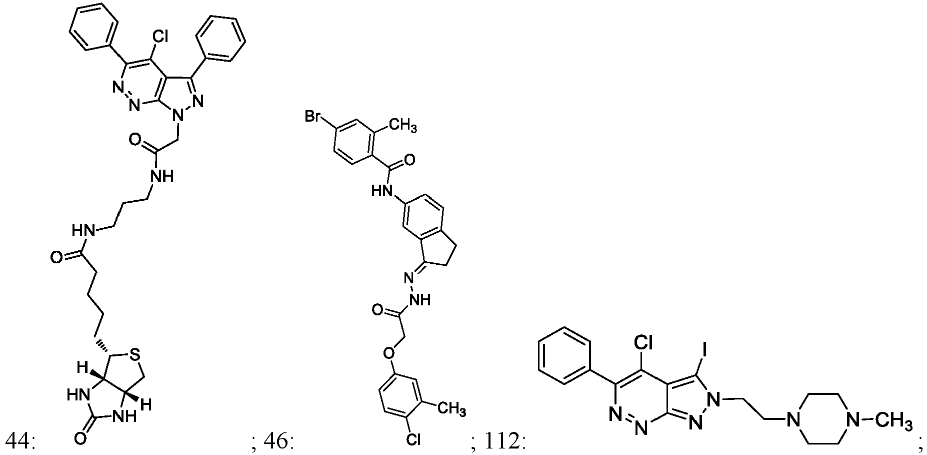 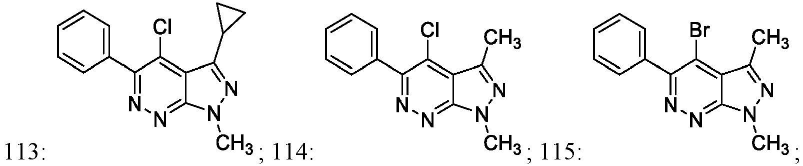 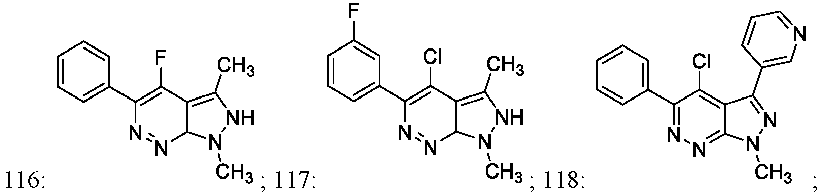 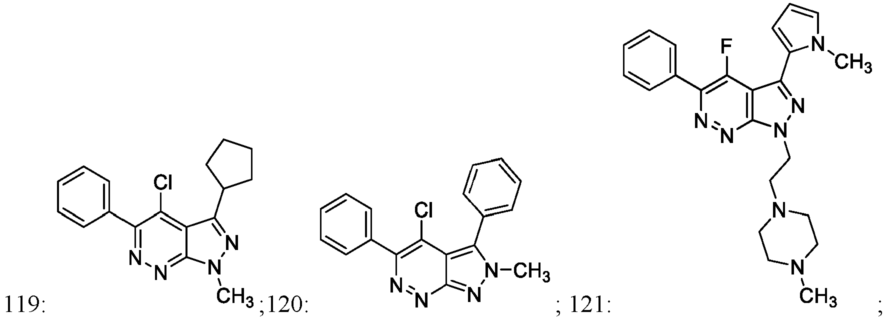 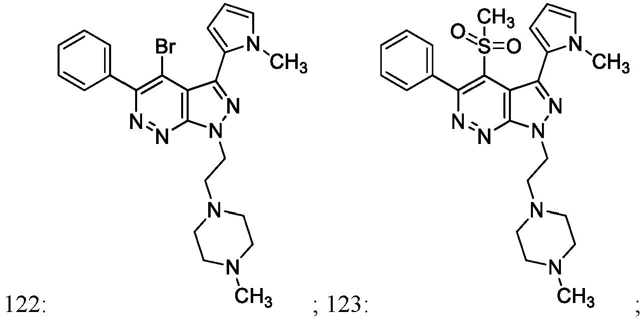 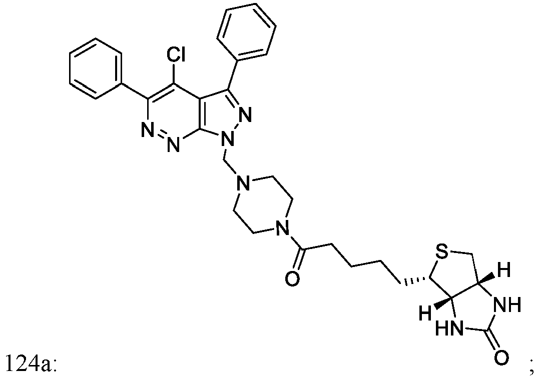 or 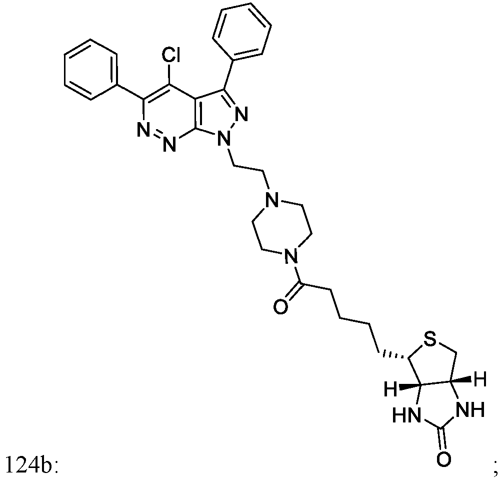 or a pharmaceutically acceptable salt thereof.

### Non-Pyrazolopyridazine Compounds

A compound or a pharmaceutically acceptable salt of the compound of Table 1 below is a non-pyrazolopyridazine compound.

Also disclosed herein are non-pyrazolopyridazine compounds, and methods for treating or preventing cancer, an inflammatory disease or an autoimmune disease, or for reducing risk of developing cancer, an inflammatory disease or an autoimmune disease, comprising administering to a subject in need thereof an effective amount of a non-Pyrazolopyridazine compound or a pharmaceutically acceptable salt thereof; and a pharmaceutically acceptable carrier or vehicle.

**Table 1. Non-Pyrazolopyridazine compounds of the invention**

| **Compound No.** | **Structure** |
|---|---|
| **125** | 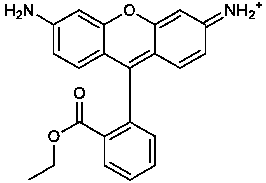 |
| **126** | 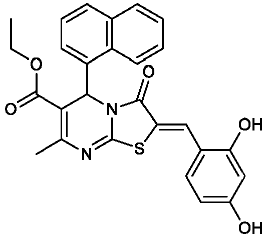 |
| **127** | 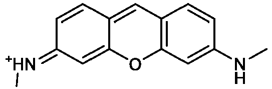 |
| **128** | 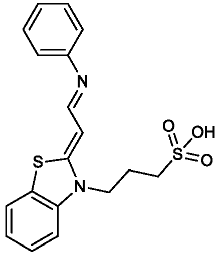 |
| **129** | 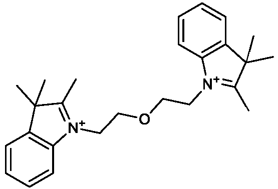 |
| **130** | 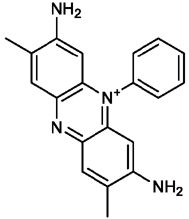 |
| **131** | 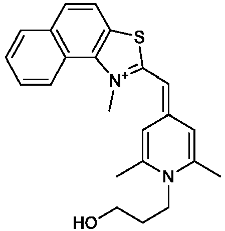 |
| **132** | 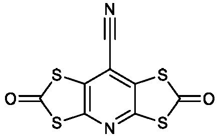 |
| **133** | 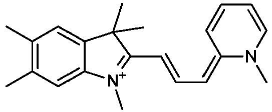 |
| **134** | 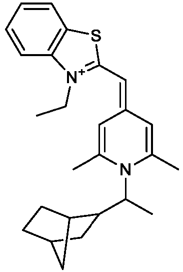 |
| **135** | 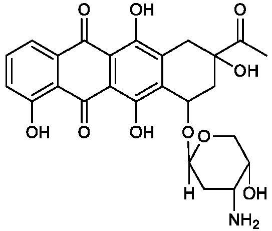 |
| **136** | 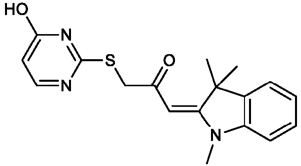 |
| **137** | 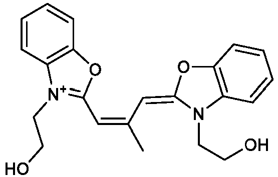 |
| **138** | 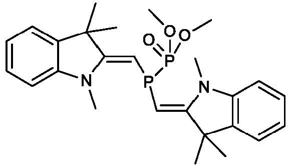 |
| **139** | 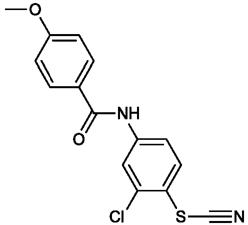 |
| **140** | 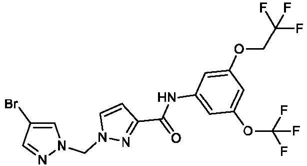 |
| **141** | 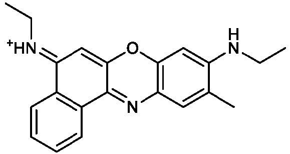 |
| **142** | 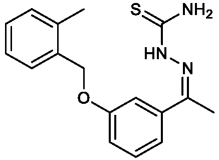 |
| **143** | 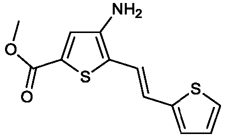 |
| **144** | 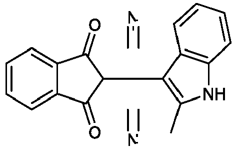 |
| **145** | 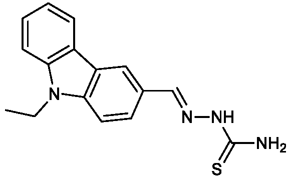 |
| **146** | 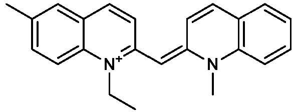 |
| **147** | 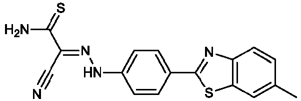 |
| **148** | 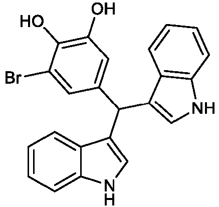 |
| **149** | 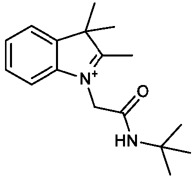 |
| **150** | 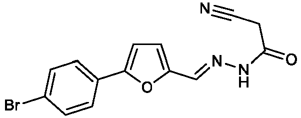 |
| **151** | 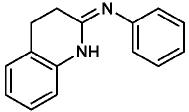 |
| **152** | 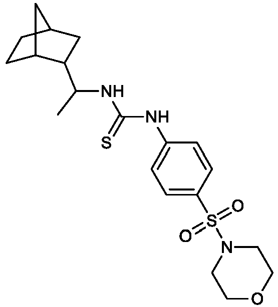 |
| **153** | 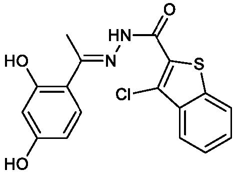 |
| **154** | 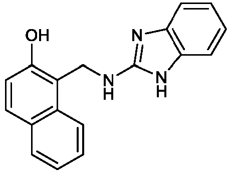 |
| **155** | 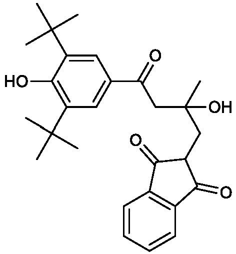 |
| **156** | 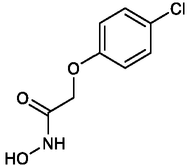 |
| **157** | 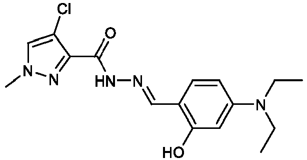 |
| **158** | 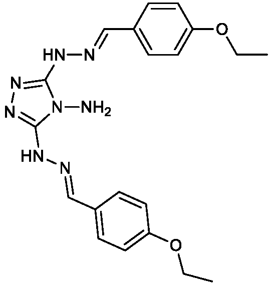 |
| **159** | 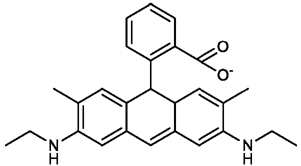 |
| **160** | 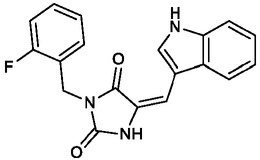 |
| **161** | 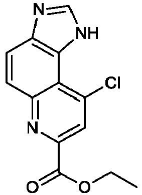 |
| **162** | 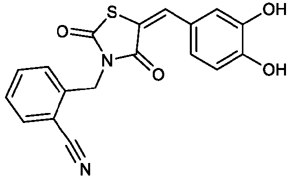 |
| **163** | 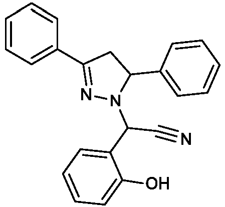 |
| **164** | 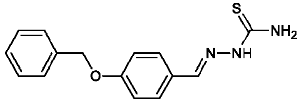 |
| **165** | 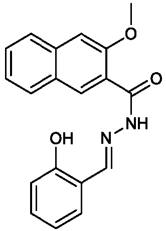 |
| **166** | 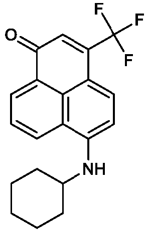 |
| **167** | 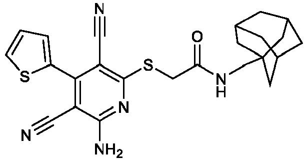 |
| **168** | 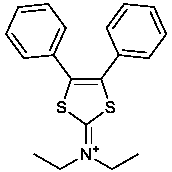 |
| **169** | 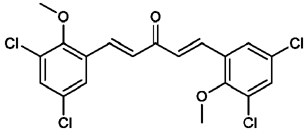 |
| **170** | 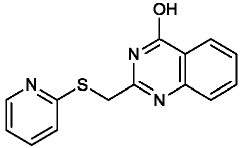 |
| **171** | 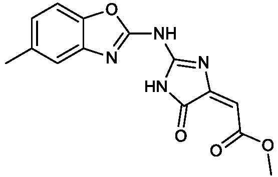 |
| **172** | 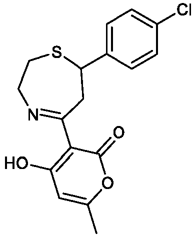 |
| **173** | 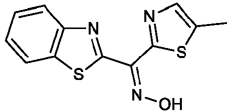 |
| **174** | 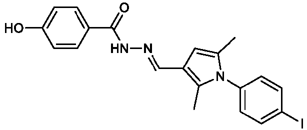 |
| **175** | 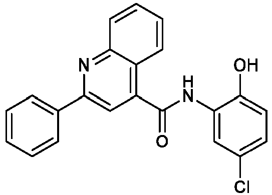 |
| **176** | 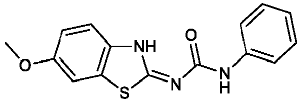 |
| **177** | 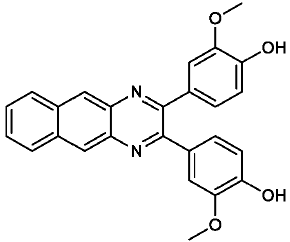 |
| **178** | 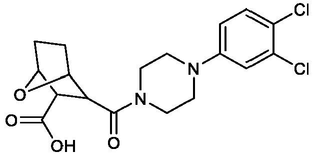 |
| **179** | 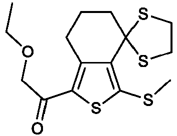 |
| **180** | 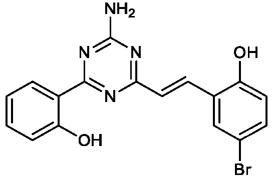 |
| **181** | 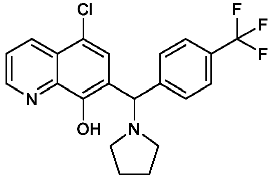 |
| **182** | 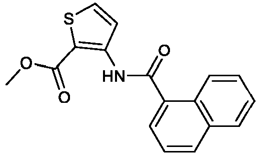 |
| **183** | 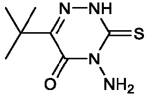 |
| **184** | 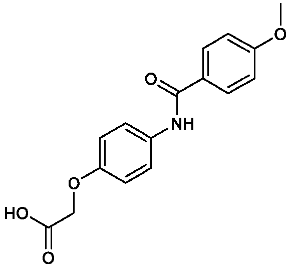 |
| **185** | 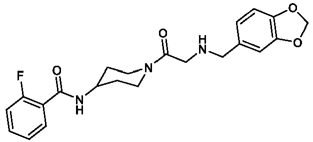 |
| **186** | 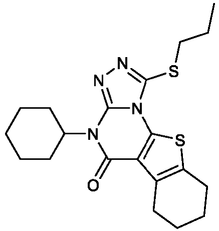 |
| **187** | 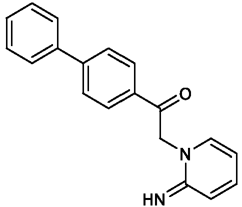 |
| **188** | 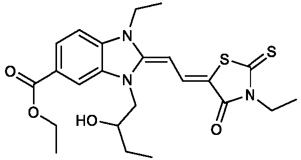 |
| **189** | 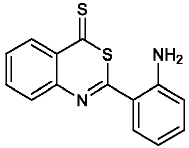 |
| **190** | 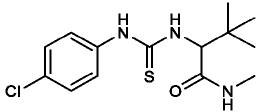 |
| **191** | 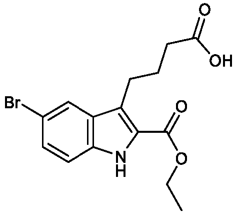 |
| **192** | 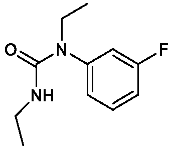 |
| **193** | 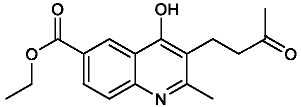 |
| **194** | 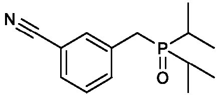 |
| **195** | 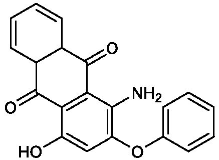 |
| **196** | 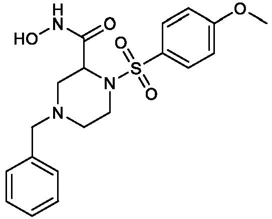 |
| **197** | 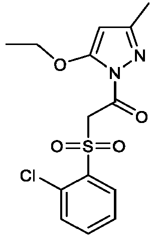 |
| **198** | 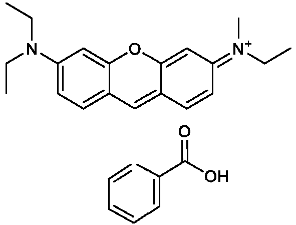 |
| **199** | 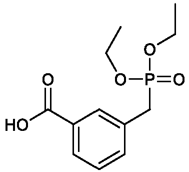 |
| **200** | 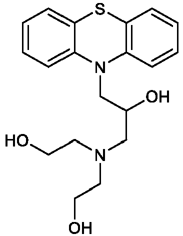 |
| **201** | 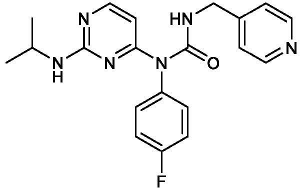 |
| **202** | 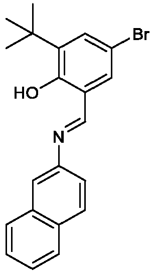 |
| **203** | 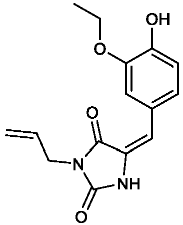 |
| **204** | 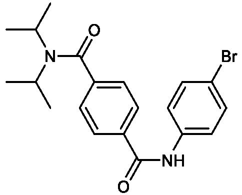 |
| **205** | 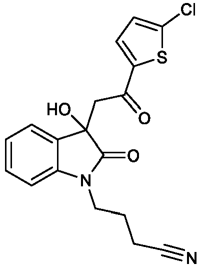 |
| **206** | 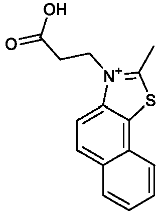 |
| **207** | 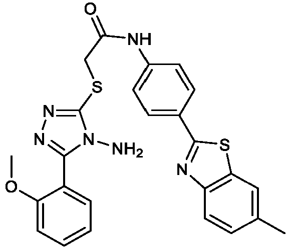 |
| **208** | 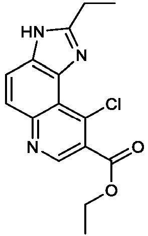 |
| **209** | 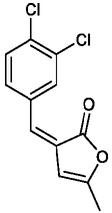 |
| **210** | 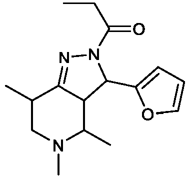 |
| **211** | 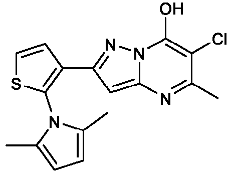 |
| **212** | 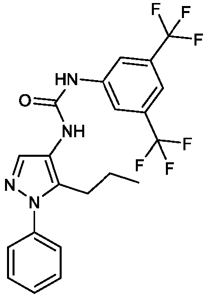 |
| **213** | 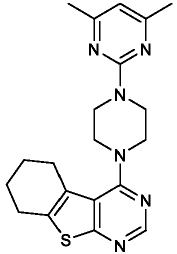 |

Some of the compounds disclosed herein, for example, Compounds 44, 63, 72, 74, 83, 88, 89, 98-101, 111, 124a, 124b, Vp, Vq, Vt, VIj, VIt, VIu, VIx, VIy, XIIIa, XIIIe, XIIIf, XIIIg, XIIIh, XIIIi, XIIIv, and XIIIw are depicted having a bold or hatched wedge, indicating absolute stereochemistry.

In some examples, each of one or more hydrogen atoms of a compound of the disclosure is replaced with a deuterium atom.

The compounds of the disclosure can be in the form of a salt, for example a pharmaceutically acceptable salt. The compound of the invention (Compound 85) can be in the form of a pharmaceutically acceptable salt. Pharmaceutically acceptable salts include, for example, acid-addition salts and base-addition salts. The acid that forms an acid-addition salt can be an organic acid or an inorganic acid. A base that forms a base-addition salt can be an organic base or an inorganic base. In some embodiments, a pharmaceutically acceptable salt is a metal salt. In some embodiments, a pharmaceutically acceptable salt is an ammonium salt.

Acid-addition salts can arise from the addition of an acid to the free-base form of a compound. The acid may be organic or may be inorganic. Non-limiting examples of suitable acids include hydrochloric acid, hydrobromic acid, hydroiodic acid, nitric acid, nitrous acid, sulfuric acid, sulfurous acid, a phosphoric acid, nicotinic acid, isonicotinic acid, lactic acid, salicylic acid, 4-aminosalicylic acid, tartaric acid, ascorbic acid, gentisinic acid, gluconic acid, glucaronic acid, saccaric acid, formic acid, benzoic acid, glutamic acid, pantothenic acid, acetic acid, propionic acid, butyric acid, fumaric acid, succinic acid, citric acid, oxalic acid, maleic acid, hydroxymaleic acid, methylmaleic acid, glycolic acid, malic acid, cinnamic acid, mandelic acid, 2-phenoxybenzoic acid, 2-acetoxybenzoic acid, embonic acid, phenylacetic acid, N-cyclohexylsulfamic acid, methanesulfonic acid, ethanesulfonic acid, benzenesulfonic acid, p-toluenesulfonic acid, 2-hydroxyethanesulfonic acid, ethane-1,2-disulfonic acid, 4-methylbenzenesulfonic acid, naphthalene-2-sulfonic acid, naphthalene-1,5-disulfonic acid, 2-phosphoglyceric acid, 3-phosphoglyceric acid, glucose-6-phosphoric acid, and an amino acid.

Non-limiting examples of suitable acid-addition salts include a hydrochloride salt, a hydrobromide salt, a hydroiodide salt, a nitrate salt, a nitrite salt, a sulfate salt, a sulfite salt, a phosphate salt, a hydrogen phosphate salt, a dihydrogen phosphate salt, a carbonate salt, a bicarbonate salt, a nicotinate salt, an isonicotinate salt, a lactate salt, a salicylate salt, a 4-aminosalicylate salt, a tartrate salt, an ascorbate salt, a gentisinate salt, a gluconate salt, a glucaronate salt, a saccarate salt, a formate salt, a benzoate salt, a glutamate salt, a pantothenate salt, an acetate salt, a propionate salt, a butyrate salt, a fumarate salt, a succinate salt, a citrate salt, an oxalate salt, a maleate salt, a hydroxymaleate salt, a methylmaleate salt, a glycolate salt, a malate salt, a cinnamate salt, a mandelate salt, a 2-phenoxybenzoate salt, a 2-acetoxybenzoate salt, an embonate salt, a phenylacetate salt, an N-cyclohexylsulfamate salt, a methanesulfonate salt, an ethanesulfonate salt, a benzenesulfonate salt, a p-toluenesulfonate salt, a 2-hydroxyethanesulfonate salt, an ethane-1,2-disulfonate salt, a 4-methylbenzenesulfonate salt, a naphthalene-2-sulfonate salt, a naphthalene-1,5-disulfonate salt, a 2-phosphoglycerate salt, a 3-phosphoglycerate salt, a glucose-6-phosphate salt, and an amino acid salt.

Metal salts can arise from the addition of an inorganic base to a compound having a carboxyl group. The inorganic base consists of a metal cation paired with a basic counterion, such as, for example, hydroxide, carbonate, bicarbonate, or phosphate. The metal can be an alkali metal, alkaline earth metal, transition metal, or main group metal. Non-limiting examples of suitable metals include lithium, sodium, potassium, cesium, cerium, magnesium, manganese, iron, calcium, strontium, cobalt, titanium, aluminum, copper, cadmium, and zinc.

Non-limiting examples of suitable metal salts include a lithium salt, a sodium salt, a potassium salt, a cesium salt, a cerium salt, a magnesium salt, a manganese salt, an iron salt, a calcium salt, a strontium salt, a cobalt salt, a titanium salt, an aluminum salt, a copper salt, a cadmium salt, and a zinc salt.

Ammonium salts can arise from the addition of ammonia or an organic amine to a compound having a carboxyl group. Non-limiting examples of suitable organic amines include triethyl amine, diisopropyl amine, ethanol amine, diethanol amine, triethanol amine, morpholine, N-methylmorpholine, piperidine, N-methylpiperidine, N-ethylpiperidine, dibenzyl amine, piperazine, pyridine, pyrrazole, imidazole, pyrazine, pipyrazine, ethylenediamine, N,N'-dibenzylethylene diamine, procaine, chloroprocaine, choline, dicyclohexyl amine, and N-methylglucamine.

Non-limiting examples of suitable ammonium salts include is a triethylammonium salt, a diisopropylammonium salt, an ethanolammonium salt, a diethanolammonium salt, a triethanolammonium salt, a morpholinium salt, an N-methylmorpholinium salt, a piperidinium salt, an N-methylpiperidinium salt, an N-ethylpiperidinium salt, a dibenzylammonium salt, a piperazinium salt, a pyridinium salt, a pyrrazolium salt, an imidazolium salt, a pyrazinium salt, an ethylenediammonium salt, an N,N'-dibenzylethylenediammonium salt, a procaine salt, a chloroprocaine salt, a choline salt, a dicyclohexylammonium salt, and a N-methylglucamine salt.

### Methods for Treatment or Prevention

Disclosed herein are methods for treating cancer, comprising administering to a subject in need thereof an effective amount of a compound of the disclosure, wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma. In accordance with the present invention, the compound is Compound 85 or a pharmaceutically acceptable salt thereof. In some embodiments, the subject is human. In some embodiments, the subject is a *Canis sp.,* e.g., a dog, and in some embodiments, the subject is a *Felis sp.,* e.g., a cat.

Also disclosed herein are methods for preventing cancer, comprising administering to a subject in need thereof an effective amount of a compound of the disclosure, wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma. In accordance with the present invention, the compound is Compound 85 or a pharmaceutically acceptable salt thereof. In some embodiments, the subject is human. In some embodiments, the subject is a *Canis sp.,* e.g., a dog, and in some embodiments, the subject is a *Felis sp.,* e.g., a cat.

Also disclosed herein are methods for reducing risk of developing cancer, comprising administering to a subject in need thereof an effective amount of a compound of the disclosure, wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma. In accordance with the present invention, the compound is Compound 85 or a pharmaceutically acceptable salt thereof. In some embodiments, the subject is human. In some embodiments, the subject is a *Canis sp.,* e.g., a dog, and in some embodiments, the subject is a *Felis sp.,* e.g., a cat.

In some embodiments, colorectal cancer is colorectal adenocarcinoma, gastrointestinal carcinoid tumor, primary colorectal lymphoma, gastrointestinal stromal tumor, leiomyosarcoma of the colon or the rectum, or melanoma of the colon or the rectum.

In some embodiments, glioblastoma is primary glioblastoma or secondary glioblastoma.

In some embodiments, lung cancer is non-small cell lung cancer (NSCLC), small cell lung cancer (SCLC), lung carcinoid tumor, adenoid cystic carcinoma of the lungs, lymphoma of the lungs, or sarcoma of the lungs. In some embodiments, NSCLC is adenocarcinoma, squamous cell carcinoma, large cell carcinoma, adenosquamous carcinoma or sarcomatoid carcinoma.

In some embodiments, ovarian cancer is epithelial ovarian carcinoma, an ovarian germ cell tumor, an ovarian stromal tumor, primary peritoneal carcinoma (also known as extra-ovarian primary peritoneal carcinoma or serous surface papillary carcinoma), or fallopian tube cancer. In some embodiments, epithelial ovarian carcinoma is serous carcinoma, clear cell carcinoma, mucinous carcinoma, endometrioid carcinoma. In some embodiments, epithelial ovarian carcinoma is Grade 1 epithelial ovarian carcinoma or Grade 3 epithelial ovarian carcinoma. In some embodiments, the ovarian germ cell tumor is a teratoma, dysgerminoma, endodermal sinus tumor, or choriocarcinoma. In some embodiments, the ovarian stromal tumor is a granulosa cell tumor, granulosa-theca tumor, or Sertoli-Leydig cell tumor.

In some embodiments, pancreatic cancer is cancer of the exocrine pancreas or ampullary cancer. In some embodiments, cancer of the exocrine pancreas is pancreatic adenocarcinoma, an adenosquamous carcinoma, a squamous cell carcinoma, signet ring cell carcinoma, undifferentiated carcinoma, or undifferentiated carcinoma with giant cells. In some embodiments, pancreatic cancer is pancreatic adenocarcinoma.

In some embodiments, cervical cancer is squamous cell carcinoma, adenocarcinoma, or adenosquamous carcinoma (also known as mixed carcinoma). In some embodiments, cervical cancer is melanoma developed in the cervix, sarcoma developed in the cervix, or lymphoma developed in the cervix.

In some embodiments, prostate cancer is adenocarcinoma, small cell carcinoma, a neuroendocrine tumor (other than a small cell carcinoma), transitional cell carcinoma, or sarcoma. In some embodiments, prostate cancer is prostatic adenocarcinoma.

In some embodiments, breast cancer is invasive breast cancer, noninvasive breast cancer, inflammatory breast cancer, sarcoma of the breast, metaplastic carcinoma, adenocystic carcinoma, phyllodes tumor or angiosarcoma. In some embodiments, breast cancer is estrogen-positive, HER2-positive, or triple-negative.

In some embodiments, gastric cancer (also known as stomach cancer) is gastric adenocarcinoma, lymphoma, gastrointestinal stromal tumor (GIST), a carcinoid tumor, squamous cell carcinoma, small cell carcinoma, or leiomyosarcoma. In some embodiments, gastric cancer is gastric adenocarcinoma.

In some embodiments, head and neck cancer is head and neck squamous cell cancer. In some embodiments, head and neck cancer is cancer of the oral cavity, cancer of the hyarynx, cancer of the larynx, cancer of the paranasal sinuses, cancer of the nasal cavity, or cancer of the salivary glands. In some embodiments, head and neck cancer is metastatic squamous neck cancer with unknown (occult) primary.

In some embodiments, liver cancer is primary liver cancer or secondary liver cancer (also known as metastatic liver cancer). In some embodiments, primary liver cancer is hepatocellular carcinoma (HCC), intrahepatic cholangiocarcinoma (bile duct cancer), angiosarcoma of the liver, hemangiosarcoma of the liver, or hepatoblastoma.

In some embodiments, melanoma is skin melanoma. In some embodiments, skin melanoma is superficial spreading melanoma, nodular melanoma, lentigo maligna melanoma, or acral lentiginous melanoma. In some embodiments, melanoma is melanoma formed in the eyes, mouth, genitals, or anal area.

In some embodiments, lymphopoietic cancer is lymphosarcoma, reticulosarcoma, Hodgkin's disease, leukaemia, aleukaemia, or cancer of the lymphatic tissue.

In some embodiments, hematopoietic cancer is leukemia, lymphoma or myeloma. In some embodiments, hematopoietic cancer is non-Hodgkin's lymphoma (NHL), Burkitt's lymphoma (BL), multiple myeloma (MM), B chronic lymphocytic leukemia (B-CLL), B and T acute lymphocytic leukemia (ALL), T cell lymphoma (TCL), acute myeloid leukemia (AML), hairy cell leukemia (HCL), Hodgkin's Lymphoma (HL), or chronic myeloid leukemia (CML).

In some embodiments, soft tissue cancer is angiosarcoma, dermatofibrosarcoma protuberans, epithelioid sarcoma, gastrointestinal stromal tumor (GIST), Kaposi's sarcoma, leiomyosarcoma, liposarcoma, malignant peripheral nerve sheath tumors, myxofibrosarcoma, rhabdomyosarcoma, solitary fibrous tumor, synovial sarcoma, undifferentiated pleomorphic sarcoma, adult fibrosarcoma, alveolar soft-part sarcoma, clear cell sarcoma, desmoplastic small round cell tumor, fibromyxoid sarcoma, malignant mesenchymoma, or malignant peripheral nerve sheath tumor. In some embodiments, angiosarcoma is hemangiosarcoma or lymphangiosarcoma. In some embodiments, malignant peripheral nerve sheath tumor is neurofibrosarcoma, malignant schwannoma, or neurogenic sarcoma.

In some embodiments, the osteosarcoma (also known as osteogenic sarcoma) is osteoblastic osteosarcoma, chondroblastic osteosarcoma, fibroblastic osteosarcoma, small-cell vsarcoma, telangiectatic osteosarcoma, high-grade surface (juxtacortical high grade) osteosarcoma, pagetoid osteosarcoma, extraskeletal osteosarcoma, post-radiation osteosarcoma, periosteal (juxtacortical intermediate grade) osteosarcoma, parosteal (juxtacortical low grade) osteosarcoma, or intramedullary or intraosseous well differentiated osteosarcoma.

In some cases, the methods for treating osteosarcoma, preventing osteosarcoma, or reducing risk of developing osteosarcoma is in a *Canis sp.,* e.g., a dog. In some embodiments, the dog weighs about 10 pounds or greater, about 30 pounds or greater, about 50 pounds or greater, or about 110 pounds or greater. For example, the dog may be of the breed or mixture comprising the breed of Irish Wolfhound, Greyhound, Akbash, Saint Bernard, Leonberger, Rottweiler, Caucasian Ovtcharka, Scottish Deerhound, Curly-Coated Retriever, Anatolian Shepherd, Mastiff, Great Pyrenees, Tosa (Japanese Mastiff), Great Dane, Flat-Coated Retriever, Mastiff (Bull), Brazilian Fila, Irish Setter, Irish Water Spaniel, Mastiff (Tibetian), Golden Retriever, Labrador Retriever, Great Dane, Boxers, Doberman Pinscher, German Shepherd, Bernese Mountain dog, Samoyed, Borzoi, Weimaraner, Miniature Schnauzer, Cocker Sapniel, or Cairn Terrier. In some examples, the dog's age is in the range of about 4 to about 18 years old. In some examples, the dog's age is in the range of about 5 to about 14 years old.

In some embodiments, cancer is characterized by one or more mutations in the breast cancer 1 (*BRCA1*) or breast cancer 2 (*BRCA2*) genes. *BRCA1* and *BRCA2* are tumor suppressor genes, and encode proteins involved in DNA damage repair. Mutations that alter expression or activity of the BRCA1 or BRCA2 proteins may lead to the accumulation of genetic alterations in a cell, and can lead to cancer in a subject. Such mutations are referred to herein as "disease-associated mutations." In some embodiments, the cancer is characterized one or more mutations in *BRCA1* and *BRCA2* genes. In some embodiments, the cancer is characterized one or more mutations in *BRCA1* gene but has no mutations in *BRCA2* gene. In some embodiments, the cancer is characterized one or more mutations in *BRCA2* gene but has no mutations in *BRCA1* gene. In some embodiments, the cancer has no mutations in *BRCA1* or *BRCA2* genes.

In some embodiments, cancer is characterized by one or more disease-associated mutations in BRCA1 or BRCA2. In some embodiments, cancer is characterized by one or more disease-associated mutations in BRCA1 and BRCA2. In some embodiments, cancer is characterized by one or more disease-associated mutations in BRCA1 but harbors no disease-associated mutations in BRCA2. In some embodiments, cancer is characterized by one or more disease-associated mutations in BRCA2 but harbors no disease-associated mutations in BRCA1. In some embodiments, cancer is characterized by one or more disease-associated mutations in BRCA1 or BRCA2.

In some embodiments, cancer has one or more deficiencies in BRCA1 or BRCA2. In some embodiments, cancer has one or more deficiencies in BRCA1 and BRCA2. In some embodiments, cancer has one or more deficiencies in BRCA1 but harbors no deficiencies BRCA2. In some embodiments, cancer has one or more deficiencies in BRCA2 but harbors no deficiencies BRCA1. In some embodiments, cancer has no deficiencies in BRCA1 or BRCA2.

In some embodiments, cancer is BRCA-driven cancer. In some embodiments, cancer is BRCA1-driven cancer. In some embodiments, cancer is BRCA2-driven cancer. In some embodiments, cancer is BRCA1- and BRCA2-driven cancer. In some embodiments, cancer is neither BRCA1- nor BRCA2-driven cancer.

In some embodiments, the method further comprises administering to the subject another anti-cancer therapy. In some embodiments, the other anti-cancer therapy is administered before administering Compound 85 or a pharmaceutically acceptable salt thereof . In some embodiments, the other anti-cancer therapy is administered concurrently with the administration of Compound 85 or a pharmaceutically acceptable salt thereof. In some embodiments, the other anti-cancer therapy is administered subsequent to administering Compound 85 or a pharmaceutically acceptable salt thereof.

In some embodiments, the other anti-cancer therapy is neoadjuvant therapy. In some embodiments, the other anti-cancer therapy is an adjuvant therapy.

In some embodiments, the other anti-cancer therapy is chemotherapy, targeted therapy, hormone therapy, immunotherapy, T-cell therapy, or stem cell therapy.

In some embodiments, the chemotherapy is an alkylating agent, an antimetabolite, an anthracycline antibiotic, a non-anthracycline antibiotic, a topoisomerase inhibitor, a mitotic inhibitor, or a corticosteroid.

In some embodiments, the alkylating agent is cisplatin, cyclophosphamide, melphalan, oxaliplatin, temozolomide, or a nitrosourea.

In some embodiments, the antimetabolite is azacitidine, 5-fluorouracil, 6-mercaptopurine, gemcitabine, hydroxyurea or methotrexate.

In some embodiments, the anthracycline or non-anthracycline antibiotic is daunorubicin, doxorubicin, epirubicin, idarubicin, valrubicin or bleomycin.

In some embodiments, the topoisomerase inhibitor is irinotecan, topotecan, etoposide (VP-16), or mitoxantrone.

In some embodiments, the mitotic inhibitor is docetaxel, nab-paclitaxel, paclitaxel, vinblastine, vincristine or vinorelbine.

In some embodiments, the corticosteroid is prednisone, methylprednisolone or dexamethasone.

In some embodiments, the targeted therapy is herceptin, mabthera or avastin.

In some embodiments, the hormone therapy is tamoxifen or triptorelin.

In some embodiments, the immunotherapy is an anti-PD-L1 antibody, an anti-PD-1 antibody, or an anti-CTLA4 antibody.

In some embodiments, the anti-PD-L1 antibody is atezolizumab, durvalumab, avelumab, cosibelimab, envafolimab, BMS-936559 (BMS), MEDI-4736 (MedImmune) MPDL3280A (Genentech/Roche), or MIH1 (Affymetrix eBioscience (catalog No. 16.5983.82)).

In some embodiments, the anti-PD-1 antibody is nivolumab, pembrolizumab, pidilizumab, lambrolizumab, avelumab, tislelizumab, cemiplimab, cetrelimab, camrelizumab, spartalizumab, sintilimab, toripalimab, dostarlimab, retifanlimab, zimberelimab, AMP-224 (MedImmune), AMP-514 (MedImmune), BMS-936559 (BMS), MEDI4736 (Roche/Genentech), or Sym021 (Symphogen).

In some embodiments, the anti-CTLA4 antibody is ipilimumab or tremelimumab

In some embodiments, the immunotherapy is atezolizumab, durvalumab, avelumab, cosibelimab, envafolimab, nivolumab, pembrolizumab, pidilizumab, lambrolizumab, avelumab, tislelizumab, cemiplimab, cetrelimab, camrelizumab, spartalizumab, sintilimab, toripalimab, dostarlimab, retifanlimab, zimberelimab, ipilimumab or tremelimumab, alemtuzumab, trastuzumab, bevacizumab, cetuximab, ibritumomab tiuxetan, brentuximab vedotin, ado-trastuzumab emtansine, denileukin diftitox, or blinatumomab.

In some embodiments, the stem cell therapy is blood-forming stem cells.

Also disclosed herein are methods for treating an inflammatory disease or an autoimmune disease, comprising administering to a subject in need thereof an effective amount of a compound of the disclosure. In some examples, the subject is human. In some examples, the subject is a *Canis sp.,* e.g., a dog, and in some examples, the subject is *a Felis sp.,* e.g., a cat. In some examples, the compound is Compound 85 or a pharmaceutically acceptable salt thereof.

Also disclosed herein are methods for preventing an inflammatory disease or an autoimmune disease, comprising administering to a subject in need thereof an effective amount of a compound of the disclosure. In some examples, the subject is human. In some examples, the subject is a *Canis sp.,* e.g., a dog, and in some examples, the subject is *a Felis sp.,* e.g., a cat. In some examples, the compound is Compound 85 or a pharmaceutically acceptable salt thereof.

Also disclosed herein are methods for reducing risk of developing an inflammatory disease or an autoimmune disease, comprising administering to a subject in need thereof an effective amount of a compound of the disclosure. In some examples, the subject is human. In some examples, the subject is a *Canis sp.,* e.g., a dog, and in some examples, the subject is *a Felis sp.,* e.g., a cat. In some examples, the compound is Compound 85 or a pharmaceutically acceptable salt thereof.

In some examples, the inflammatory disease or the autoimmune disease is rheumatoid arthritis, juvenile idiopathic arthritis, colitis, atherosclerosis, cardiac myopathy, Crohn's disease, celiac disease, dermatitis herpetiformis, autoimmune blistering disease, epidermolysis bullosa, type 1 diabetes, asthma, lupus, dermatomyositis, alopecia areata, antiphospholipid antibody syndrome, autoimmune hepatitis, multiple sclerosis, Guillain-Barre syndrome, demyelinating polyneuropathy, psoriasis, Graves's disease, Hashimoto's thyroiditis, myasthenia gravis, vasculitis, hemolytic anemia, idiopathic thrombocytopenic purpura, inflammatory bowel disease, inflammatory myopathy, primary biliary cirrhosis, scleroderma, Sjögren's syndrome, systemic lupus erythematosus, or vitiligo.

In some examples, the inflammatory disease is rheumatoid arthritis, juvenile idiopathic arthritis, colitis, atherosclerosis, cardiac myopathy, Crohn's disease, celiac disease, dermatitis herpetiformis, autoimmune blistering disease, epidermolysis bullosa, asthma, lupus, dermatomyositis, alopecia areata, autoimmune hepatitis, multiple sclerosis, Guillain-Barre syndrome, demyelinating polyneuropathy, psoriasis, Graves's disease, Hashimoto's thyroiditis, myasthenia gravis, vasculitis, idiopathic thrombocytopenic purpura, inflammatory bowel disease, inflammatory myopathy, primary biliary cirrhosis, scleroderma, Sjögren's syndrome, systemic lupus erythematosus, or vitiligo.

In some examples, the autoimmune disease is rheumatoid arthritis, juvenile idiopathic arthritis, colitis, atherosclerosis, cardiac myopathy, Crohn's disease, celiac disease, dermatitis herpetiformis, autoimmune blistering disease, epidermolysis bullosa, type 1 diabetes, asthma, lupus, dermatomyositis, alopecia areata, antiphospholipid antibody syndrome, autoimmune hepatitis, multiple sclerosis, Guillain-Barre syndrome, demyelinating polyneuropathy, psoriasis, Graves's disease, Hashimoto's thyroiditis, myasthenia gravis, vasculitis, hemolytic anemia, idiopathic thrombocytopenic purpura, inflammatory bowel disease, inflammatory myopathy, primary biliary cirrhosis, scleroderma, Sjögren's syndrome, systemic lupus erythematosus, or vitiligo.

In some examples, the autoimmune blistering disease is autoimmune blistering skin disease.

In some examples of any of the methods disclosed herein, the compound is any of Compounds 1-35, 37-39, 42, 43, 44, 45, 46, 47-97, 98-123, 124a, 124b, 125-213, Va-Vz, Vaa-Vii, VIa-VIy, VIIa-VIId, XIIIa-XIIIz, XIVa, and XVa-XVm, or a pharmaceutically acceptable salt thereof. In some examples of any of the methods disclosed herein, the compound of the invention has the structure of Formula I, II, III, IV, V, VI, VII, XIII, XIV or XV, or is a pharmaceutically acceptable salt thereof. In some examples of any of the methods disclosed herein, the compound of the invention has the structure of Formula II, or a pharmaceutically acceptable salt thereof. In some examples of any of the methods disclosed herein, the compound of the invention is Compound 85, or a pharmaceutically acceptable salt thereof.

### Heat Shock Proteins

Heat shock proteins (Hsps) are classified according to their molecular weight and include the small Hsps, Hsp40, Hsp60, Hsp70, Hsp90 and Hsp100 families (Table 2).

**Table 2. Illustrative Families of Heat Shock Proteins**

| **Approximate molecular weight (kD)** | **Heat shock protein** |
|---|---|
| 10 kD | Hsp10 |
| 20-30 kD | HspB group, includes Hsp27 |
| 40 kD | Hsp40 |
| 60 kD | Hsp60 |
| 70 kD | HspA group, includes Hsp71, Hsp70, Hsp72, Grp78 (BiP), Hsx70 in primates |
| 70 kD | Ribosome-associated complex (RAC) |
| 90 kD | HspC group, includes Hsp90, Grp94 |
| 100 kD | HspH group, includes Hsp104, Hsp110 |

In some embodiments, a compound of the invention or a metabolite thereof binds to an Hsp. In some embodiments, a compound of the invention or a metabolite thereof covalently binds to an Hsp.

In some examples, the binding of a compound as disclosed herein or a metabolite thereof to an Hsp results in the treatment, prevention, or reduction of risk of developing cancer, wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma. In some embodiments, the compound is Compound 85 or a pharmaceutically acceptable salt thereof.

In some examples, the binding of a compound as disclosed herein or a metabolite thereof to an Hsp results in the treatment, prevention, or reduction of risk of developing an inflammatory disease or an autoimmune disease. In some embodiments, the compound is Compound 85 or a pharmaceutically acceptable salt thereof.

In some examples, the Hsp is a member of the Hsp10 family, Hsp40 family, Hsp60 family, Hsp70 family, Hsp90 family, or Hsp100 family.

### Compositions

The compound of the invention can be administered to a subject as a component of a composition that comprises a pharmaceutically acceptable carrier or vehicle. Non-limiting examples of suitable pharmaceutical carriers or vehicles include starch, glucose, lactose, sucrose, gelatin, malt, rice, flour, chalk, silica gel, magnesium carbonate, magnesium stearate, sodium stearate, glycerol monostearate, talc, sodium chloride, dried skim milk, glycerol, propylene, glycol, water, ethanol, buffered water, and phosphate buffered saline. These compositions can be administered as, for example, drops, solutions, suspensions, tablets, pills, capsules, powders, and sustained-release formulations. In some embodiments, the compositions comprise, for example, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate, and mineral oil. The compositions can additionally comprise lubricating agents, wetting agents, emulsifying and suspending agents, preserving agents, sweetening agents or flavoring agents.

The compositions can comprise an effective amount of a compound of the invention. The compositions can be formulated in a unit dosage form that comprises an effective amount of a compound of the invention. In some examples, the compositions comprise, for example, from about 1 ng to about 1,000 mg of a compound of the disclosure. In some examples, the compositions comprise from about 100 mg to about 1,000 mg of a compound of the disclosure. In some examples, the compositions comprise from about 100 mg to about 500 mg of a compound of the disclosure. In some examples, the compositions comprise from about 200 mg to about 300 mg of a compound of the disclosure.

The dosage of a compound of the disclosure can vary depending on the symptoms, age, and body weight of the subject, the nature and severity of cancer, inflammatory disease, and/or autoimmune disease, the route of administration, and the form of the composition. The compositions described herein can be administered in a single dose or in divided doses. In some examples, the dosage of a compound of the disclosure ranges from about 0.01 ng to about 10 g per kg body mass of the subject, from about 1 ng to about 0.1 g per kg, or from about 100 ng to about 10 mg per kg.

Administration can be, for example, topical, intraaural, intraocular, parenteral, intravenous, intra-arterial, subcutaneous, intramuscular, intracranial, intraorbital, intraventricular, intracapsular, intraspinal, intracisternal, intraperitoneal, intranasal, aerosol, suppository, or oral. Formulations for oral use include tablets containing a compound of the invention in a mixture with non-toxic pharmaceutically acceptable excipients. These excipients can be, for example, inert diluents or fillers (e.g., sucrose and sorbitol), lubricating agents, glidants, and antiadhesives (e.g., magnesium stearate, zinc stearate, stearic acid, silicas, hydrogenated vegetable oils, or talc). Formulations for ocular use can be in the form of eyedrops.

A compound of the disclosure can be provided in lyophilized form for reconstituting, for instance, in isotonic, aqueous, or saline buffers for parental, subcutaneous, intradermal, intramuscular, or intravenous administration. A composition can also be in the form of a liquid preparation useful for oral, intraaural, nasal, or sublingual administration, such as a suspension, syrup or elixir. A composition can also be in a form suitable for oral administration, such as a capsule, tablet, pill, and chewable solid formulation. A composition can also be prepared as a cream for dermal administration as a liquid, a viscous liquid, a paste, or a powder. A composition can also be prepared as a powder for pulmonary administration with or without an aerosolizing component.

The compositions can be in oral, intraaural, intranasal, sublingual, intraduodenal, subcutaneous, buccal, intracolonic, rectal, vaginal, mucosal, pulmonary, transdermal, intradermal, parenteral, intravenous, intramuscular and ocular dosage forms as well as being able to traverse the blood-brain barrier.

The compositions can be administered by various means known in the art. For example, the compositions can be administered orally, and can be formulated as tablets, capsules, granules, powders or syrups. Alternatively, compositions can be administered parenterally as injections (for example, intravenous, intramuscular or subcutaneous), drop infusion preparations or suppositories. For ophthalmic application compositions can be formulated as eye drops or eye ointments. Aural compositions can be formulated as ear drops, ointments, creams, liquids, gels, or salves for application to the ear, either internally or superficially. These formulations can be prepared by conventional means, and the compositions can be mixed with any conventional additive, such as an excipient, a binder, a disintegrating agent, a lubricant, a solubilizing agent, a suspension aid, an emulsifying agent, or a coating agent.

Compositions can include wetting agents, emulsifiers, and lubricants, coloring agents, release agents, coating agents, sweetening, flavoring and perfuming agents, preservatives and antioxidants.

Compositions can be suitable, for example, for oral, intraaural, intraocular, nasal, topical (including buccal and sublingual), rectal, vaginal, aerosol and/or parenteral administration. The compositions can be provided in a unit dosage form, and can be prepared by any methods known in the art.

Formulations suitable for oral administration may be in the form of capsules, cachets, pills, tablets, lozenges, powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia. Compositions can also be administered as a bolus, electuary, or paste.

Additional examples of pharmaceutically acceptable carriers or vehicles include: (1) fillers or extenders, such as starches, lactose, sucrose, glucose, mannitol, and/or silicic acid; (2) binders, such as carboxymethyl cellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as acetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; (10) coloring agents; and (11) buffering agents. Similar compositions can be employed as fillers in soft- or hard-filled gelatin capsules.

Liquid dosage forms for oral administration include pharmaceutically acceptable emulsions, microemulsions, gels, solutions, suspensions, syrups and elixirs. The liquid dosage form can contain inert diluents commonly used in the art, for example, water or other solvents, solubilizing agents and emulsifiers, such as ethyl alcohol, isopropyl alcohol, diethyl carbonate, ethyl acetate, benzyl alcohol, benzyl benzoate, propylene glycol, 1,3-butylene glycol, oils such as, cottonseed, groundnut, corn, germ, olive, castor and sesame oils, glycerol, tetrahydrofuryl alcohol, polyethylene glycols, fatty acid esters of sorbitan, and mixtures thereof.

Suspension dosage forms can contain suspending, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

The dosage forms for transdermal administration of a subject composition include drops, powders, sprays, ointments, pastes, creams, lotions, gels, solutions, and patches. The ointments, pastes, creams, and gels can contain excipients, such as animal and vegetable fats, oils, waxes, paraffin, starch, tragacanth, cellulose derivatives, polyethylene glycols, silicones, bentonite, silicic acid, talc and zinc oxide, or mixtures thereof.

Powders and sprays can contain excipients such as lactose, talc, silicic acid, aluminum hydroxide, calcium silicates, polyamide powder, or mixtures thereof. Sprays may additionally contain customary propellants, such as chlorofluorohydrocarbons and volatile unsubstituted hydrocarbons, such as butane and propane.

Compositions can be administered by aerosol of solid particles. A non-aqueous (e.g., fluorocarbon propellant) suspension could be used. Sonic nebulizers can be used because they minimize exposure to shear, which might cause degradation.

An aqueous aerosol can be made by formulating an aqueous solution or suspension of a compound of the invention with any conventional pharmaceutically acceptable carriers or vehicles such non-ionic surfactants (Tweens, Pluronics, or polyethylene glycol); proteins such as serum albumin; sorbitan esters; fatty acids; lecithin; amino acids; buffers; salts; sugars; or sugar alcohols.

Compositions suitable for parenteral administration comprise a compound of the invention and one or more pharmaceutically acceptable sterile isotonic aqueous or non-aqueous solutions, dispersions, suspensions, or emulsions, or sterile powders which can be reconstituted into sterile injectable solutions or dispersions just prior to use, which can contain antioxidants, buffers, bacteriostats, or solutes, which render the formulation isotonic with the blood of the subject, and suspending or thickening agents.

In some examples, the compound is Compound 85 or a pharmaceutically acceptable salt thereof.

Having described the invention with reference to certain embodiments, other embodiments will become apparent to one skilled in the art from consideration of the specification and claims.

### Methods for Making the Compounds

Methods for making the compounds discussed herein are disclosed in U.S. Patent Nos. 9,227,976, 9,079,909 and 8,765,762. Compound 85 can be synthesized according to Example 78 of U.S. Patent No. 9,079,909.

Compound 114 can be synthesized according to Vasilevsky, S.F. & Tretyakov, E.V. Cinnolines and pyrazolopyridazines. - Novel synthetic and mechanistic aspects of the Richter reaction. Liebigs Annalen 1995, 775-779 (1995).

Non-limiting examples of synthetic schema that are useful for synthesizing the compounds include the following.

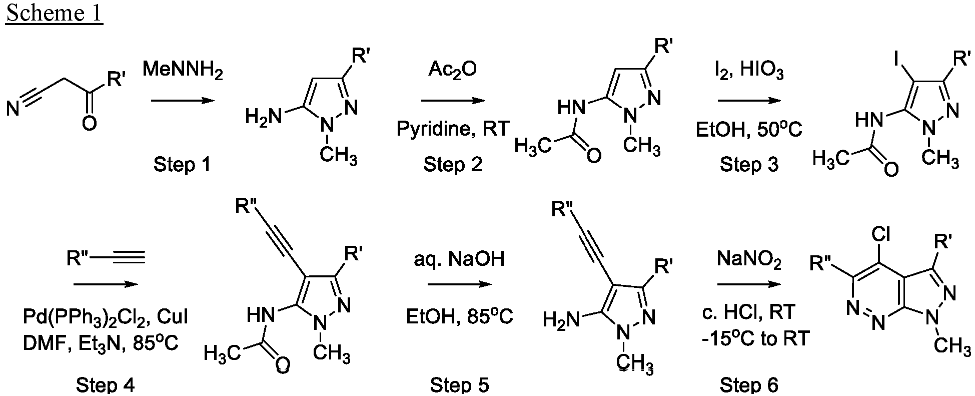

Scheme 1 generally describes the preparation of compounds having a 1-N-methyl group and where R' and R" are independently an unsubstituted or a substituted phenyl group. For example, a 2-cyanocarbonyl compound in which R' is unsubstituted or substituted phenyl is condensed with *N*-methylhydrazine to provide a 3-substituted-1-methyl-1*H*-pyrazol-5-amine. The 5-amino group is acylated, for example, with acetic anhydride in the presence of a base, such as pyridine, to provide a 5-amido compound. The 5-amido compound is iodinated, for example, with a mixture of iodine and iodic acid in a solvent such as ethanol (EtOH) to provide an *N*-(3-substituted-4-iodo-1-methyl-1*H*-pyrazol-5-yl)acetamide. A palladium-mediated cross-coupling, such as a Sonagashira cross-coupling, of the acetamide with an R"-substituted terminal alkyne, catalyzed, for example, by a palladium complex such as palladium (II) bistriphenylphosphine dichloride in the presence of copper (I) iodide in a solvent such as dimethylformamide (DMF) with a base such as triethylamine provides a disubstituted alkyne in which R" is unsubstituted or substituted phenyl. Saponification of the alkyne acetamide with a base such as sodium hydroxide in a solvent such as ethanol provides the primary amine. Diazotization of the primary amine with sodium nitrite in concentrated hydrochloric acid provides a diazo intermediate, which cyclizes to provide a compound having a 1-N-methyl group and where R' and R" are independently an unsubstituted or a substituted phenyl group.

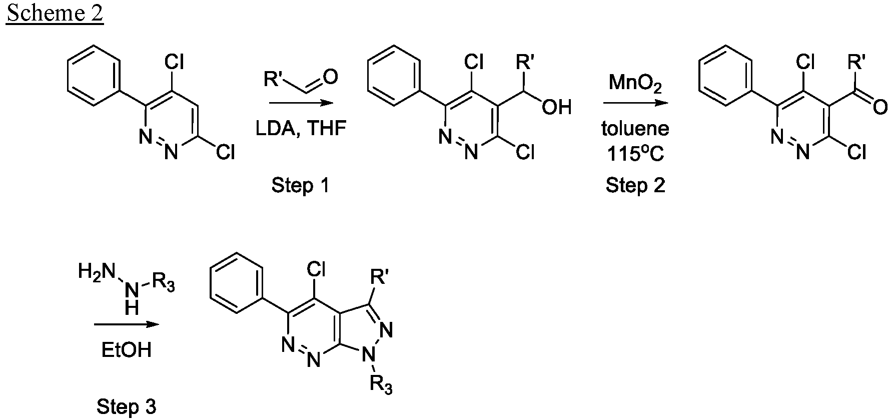

Scheme 2 generally describes the preparation of compounds having an R₃ group and in which R' is an unsubstituted or a substituted phenyl group. R' and R₃ can be the same or different. For example, 4,6-dichloro-3-phenylpyridazine is deprotonated with a base such as lithium diisopropyl amide (LDA) in a solvent such as tetrahydrofuran (THF), and the resultant 5-lithio species is condensed with an unsubstituted or a substituted benzaldehyde to provide a secondary alcohol. The alcohol is oxidized to a ketone with an oxidizing agent such as manganese dioxide in a solvent such as toluene. The ketone is condensed with an R₃-substituted hydrazine in a solvent such as ethanol to provide an intermediate hydrazone, which cyclizes to provide a compound having a 1-N- R₃ group, in which R₃ is defined as in Formulas II and III and in which R' is an unsubstituted or a substituted phenyl group.

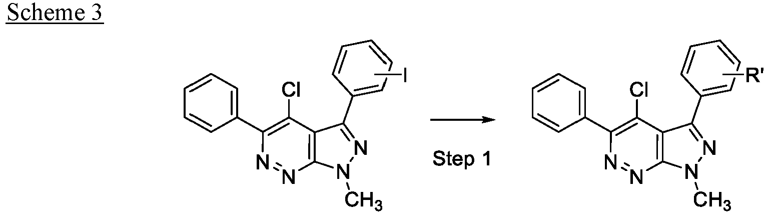

Scheme 3 generally describes the preparation of compounds having a 1-N-methyl group and where R' is a cyano group, an alkyne, an alkene or an aryl group. For example, 1-methyl-3-iodophenyl-4-chloro-5-phenyl-1H-pyrazolo[3,4-c]pyridazine is coupled with a suitable coupling partner, such as a cyanide salt, a terminal alkyne, an alkenyl halide, or an aryl halide, optionally in the presence of a suitable catalyst such as a palladium complex, optionally in the presence of a non-palladium transition metal salt such as a zinc or copper salt, optionally in the presence of an additive such as triphenylphosphine or an organic amine base, to provide a compound having a 1-N-methyl group and where R' is a cyano group, an alkyne, an alkene or an aryl group. The position of R', i.e., *ortho, meta* or *para,* in the product is the same as the position of the iodo group in the starting material.

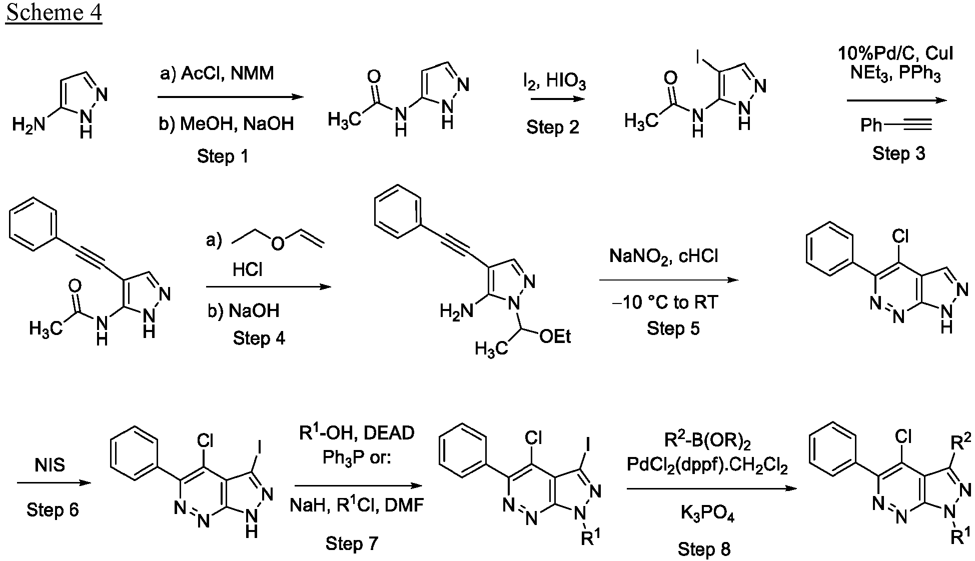

Scheme 4 generally describes the preparation of compounds of the invention. 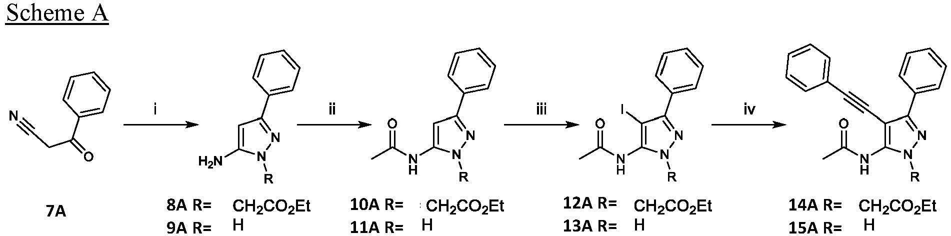 (i) ethyl hydrazinoacetate hydrochloride, EtOH, reflux, 2 h; (ii) Ac₂O, pyridine, 25°C, 16 h, or AcCl, N-methylmorpholine, CH₂Cl₂, 25°C, 3 h; (iii) I₂, HIO₃, EtOH, 50°C; (iv) Phenyl acetylene, Pd(PPh₃)₂Cl₂, CuI, Et₃N, DMF, 90°C.

Scheme A generally describes the preparation of Ethyl 2-[5-acetamido-3-phenyl-4-(2-phenylethynyl)-1*H*-pyrazol-1-yl]acetate and N-[3-Phenyl-4-(2-phenylethynyl)-1*H-*pyrazol-5-yl]acetamide from benzoylacetonitrile.

### Compound 8A of Scheme A: Ethyl 2-(5-amino-3-(phenyl)-1H-pyrazol-1-yl)acetate

A mixture of benzoylacetonitrile **(7A,** 44 g, 304 mmol) and ethyl hydrazinoacetate hydrochloride (47 g, 304 mmol) in ethanol (400 mL) is heated to reflux for 2 h. The reaction mixture is concentrated *in vacuo.* The crude reaction mixture is partitioned between CH₂Cl₂ (400 mL) and saturated NaHCO₃ (aq). The aqueous phase is extracted with CH₂Cl₂ and the organic phases are combined, dried over MgSO₄, filtered and evaporated to give compound **8A** as a solid (70 g, 95% yield). ¹H NMR (400 MHz, CDCl₃) δ (ppm) 7.73 (m, 2H), 7.38 (m, 2H), 7.26 (m, 1H), 5.96 (s, 1H), 4.86 (s, 2H), 4.25 (m, 2H), 3.7 (s, 2H), 1.28 (s, 3H).

### Compound 10A of Scheme A: Ethyl 2-(5-acetamido-3-phenyl-1H-pyrazol-1-yl)acetate

To a solution of ethyl 2-(5-amino-3-(phenyl)-1*H*-pyrazol-1-yl)acetate **(8A,** 41.7 g, 0.17 mol) in pyridine (200 mL) is added acetic anhydride (17.4 g, 0.17 mol) dropwise at 0°C under an atmosphere of nitrogen. The reaction mixture is stirred at room temperature (RT) for 16 h. The reaction mixture is concentrated *in vacuo.* The residue is diluted with CH₂Cl₂ and water. The layers are separated and the organic layer is washed with water and brine, dried (MgSO₄) and concentrated *in vacuo.* CH₂Cl₂ is added to the residue and the solid is collected by filtration, yielding compound **10A** as a solid (22 g, 45% yield). The mother liquors are concentrated *in vacuo* and washed with cold CH₂Cl₂ to give a second batch of compound **10A** (15 g, 31% yield). ¹H NMR (400 MHz, DMSO-d₆) δ (ppm) 10.13 (s, 1H), 7.81 (d, J = 7.6 Hz, 2H), 7.45 (t, J = 7.6 Hz, 2H), 7.40-7.32 (m, 1H), 6.80 (s, 1H), 5.07 (s, 2H), 4.22 (q, J = 7.1 Hz, 2H), 2.14 (s, 3H), 1.28 (t, J = 7.1 Hz, 3H).

### Compound 11A of Scheme A: N-(3-Phenyl-1H-pyrazol-5-yl)acetamide

To a solution of 3-phenyl-1H-pyrazol-5-amine (9, 18.6 g, 0.117 mol) and N-methylmorpholine (30.8 mL, 0.281 mol) in CH₂Cl₂ (250 mL) is added acetyl chloride (20 mL, 0.281 mol) dropwise at 0°C under an atmosphere of nitrogen. The reaction mixture is stirred at RT for 3 h. The reaction mixture is diluted with CH₂Cl₂ and water. The layers are separated and the organic layer is washed with water and brine, dried (phase separator cartridge) and concentrated *in vacuo.* Diethyl ether is added to the residue and the solid is collected by filtration, yielding compound **11A** as a solid (25.1 g, 88% yield). ¹H NMR (400 MHz, DMSO-d₆) δ (ppm) 12.79 (s, 1H), 10.40 (s, 1H), 7.71 (d, J = 7.5 Hz, 2H), 7.44 (dd, J = 7.6, 7.6 Hz, 2H), 7.34 (dd, J = 7.2, 7.2 Hz, 1H), 6.88 (s, 1H), 2.02 (s, 3H).

### Compound 12A of Scheme A: Ethyl 2-(5-acetamido-4-iodo-3-phenyl-1H-pyrazol-1-yl)acetate

A suspension of compound **10A** (37 g, 129 mmol), iodic acid (5.6 g, 32 mmol) and iodine (19.7 g, 77 mmol) in ethanol (400 mL) is heated at 50°C for 2 h and cooled to RT. The reaction mixture is concentrated *in vacuo* and the residue is eluted through a pad of silica gel with CH₂Cl₂ / diethyl ether (1:0 to 97:3). The residue is partitioned between CH₂Cl₂ and 2 M Na₂S₂O₃ solution (aq). The layers are separated and the organic washed is dried (MgSO₄), and concentrated *in vacuo* to give a residue that is partially purified by chromatography (silica gel, CH₂Cl₂/isohexane 1:1 to 1:0, then CH₂Cl₂/diethyl ether 9:1 to 8:2), then triturated with diethyl ether yielding compound **12A** as an off-white solid (43 g, 81% yield). ¹H NMR (400 MHz, CDCl₃) as a 3:1 mixture of rotamers δ (ppm) 7.81 (d, J = 7.6 Hz, 2H), 7.45-7.35 (m, 3H), 7.15 (br s, 0.75H), 6.85 (br s, 0.25H), 4.97 (s, 2H), 4.25 (q, J = 7.1 Hz, 2H), 2.24 (s, 2.25H), 2.04 (s, 0.75H), 1.30 (t, J = 7.1 Hz, 3H).

### Compound 13A of Scheme A: N-(4-Iodo-3-phenyl-1H-pyrazol-5-yl)acetamide

A suspension of compound **11A** (25.1 g, 0.103 mol), iodic acid (4.5 g, 0.026 mol) and iodine (15.7 g, 0.062 mol) in ethanol (250 mL) is heated at 50°C for 3 h and cooled to RT. The reaction mixture i concentrated *in vacuo* and partitioned between CH₂Cl₂ and 2 M Na₂S₂O₃ solution (aq). The layers are separated and the organic washed with brine, dried (phase separator cartridge), and concentrated *in vacuo* to give a mixture of compound **13A** and starting material **11A** (2.2:1, 30.3 g). The mixture is put in reaction again using iodic acid (1.6 g, 9.6 mmol) and iodine (9.7 g, 38 mmol) in ethanol (250 mL) under the same conditions, to give compound **13A** as a solid (31.9 g, 84% yield). ¹H NMR (400 MHz, CDCl₃) δ (ppm) 11.74 - 11.74 (m, 1H), 7.81 (d, J = 7.2 Hz, 2H), 7.59 (s, 1H), 7.49 - 7.38 (m, 3H), 2.31 (s, 3H).

### Compound 14A of Scheme A: Ethyl 2-[5-acetamido-3-phenyl-4-(2-phenylethynyl)-1H-pyrazol-1-yl]acetate

Nitrogen is bubbled through a mixture of compound **12A** (18.6 g, 45 mmol), phenyl acetylene (9.2 g, 90 mmol), copper iodide (860 mg, 4.5 mmol), triethylamine (200 mL) and DMF (75 mL) for 15 min. Bis(triphenylphosphine)palladium(II) dichloride (1.6 g, 2.25 mmol) is added and the reaction mixture is stirred at 90°C under nitrogen for 4.5 h. The reaction mixture is cooled to RT, diluted with ethyl acetate and water. The organic phase is washed with water and brine, dried (MgSO₄), filtered and concentrated *in vacuo.* The residue is partially purified by column chromatography (silica gel, CH₂Cl₂, then isohexane/ethyl acetate 1:1 followed by CH₂Cl₂/ethyl acetate 9:1 to 8:2), then triturated with diethyl ether yielding compound **14A** as a solid (13 g, 75% yield). ¹H NMR (400 MHz, DMSO-d₆) δ (ppm) 10.38 (s, 1H), 8.13-8.09 (m, 2H), 7.60-7.44 (m, 8H), 5.03 (s, 2H), 4.23 (q, J = 7.1 Hz, 2H), 2.17 (s, 3H), 1.28 (t, J = 7.1 Hz, 3H).

### Compound 15A of Scheme A: N-[3-Phenyl-4-(2-phenylethynyl)-1H-pyrazol-5-yl]acetamide

By a similar procedure to that described for the synthesis of compound **14A,** compound **15A** (12.5 g, 48% yield) is obtained from compound **13A** (31.87 g, 86 mmol). ¹H NMR (400 MHz, CDCl₃) δ (ppm) 11.57 - 11.57 (m, 1H), 8.11 (d, J = 7.4 Hz, 2H), 7.91 (s, 1H), 7.55 - 7.49 (m, 2H), 7.44 (dd, J = 7.5, 7.5 Hz, 2H), 7.37 (dd, J = 1.9, 5.0 Hz, 4H), 2.32 (s, 3H). 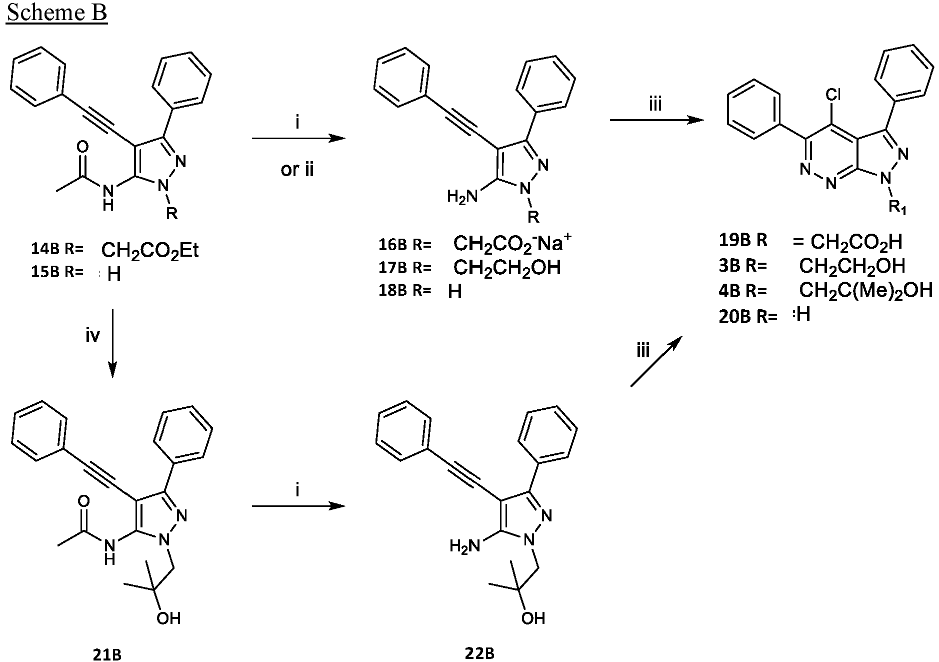 (i) NaOH, EtOH, 80°C; (ii) (a) NaBH₄, EtOH, 25°C; (b) NaOH, EtOH, 80°C; (iii) NaNO₂, c. HCl, -10°C to 25°C; (iv) MeMgCl, THF, 0°C to 25°C.

Scheme B generally describes the preparation of compound **3B** and compound **4B.**

### Compound 16B of Scheme B: Sodium 2-[5-amino-3-phenyl-4-(2-phenylethynyl)-1H-pyrazol-1-yl]acetate

A mixture of compound **14B** (13 g, 34 mmol), ethanol (150 mL) and 25% NaOH solution (aq) (150 mL) is stirred and heated to 80°C for 8 h and cooled to RT. Upon cooling, a precipitate is formed. The precipitate is filtered and washed with a cooled mixture of ethyl acetate/water (1:1). The solid is further triturated with diethyl ether, filtered and dried (MgSO₄), yielding compound **16B** as a solid (9.8 g, 85% yield). ¹H NMR (400 MHz, DMSO-d₆) δ (ppm) 8.06 (d, J = 7.8 Hz, 2H), 7.56 (d, J = 7.6 Hz, 2H), 7.50-7.39 (m, 4H), 7.39-7.31 (m, 2H), 4.31 (s, 2H).

### Compound 17B of Scheme B: 2-[5-Amino-3-phenyl-4-(2-phenylethynyl-1H-)pyrazol-1-yl]ethan1-ol

To a suspension of compound **14B** (22.4 g, 58 mmol) in ethanol (290 mL) is added sodium borohydride (11 g, 289 mmol) and the reaction mixture is stirred at RT for 16 h. The reaction mixture is partially concentrated to a final volume of 250 mL. A 25% NaOH solution (aq) (250 mL) is added and the reaction mixture is stirred at 80°C for 4 h. The reaction mixture is cooled down to room temperature and phases are separated. The aqueous phase is extracted with ethyl acetate three times and the organic phases combined, dried (MgSO₄), filtered and concentrated *in vacuo.* The residue is triturated from diethyl ether (20 mL) and the product is filtered and dried *in vacuo* yielding compound **17B** as an off-white solid (9.96 g, 57% yield). The mother liquor is concentrated *in vacuo* and purified by column chromatography (silica gel, gradient 0 to 100% ethyl acetate/isohexane) yielding a further crop (1.79 g, 10% yield). ¹H NMR (400 MHz, CDCl₃) δ (ppm) 7.78-7.74 (m, 4H), 7.55-7.48 (m, 6H), 4.98 (t, J = 4.8 Hz, 2H), 4.29 (m, 2H), 3.03 (t, J = 6.4 Hz, 1H).

### Compound 18B of Scheme B: 3-Phenyl-4-(2-phenylethynyl)-1H-pyrazol-5-amine

By a similar procedure to that described for the synthesis of compound **16B,** compound **18B** (5.4 g, 50% yield) is obtained from compound **15B** (12.5 g, 41 mmol). ¹H NMR (400 MHz, CDCl₃) δ (ppm) 7.87 (d, J = 7.2 Hz, 2H), 7.51 - 7.43 (m, 4H), 7.42 - 7.32 (m, 4H), 4.09 (s, 2H).

### Compound 19B of Scheme B: 2-(4-Chloro-3,5-diphenyl-1H-pyrazolo[3,4-c]pyridazin-1-yl)acetic acid

Sodium nitrite (1.86 g, 26.9 mmol) is added portion-wise to concentrated HCl (30 mL) at 0°C and stirred for 15 min and then compound **16B** (3 g, 8.85 mmol) is added as a solid to the reaction mixture, portion-wise. The suspension is then stirred at RT for 16 h. The reaction mixture is diluted with CH₂Cl₂ and washed with water and brine. The organic layer is dried (MgSO₄) and concentrated *in vacuo.* The residue is purified by column chromatography (silica gel, diethyl ether/ CH₂Cl₂ 1:9) yielding compound **19B** as a solid (1.7 g, 53% yield). ¹H NMR (400 MHz, CDCl₃) δ (ppm) 7.80-7.72 (m, 4H), 7.56-7.47 (m, 6H), 5.64 (s, 2H), 2.10 (s, 1H).

### Compound 3B of Scheme B: 2-(4-Chloro-3,5-diphenyl-1H-pyrazolo[3,4-c]pyridazin-1-yl)ethan-1-ol

Sodium nitrite (4.58 g, 66.3 mmol) is added portion-wise to concentrated HCl (220 mL) at -10°C and stirred for 10 min. Compound **17B** (6.7 g, 22.1 mmol) is added as a solid. The reaction mixture is allowed to warm up, is sonicated for 5 min then stirred at RT for 2 h. The reaction mixture is diluted with CH₂Cl₂ and water and the aqueous phase is extracted with CH₂Cl₂. The organic phases are combined, dried (MgSO₄), filtered and concentrated *in vacuo.* The residue is partially purified by column chromatography (silica gel, gradient 0 to 100% ethyl acetate/isohexane). The resulting residue is triturated from diethyl ether then from ethyl acetate, yielding compound **3B** as a solid (900 mg, 12% yield). ¹H NMR (400 MHz, CDCl₃) δ (ppm) 7.79-7.75 (m, 4H), 7.55-7.46 (m, 6H), 4.98 (m, 2H), 4.32-4.25 (m, 2H), 3.04 (t, J = 6.4 Hz, 1H). ¹³C NMR (100 MHz, CDCl₃) δ (ppm) 153.09, 151.98, 143.65, 134.48, 130.11, 129.35, 129.33, 129.06, 128.29, 128.17, 127.39, 127.33, 113.70, 60.64, 50.63. LC-MS (analytical method 1: HPLC (Phenomenex Luna 5 µm C18, 100 x 4.6 mm) with gradient of 5-95% acetonitrile in water (with 0.1% formic acid in each mobile phase)) Rt 4.14 min; m/z 351 [M+H] 99.04% purity.

### Compound 21B of Scheme B: N-(1-(2-Hydroxy-2-methylpropyl)-3-phenyl-4-(phenylethynyl)-1H-pyrazol-5-yl)acetamide

To a solution of compound **14B** (1.0 g, 2.58 mmol) in THF (26 mL) is added methyl magnesium chloride (3 M solution in THF, 3 mL, 9 mmol) at 0°C. The solution obtained is stirred at RT for 3.5 h then successively diluted with ethyl acetate and quenched by addition of 1 M HCl (aq). The aqueous phase is extracted with ethyl acetate, and the combined organic layers are dried (MgSO₄) and concentrated *in vacuo.* The resultant residue is purified using chromatography (silica gel, gradient 0 to 75% ethyl acetate/isohexane) yielding compound **21B** as a solid (529 mg, 55% yield). ¹H NMR (400 MHz, CDCl₃) as a 1.5:1 mixture of compound **21B** δ (ppm) 8.11 (dd, J = 7.5, 12.3 Hz, 2H), 7.51 - 7.40 (m, 4H), 7.37 - 7.31 (m, 4H), 4.15 - 4.07 (m, 2H), 2.24 - 2.23 (m, 3H), 1.28 (s, 6H) and N-[2-acetonyl-5-phenyl-4-(2-phenylethynyl)pyrazol-3-yl]acetamide δ (ppm) 8.11 (dd, J = 7.5, 12.3 Hz, 2H), 7.51 - 7.40 (m, 4H), 7.37 - 7.31 (m, 4H), 4.95 (s, 2H), 2.24 - 2.23 (m, 3H), 1.28 (s, 6H).

### Compound 22B of Scheme B: 1-(5-Amino-3-phenyl-4-(phenylethynyl)-1H-pyrazol-1-yl)-2-methylpropan-2-ol

Compound **22B** (310 mg, yield 59%) is synthesized from compound **21B** (597 mg, 1.6 mmol) following similar procedures outlined in the synthesis of compound **16B.** ¹H NMR (400 MHz, CDCl₃) δ (ppm) 8.11 - 8.08 (m, 2H), 7.50 - 7.47 (m, 2H), 7.41 (dd, J = 7.5, 7.5 Hz, 2H), 7.37 - 7.29 (m, 4H), 4.48 (s, 2H), 4.01 (s, 2H), 2.70 (s, 1H), 1.32 - 1.31 (m, 6H).

### Compound 4B of Scheme B: 1-(4-Chloro-3,5-diphenyl-1H-pyrazolo[3,4-c]pyridazin-1-yl)-2-methylpropan-2-ol

To cooled (cooling bath -15°C) concentrated HCl (9 mL) is added sodium nitrite in one portion (121 mg, 1.75 mmol) and the suspension is left to stir for 10 min after which compound **22B** (290 mg, 0.88 mmol) is added. After 5 min, the cooling bath is removed and the reaction mixture is stirred at RT for 3 h. The reaction is cooled again (0°C) and CH₂Cl₂ is added followed by water. The aqueous phase is extracted with CH₂Cl₂ and the organic phases are combined, dried (MgSO₄), filtered and concentrated *in vacuo.* Crude material is purified by column chromatography (silica gel, gradient 0 to 50% ethyl acetate/isohexane) yielding compound **4B** as orange oil (56 mg). The material obtained is further purified by preparative HPLC, yielding compound **4B** as a solid (34 mg, 10% yield). ¹H NMR (400 MHz, CDCl₃) δ (ppm) 7.81-7.75 (m, 4H), 7.55-7.50 (m, 6H), 4.85 (s, 2H), 3.50 (s, 1H), 1.36 (s, 6H). ¹³C NMR (100 MHz, CDCl₃) δ (ppm) 154.36, 152.91, 144.60, 135.42, 130.96, 130.29, 130.28, 129.93, 129.23, 129.07, 128.29, 128.24, 114.16, 71.52, 58.60, 27.26. LCMS (analytical method 1: HPLC (Phenomenex Luna 5 µm C18, 100 x 4.6 mm) with gradient of 5-95% acetonitrile in water (with 0.1% formic acid in each mobile phase)) Rt 4.49 min; m/z 379 [M+H] 99.71% purity.

### Compound 20B of Scheme B: 4-Chloro-3,5-diphenyl-1H-pyrazolo[3,4-c]pyridazine

Sodium nitrite (2.88 g, 42 mmol) is added portion-wise to concentrated HCl (314 mL) at -15°C and stirred for 15 min. Compound **18B** (5.4 g, 21 mmol) is added as a solid, followed by the addition of CH₂Cl₂ (10 mL). The reaction mixture is allowed to warm up and stirred at RT for 1 h. The reaction mixture is diluted with CH₂Cl₂ (44 mL) and NaCl (2.7 g) is added. The reaction mixture is heated to 50°C for 1 d. The layers are separated and the organic layer is washed with water, dried (phase separator cartridge) and concentrated *in vacuo.* The residue is purified by column chromatography (silica gel, isohexane/ethyl acetate 4:1, then CH₂Cl₂/ethyl acetate 1:0 to 4:1) yielding compound **20B** as a solid (3.0 g, 47% yield). ¹H NMR (400 MHz, DMSO-d₆) δ (ppm) 15.08 (s, 1H), 7.81 - 7.73 (m, 4H), 7.58 - 7.51 (m, 6H). 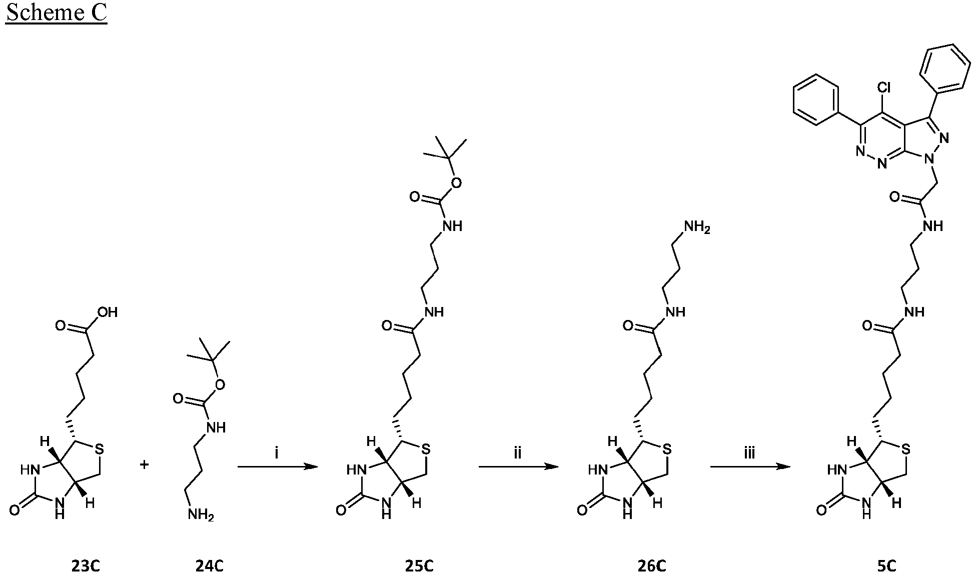 (i) HATU, DIPEA, DMF, 25°C; (ii) TFA, DCM, 25°C; (iii) compound **19B,** HATU, DIPEA, DMF, 25°C.

Scheme C generally describes the preparation of compound **5C.**

### Compound 25C of Scheme C: tert-Butyl (3-(5-((3aS,4S,6aR)-2-oxohexahydro-1H-thieno[3,4-d]imidazol-4-yl)pentanamido)propyl)carbamate

To a solution of biotin **(23C,** 350 mg, 1.43 mmol) in DMF (7.2 mL) are added tert-butyl N-(3-aminopropyl)carbamate **(24C,** 250 mg, 1.43 mmol), DIPEA (0.375 mL, 2.15 mmol) and HATU (816 mg, 2.15 mmol). The reaction mixture is stirred at RT for 20 h, and then diluted with ethyl acetate and 4% LiCl aqueous solution. The aqueous phase is extracted with ethyl acetate twice, and the combined organic layers are dried (MgSO₄) and concentrated *in vacuo.* The resultant residue is purified using chromatography (silica gel, gradient 0 to 12% 7 M NH₃ in MeOH/ CH₂Cl₂) yielding compound 25C as a solid (180 mg, 31% yield). ¹H NMR (400 MHz, DMSO-d₆) δ (ppm) 7.75 (t, J = 5.2 Hz, 1H), 6.77 (s, 1H), 6.43 (s, 1H), 6.37 (s, 1H), 4.38-4.34 (m, 1H), 4.21-4.16 (m, 1H), 3.23 (d, J = 5.3 Hz, 1H), 3.16 (dq, J = 6.2, 4.3 Hz, 1H), 3.07 (dd, J = 6.8, 12.9 Hz, 2H), 2.96 (dd, J = 6.6, 13.0 Hz, 2H), 2.88 (dd, J = 5.2, 12.4 Hz, 1H), 2.11 (t, J = 7.5 Hz, 2H), 1.73-1.62 (m, 1H), 1.61-1.48 (m, 5H), 1.43 (s, 9H), 1.40-1.29 (m, 2H).

### Compound 26C of Scheme C: N-(3-Aminopropyl)-5-((3aS,4S,6aR)-2-oxohexahydro-1H-thieno[3,4-d]imidazol-4-yl)pentanamide

To a solution of compound **25C** (166 mg, 0.415 mmol) in CH₂Cl₂ (1 mL) is added TFA (1 mL). The reaction mixture is stirred at RT for 2 h, then concentrated *in vacuo.* The resultant residue is dissolved in CH₂Cl₂ (2 mL) and Biotage MP-carbonate resin (550 mg, 1.66 mmol) is added. The reaction mixture is stirred at RT for 30 min. Beads are filtered off and washed with CH₂Cl₂/MeOH (1:1, 2 mL) and the filtrate is concentrated *in vacuo* to afford compound **26C** as a colorless oil (124 mg, 100% yield), which is used as such in the next step.

### Compound 5C of Scheme C: N-(3-(2-(4-Chloro-3,5-diphenyl-1H-pyrazolo[3,4-c]pyridazin-1-yl)acetamido)propyl)-5-((3aS,4S,6aR)-2-oxohexahydro-1H-thieno[3,4-d]imidazol-4-yl)pentanamide

To a solution of compound **26C** (124 mg, 0.415 mmol) in DMF (2 mL) are added compound **19B** (151 mg, 0.415 mmol), DIPEA (0.11 mL, 0.62 mmol) and HATU (236 mg, 0.62 mmol). The reaction mixture is stirred at RT for 1.5 h and then diluted with CH₂Cl₂ and 4% LiCl aqueous solution. The aqueous phase is extracted with CH₂Cl₂ twice, and the combined organic layers are dried (phase separation) and concentrated *in vacuo.* The resultant residue is first purified by prep HPLC yielding 70 mg, which is further purified by silica gel chromatography, yielding the desired compound **5C** as a solid (44 mg, 16% yield). ¹H NMR (400 MHz, DMSO-d₆) δ (ppm) 8.39 (dd, J = 5.7, 5.7 Hz, 1H), 7.87 - 7.78 (m, 5H), 7.64 - 7.57 (m, 6H), 6.45 (s, 1H), 6.39 (s, 1H), 5.51 (s, 2H), 4.33 (dd, J = 5.3, 7.6 Hz, 1H), 4.18 - 4.13 (m, 1H), 3.22 - 3.08 (m, 5H), 2.85 (dd, J = 5.2, 12.5 Hz, 1H), 2.61 (d, J = 12.4 Hz, 1H), 2.10 (dd, J = 7.5, 7.5 Hz, 2H), 1.66 - 1.48 (m, 6H), 1.39 - 1.28 (m, 2H). ¹³C NMR (100 MHz, CDCl₃) δ (ppm) 172.51, 166.25, 163.16, 154.31, 152.75, 144.06, 135.95, 131.35, 130.58, 130.52, 129.58, 129.44, 129.42, 128.75, 128.70, 114.18, 61.47, 59.64, 55.85, 50.91, 37.21, 36.66, 35.66, 29.64, 28.66, 28.48, 25.75. LCMS (analytical method 1: HPLC (Phenomenex Luna 5 µm C18, 100 x 4.6 mm) with gradient of 5-95% acetonitrile in water (with 0.1% formic acid in each mobile phase)) Rt 3.52 min; m/z 647 [M+H] 98.38% purity. 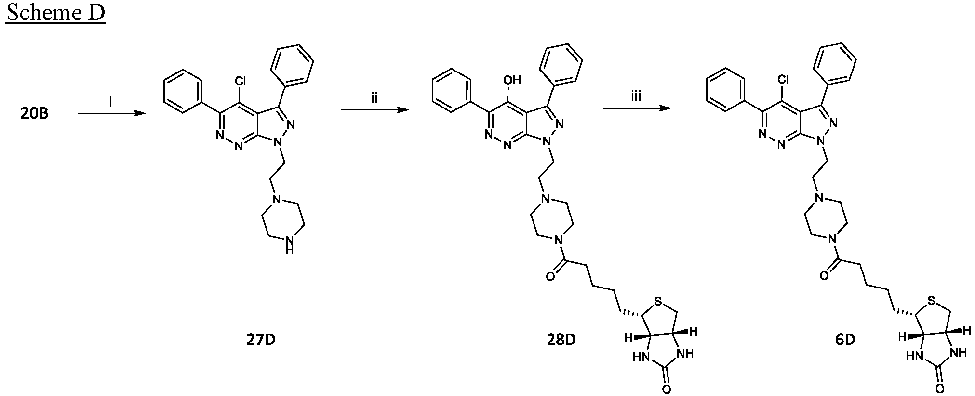 (i) (a) 1-*tert*-Butoxycarbonyl-4-(2-hydroxyethyl)piperazine, DEAD, PPh₃, 1,4-dioxane, 120°C, 1 h; (*b*) HCl in 1,4-dioxane, 25°C; (ii) (*a*) HATU, DIPEA, DMF, 25°C; (*b*) NaOH, 40°C, 1 h; (iii) POCl₃, 25°C.

Scheme D generally describes the preparation of compound **6D.**

### Compound 27D of Scheme D: 4-Chloro-3,5-diphenyl-1-(2-(piperazin-1-yl)ethyl)-1H-pyrazolo[3,4-c]pyridazine

To a mixture of compound **20B** (345 mg, 1.13 mmol), 1-tert-butoxycarbonyl-4-(2-hydroxyethyl)piperazine (520 mg, 2.26 mmol) and triphenylphosphine (888 mg, 2.26 mmol) in 1,4-dioxane (8.4 mL) is slowly added to diethyl azodicarboxylate (0.355 mL, 2.26 mmol) at RT. The reaction mixture is then heated using microwave irradiation to 120°C for 1 h. The reaction mixture is cooled down to RT and 4 M HCl in 1,4-dioxane (4 mL) is added. The reaction mixture is stirred at RT for 4 h, diluted with CH₂Cl₂ (10 mL) and the solution is loaded onto a Biotage SCX-2 cartridge (20 g), eluted with methanol, then 7 M NH₃ in methanol. Fractions were concentrated *in vacuo* to give compound **27D** in a 1:1 ratio with 2-hydroxyethyl)piperazine (950 mg, 100% yield) and is used as such in the next step.

### Compound 28D of Scheme D: (3aS,4S,6aR)-4-(5-(4-(2-(4-Hydroxy-3,5-diphenyl-1H-pyrazolo[3,4-c]pyridazin-1-yl)ethyl)piperazin-1-yl)-5-oxopentyl)tetrahydro-1H-thieno[3,4-d]imidazol-2(3H)-one

To a solution of compound **27D** (950 mg crude, 1.13 mmol) in DMF (5.7 mL), DIPEA (0.59 mL, 3.39 mmol), biotin (678 mg, 2.78 mmol) and HATU (1.29 g, 3.39 mmol) are added. The reaction mixture is stirred at RT for 24 h and then diluted with DMSO (5 mL). NaOH (2 M, 5 mL) is added and the reaction mixture is heated to 40°C for 1 h, then stirred at RT for 2 days. The reaction mixture is purified by preparative HPLC to yield compound **28D** (100 mg, 14% yield). ¹H NMR

### Compound 6D of Scheme D: (3aS,4S,6aR)-4-(5-(4-(2-(4-Chloro-3,5-diphenyl-1H-pyrazolo[3,4-c]pyridazin-1-yl)ethyl)piperazin-1-yl)-5-oxopentyl)tetrahydro-1H-thieno[3,4-d]imidazol-2(3H)-one

A solution of compound **28D** (97 mg, 0.155 mmol) in phosphorous oxychloride (6 mL) is stirred at RT for 2 days. The reaction mixture is diluted with CH₂Cl₂ and 2 M Na₂CO₃ solution. The aqueous phase is extracted twice with CH₂Cl₂. Combined organic layers are dried (MgSO₄) and concentrated *in vacuo.* The resultant residue is purified using chromatography (silica gel, gradient 0 to 12% 7 M NH₃ in MeOH/ CH₂Cl₂) yielding compound **6D** as a solid (42 mg, 42% yield). ¹H NMR (400 MHz, DMSO-d₆) δ (ppm) 7.86-7.78 (m, 4H), 7.66-7.56 (m, 6H), 6.47 (s, 1H), 6.39 (s, 1H), 5.01-4.94 (m, 2H), 4.38-4.32 (m, 1H), 4.20-4.15 (m, 1H), 3.17-3.10 (m, 1H), 3.08-3.01 (m, 2H), 2.87 (dd, J = 12.4, 5.1 Hz, 1H), 2.63 (d, J = 12.4 Hz, 1H), 2.58 (m, 1H), 2.57-2.54 (m, 2H), 2.51 (m, 2H), 2.36-2.28 (m, 2H), 1.71-1.61 (m, 1H), 1.56-1.45 (m, 3H), 1.38 (m, 2H), 1.27-1.17 (m, 1H). ¹³C NMR (100 MHz, CDCl₃) δ (ppm) 170.51, 162.70, 153.69, 151.95, 143.18, 135.52, 131.06, 130.15, 130.11, 129.03, 128.94, 128.84, 128.26, 128.20, 113.42, 61.04, 59.76, 59.19, 56.16, 55.48, 52.71, 52.28, 45.27, 32.05, 28.29, 28.11, 24.85. LCMS (analytical method 2: HPLC (Hichrom ACE 3 C18-AR mixed mode column 100 x 4.6 mm) with gradient of 2-100% acetonitrile in water (with 0.1% formic acid in each mobile phase)) Rt 9.77 min; m/z 645 [M+H] 93.27% purity.

### EXAMPLES

### Example 1. In Vitro Anti-Cancer Assay of Compound 85

Compound **85** was assessed in 10 human tumor cell lines (see Table 3) for anti-cancer activity *in vitro* using a CellTiter-Blue^{®} based 2D monolayer assay.

CellTiter-Blue^{®} Cell Viability Assay: Tumor cells were grown at 37 °C in a humidified atmosphere with 5% CO₂ in RPMI medium, supplemented with 10% (v/v) fetal calf serum and 50 µg/ml gentamicin (140 µl/well). Cultures were incubated at 37 °C and 5% CO₂ in a humidified atmosphere. After 24 hours, 10 µl of Compound **85** was added, and left on the cells for another 72 hours (incubation period). Compound **85** was tested at 5 concentrations (0.003 µM, 0.03 µM, 0.3 µM, 3 µM, and 30 µM) and serially diluted in DMSO, mixed with cell culture medium, and added to the assay plates by using a Tecan Freedom EVO 200 robotic platform. The DMSO concentration was kept constant at 0.3% v/v across the assay plate. Every 96 well plate included six DMSO-treated control wells and Compound **85**-treated wells in duplicate at 5 concentrations. Viability of cells was quantified using the CellTiter-Blue^{®} cell viability assay (Promega G8081). After incubation of cells, the CellTiter-Blue^{®} reagent was brought to ambient temperature. Next, 20 µl of CellTiter-Blue^{®} reagent were added to each well. After incubation for up to 4 hours, fluorescence (FU) was measured using the EnSpire^{®} multimode plate reader (Perkin Elmer) (excitation λ = 600 nm).

Data Evaluation: Sigmoidal concentration-response curves were fitted to the data points (test-versus-control; T/C values) obtained for each tumor model using 4 parameter non-linear curve fit (Charles River DRS Datawarehouse Software). Drug effects are expressed in terms of the percentage of the fluorescence signal, obtained by comparison of the mean signal in the treated wells with the mean signal of the untreated controls (T/C-value [%]) (Table 4). IC₅₀ and IC₇₀ values were reported as relative and absolute IC₅₀ and IC₇₀ values (Table 5). The absolute IC₅₀ and IC₇₀ values reflect the concentration of Compound **85** that achieves T/C = 50 % and T/C = 30 %, respectively. The relative IC₅₀ value is the concentration of Compound **85** that gives a response half way between the top and bottom plateau of the sigmoidal concentration-response curve (inflection point of the curve).

Compound **85** showed concentration-dependent inhibition of the tumor cell growth in all cell lines of Table 3, with a geometric mean absolute IC₅₀ value of 0.95 µM. The curves were very steep and Compound **85** showed fairly similar concentration-response curve in all the cell lines tested, with IC₅₀ values ranging from 0.215 µM (CXF LS 174T) to 1.847 µM (PAXF 1657).

**Table 3. Anti-Cancer Activity of Compound 85**

| **Cell Line** | | **Tumor type** | **BRCA mutation** |
|---|---|---|---|
| PRXF | PC-3 | Prostate | none |
| PRXF | DU-145 | Prostate | BRCA2 mutation |
| PAXF | HUP-T3 | Pancreas | BRCA2 mutation |
| PAXF | 1657 | Pancreas | none |
| OVXF | A2780 | Ovarian | none |
| OVXF | 899 | Ovarian | BRCA1 mutation |
| CXF | LS 174T | Colon | BRCA1 mutation |
| CXF | HCC-2998 | Colon | none |
| CNXF | U-87 MG | Glioblastoma | none |
| CNXF | 498 | Glioblastoma | none |

**Table 4. Test-versus-Control Values for Compound 85 at Different Concentrations**

| **Cell Line** | | **Test/Control (%) at Drug Concentration [µM]** | | | | |
|---|---|---|---|---|---|---|
| | | **0.003** | **0.03** | **0.3** | **3** | **30** |
| PRXF | PC-3 | 104 | 103 | 103 | 2 | 1 |
| PRXF | DU-145 | 100 | 110 | 102 | 11 | 6 |
| PAXF | HUP-T3 | 100 | 101 | 79 | 16 | 0 |
| PAXF | 1657 | 100 | 100 | 100 | 3 | 1 |
| OVXF | A2780 | 101 | 94 | 101 | 1 | 0 |
| OVXF | 899 | 108 | 106 | 86 | 2 | 1 |
| CXF | LS 174T | 103 | 101 | 35 | 4 | 0 |
| CXF | HCC-2998 | 97 | 100 | 90 | 13 | 1 |
| CNXF | U-87 MG | 99 | 102 | 102 | 1 | 4 |
| CNXF | 498 | 106 | 107 | 113 | 2 | 1 |

**Table 5. Anti-Cancer Activity of Compound 85**

| **Cell Line** | | **Top (%)** | **Bot. (%)** | **Unit** | **Rel. IC₅₀** | **Rel. IC₇₀** | **Abs. IC₅₀** | **Abs. IC₇₀** |
|---|---|---|---|---|---|---|---|---|
| PRXF | PC-3 | 103 | 1 | µM | 1.076 | 1.326 | 1.096 | 1.349 |
| PRXF | DU-145 | 105 | 6 | µM | 1.005 | 1.376 | 1.088 | 1.528 |
| PAXF | HUP-T3 | 101 | 0 | µM | 0.785 | 1.505 | 0.794 | 1.518 |
| PAXF | 1657 | 100 | 1 | µM | 1.841 | 2.043 | 1.847 | 2.055 |
| OVXF | A2780 | 99 | 0 | µM | 1.699 | 1.872 | 1.694 | 1.868 |
| OVXF | 899 | 107 | 1 | µM | 0.522 | 0.739 | 0.553 | 0.775 |
| CXF | LS 174T | 103 | 1 | µM | 0.204 | 0.328 | 0.215 | 0.346 |
| CXF | HCC-2998 | 99 | 1 | µM | 1.031 | 1.630 | 1.022 | 1.628 |
| CNXF | U-87 MG | 101 | 3 | µM | 0.935 | 0.987 | 0.940 | 0.994 |
| CNXF | 498 | 109 | 1 | µM | 1.706 | 1.868 | 1.740 | 1.901 |
| **Geometric mean IC value [µM]:** | | | | | **0.926** | **1.226** | **0.948** | **1.258** |

### Example 2. Cell Viability Assay of Compound 85

The CellTiter-Blue^{®} Cell Viability Assay was used to assess the viability of cells from various cancer cell lines, in the presence or absence of Compound **85** (at different concentrations). This assay provides a homogeneous, fluorometric method for estimating the number of viable cells present in multi-well plates. Viable cells retain the ability to reduce resazurin to resorufin, which is highly fluorescent. Nonviable cells rapidly lose metabolic capacity, do not reduce the indicator dye (resazurin) due to the lack of energy in the form of ATP, and thus do not generate a fluorescent signal. The fluorescence produced is proportional to the number of viable cells. There is a linear relationship between cell number and fluorescence.

Cancer cells (see Table 6) were harvested from exponential phase cultures, counted and plated in 96 well flat-bottom microtiter plates, at a cell density 6,000 - 20,000 cells/well, depending on the cell line's growth rate. The culture medium was supplemented with 10% (v/v) foetal calf serum and 50 µg/mL gentamicin (140 µL/well). Cultures were incubated at 37 °C and 5% CO₂ in a humidified atmosphere.

Compound **85** was serially diluted in DMSO, and five Compound **85** concentrations in half-log increments were used: 0.03 µM, 0.09 µM, 0.30 µM, 1.0 µM, and 3.0 µM. After 24 h, 10 µL of Compound **85** (dissolved in DMSO) or control (DMSO) medium were added, in duplicates, and left on the cells for another 72 h. Compound **85** solutions were added to the assay plates using a Tecan Freedom EVO 200 robotic platform. The DMSO concentration was kept constant at 0.3% v/v across the assay plate.

After incubation of up to 72 hours, fluorescence (FU) was measured using the EnSpire^{®} multimode plate reader (Perkin Elmer) (excitation λ= 570 nm, emission λ= 600 nm).

Cancer cell lines used: Compound **85'**s anti-cancer effect was tested in 20 human cancer-cell lines representing nine cancer types (Table 6).

**Table 6. Cancer Types and Cell Lines**

| Tumour type | Cell line 1 | Cell line 2 |
|---|---|---|
| glioblastoma | CNXF-498 | CNXF-478 MG |
| pancreas | PAXF hup-T3 (BRCA2) | PAXF-1657 |
| prostate | PRXF-du-145b (BRCA2) | PRXF-Pc-3-CDX |
| ovarian | OVXf-A2780 | OVXF-899 (BRCA1) |
| colon | CXF-HCC-2998 | CXF-Ls174T (BRCA1, MS) |
| breast | MAXFHER JIMT-1 | MAXFHER BT474 *(*BRCA2, SC*) |
| | MAXFTN MDA-MB-453 | MAXFTN-401 **(*BRCA1, n FS*) |
| melanoma | MEXF 1737 | MEXF 1341 (*BRCA1*/*BRCA2, both has FS*) |
| soft tissue sarcoma | SXFS 1301 | SXFS-Hs 729 (*BRCA1*/*BRCA2, both has MS*) |
| osteosarcoma | SXFO 678L | SXFO Saos-2 |

| | | |
|---|---|---|
| BRCA = **BR**east **CA**ncer gene, MS= missense mutation, SC= stop codon mutation, FS= frameshift mutation | | |

Results. Results are presented as:
(i) Percentage of viable cells (T/C %),
(ii) Relative and absolute IC₅₀ and IC₇₀ values, and
(iii) Concentration-effect curves/plots for individual cancers.

### Percentage of viable cells

The percentage of viable cells in the presence of Compound **85** (Test condition, T), at different concentrations, are presented in Table 7 and expressed as a percentage of the number of viable cells in the presence of DMSO without Compound **85** (Control condition, C).

Where T/C percentage is about 100, there is no effect of Compound **85** on the cancer-cell growth. A value below 100% indicates an anti-cancer effect, while a value above 100% indicates enhancement of cancer growth. Small changes in the % are not considered significant. The data show that at concentration of 0.95 µM, Compound **85** has significant anti-cancer activity in 20 human cancer-cell lines representing nine cancer types.

**Table 7. T/C % at Different Concentration of Compound 85**

| ***Cancer*** | | | **Concentration of Compound 85 (*µM*)** | | | | |
|---|---|---|---|---|---|---|---|
| ***Type*** | ***Cell line*** | | **0.03** | **0.095** | **0.3** | **0.95** | **3.0** |
| Colon | CXF | HCC-2998 | 98 | 99 | 98 | 40 | 22 |
| colon | CXF | LS 174T | 96 | 96 | 72 | 5 | 3 |
| Breast | MAXFHER | BT-474 | 101 | 106 | 99 | 46 | 17 |
| Breast | MAXFHER | JIMT-1 | 97 | 97 | 96 | 30 | 14 |
| Breast | MAXFTN | 401 | 99 | 99 | 87 | 3 | 1 |
| Breast | MAXFTN | MDA-MB-453 | 101 | 97 | 101 | 3 | 1 |
| Melanoma | MEXF | 1341 | 97 | 91 | 82 | 0 | 0 |
| Melanoma | MEXF | 1737 | 99 | 97 | 101 | 27 | 16 |
| Ovarian | OVXF | 899 | 98 | 94 | 65 | 1 | 0 |
| Ovarian | OVXF | A2780 | 102 | 96 | 87 | 0 | 0 |
| Pancreatic | PAXF | 1657 | 97 | 94 | 97 | 66 | 1 |
| Pancreatic | PAXF | HUP-T3 | 95 | 102 | 88 | 21 | 16 |
| Glioblastoma | CNXF | U-87 MG | 99 | 102 | 102 | 1 | 4 |
| Glioblastoma | CNXF | 498 | 106 | 107 | 113 | 2 | 1 |
| Prostate | PRXF | PC-3 | 104 | 103 | 103 | 2 | 1 |
| Prostate | PRXF | DU-145 | 100 | 110 | 102 | 11 | 6 |
| Soft tissues Sarcoma | SXFS | 1301 | 105 | 106 | 102 | 3 | 2 |
| Soft tissues Sarcoma | SXFS- | Hs 729 | 99 | 99 | 99 | 13 | 7 |
| Osteosarcoma | SXFO | 678 | 101 | 97 | 91 | 2 | 1 |
| Osteosarcoma | SXFO | Saos-2 | 97 | 93 | 99 | 1 | 0 |

### Relative and absolute IC₅₀ and IC₇₀ values

The **relative IC₅₀** is determined as the Compound **85** concentration giving a response halfway between the maximum cell viability signal (i.e., a 100% cell viability, no Compound **85** response) and the minimal viability signal (i.e. maximum cell viability inhibition achieved by Compound **85),** in a sigmoidal concentration-effect curve.

The **absolute IC₅₀** is determined as the concentration that is associated with a T/C ratio of 50%.

Table 8, below, shows values for absolute and relative IC₅₀ and IC₇₀ in different human cancer cell lines. The geometric means for relative and absolute IC₅₀ values were 0.583 µM and 0.596 µM, respectively. It is accepted that for most anti-cancer therapeutics a mean IC₅₀ of <1.0 µM indicates a cancer cell line that is sensitive to the therapy. The Geometric means for relative and absolute IC₇₀ values were 0.675 and 0.712 µM, respectively.

**Table 8. Absolute and relative IC₅₀ and IC₇₀ values (µM) of Compound 85 in different human cancer cell lines**

| **Cancer Type** | **Cell line** | | **Relative IC₅₀** | **Relative IC₇₀** | **Absolute IC₅₀** | **Absolute IC₇₀** |
|---|---|---|---|---|---|---|
| Colon | CXF | HCC-2998 | 0.765 | 0.891 | 0.844 | 1.125 |
| Colon | CXF | LS 174T | 0.384 | 0.467 | 0.383 | 0.474 |
| Breast | MAXFHER | BT-474 | 0.766 | 1.01 | 0.886 | 1.316 |
| Breast | MAXFHER | JIMT-1 | 0.702 | 0.844 | 0.742 | 0.957 |
| Breast | MAXFTN | 401 | 0.429 | 0.5 | 0.429 | 0.503 |
| Breast | MAXFTN | MDA-MB-453 | 0.717 | 0.763 | 0.719 | 0.765 |
| Melanoma | MEXF | 1341 | 0.399 | 0.451 | 0.391 | 0.445 |
| Melanoma | MEXF | 1737 | 0.799 | 0.864 | 0.826 | 0.925 |
| Ovarian | OVXF | 899 | 0.352 | 0.423 | 0.346 | 0.418 |
| Ovarian | OVXF | A2780 | 0.406 | 0.463 | 0.405 | 0.463 |
| Pancreatic | PAXF | 1657 | 1.049 | 1.169 | 1.041 | 1.166 |
| Pancreatic | PAXF | HUP-T3 | 0.487 | 0.602 | 0.532 | 0.723 |
| Osteosarcoma | SXFO | 678 | 0.458 | 0.531 | 0.458 | 0.532 |
| Osteosarcoma | SXFO | Saos-2 | 0.712 | 0.75 | 0.709 | 0.747 |
| Soft tissue sarcoma | SXFS | 1301 | 0.49 | 0.558 | 0.501 | 0.57 |
| Soft tissue sarcoma | SXFS | Hs 729 | 0.724 | 0.789 | 0.733 | 0.808 |
| Prostate | PRXF | PC-3 | 1.07585 | 1.32629 | 1.09606 | 1.34929 |
| Prostate | PRXF | DU-145 | 1.0054 | 1.37576 | 1.0883 | 1.52792 |
| Glioblastoma | CNXF | U-87 MG | 0.93519 | 0.98695 | 0.93966 | 0.99376 |
| Glioblastoma | CNXF | 498 | 1.70611 | 1.86824 | 1.74025 | 1.90105 |

Compound **85** showed concentration dependent anti-cancer activity in all of the 20 human cancer cell lines tested, with an absolute geometric mean IC₅₀ value of 0.6 µM. Individual absolute IC₅₀ values were in the range of 0.35 µM and 1.0 µM, indicating that Compound 85 has significant anti-cancer activity.

## Claims

1. A compound having the structure: 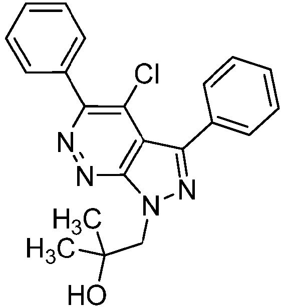
or a pharmaceutically acceptable salt thereof, for use in a method of treating cancer, preventing cancer, or reducing risk of developing cancer,
wherein the cancer is colorectal cancer, glioblastoma, lung cancer, ovarian cancer, pancreatic cancer, cervical cancer, prostate cancer, breast cancer, gastric cancer, head and neck cancer, liver cancer, melanoma, lymphopoietic cancer, hematopoietic cancer, soft tissue sarcoma, or osteosarcoma.

2. The compound for use of claim 1, wherein the cancer has one or more disease-associated mutations in BRCA1 or BRCA2.

3. The compound for use of claim 1, wherein the cancer has one or more disease-associated mutations in BRCA1 but harbors no disease-associated mutations in BRCA2.

4. The compound for use of any one of claims 1-3, wherein the method further comprises administering an anti-cancer therapy to the subject.

5. The compound for use of claim 4, wherein the anti-cancer therapy is neoadjuvant therapy or an adjuvant therapy.

6. The compound for use of claim 4 or 5, wherein the anti-cancer therapy is chemotherapy, targeted therapy, hormone therapy, immunotherapy, T-cell therapy, or stem cell therapy.

7. The compound for use of claim 6, wherein the chemotherapy is an alkylating agent, an antimetabolite, an anthracycline antibiotic, a non-anthracycline antibiotic, a topoisomerase inhibitor, a mitotic inhibitor, or a corticosteroid.

8. The compound for use of claim 6, wherein the immunotherapy is an anti-PD-L1 antibody, an anti-PD-1 antibody, or an anti-CTLA4 antibody.

## Patentansprüche

1. Verbindung mit der Struktur: 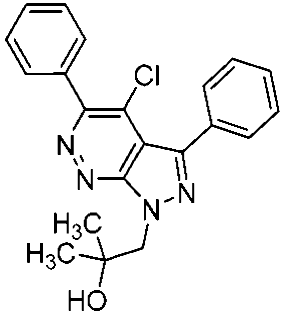
oder pharmazeutisch unbedenkliches Salz davon zur Verwendung in einem Verfahren zur Behandlung von Krebs, zur Prävention von Krebs oder zur Verringerung des Risikos, Krebs zu entwickeln,
wobei es sich bei dem Krebs um Kolorektalkrebs, Glioblastom, Lungenkrebs, Eierstockkrebs, Bauchspeicheldrüsenkrebs, Gebärmutterhalskrebs, Prostatakrebs, Brustkrebs, Magenkrebs, Kopf- und Halskrebs, Leberkrebs, Melanom, lymphopoetischen Krebs, hämatopoetischen Krebs, Weichteilsarkom oder Osteosarkom handelt.

2. Verbindung zur Verwendung nach Anspruch 1, wobei der Krebs eine oder mehrere krankheitsassoziierte Mutationen in BRCA1 oder BRCA2 aufweist.

3. Verbindung zur Verwendung nach Anspruch 1, wobei der Krebs eine oder mehrere krankheitsassoziierte Mutationen in HRCA1 aufweist, aber keine krankheitsassoziierten Mutationen in BRCA2 aufweist.

4. Verbindung zur Verwendung nach einem der Ansprüche 1-3, wobei das Verfahren weiterhin das Verabreichen einer Antikrebstherapie an das Subjekt umfasst.

5. Verbindung zur Verwendung nach Anspruch 4, wobei es sich bei der Antikrebstherapie um eine neoadjuvante Therapie oder eine adjuvante Therapie handelt.

6. Verbindung zur Verwendung nach Anspruch 4 oder 5, wobei es sich bei der Antikrebstherapie um Chemotherapie, gezielte Therapie, Hormontherapie, Immuntherapie, T-Zell-Therapie oder Stammzelltherapie handelt.

7. Verbindung zur Verwendung nach Anspruch 6, wobei es sich bei der Chemotherapie um ein Alkylierungsmittel, einen Antimetaboliten, ein Anthracyclinantibiotikum, ein Nicht-Anthracyclin-Antibiotikum, einen Topoisomerasehemmer, einen Mitosehemmer oder ein Corticosteroid handelt.

8. Verbindung zur Verwendung nach Anspruch 6, wobei es sich bei der Immuntherapie um einen Anti-PD-L1-Antikörper, einen Anti-PD-1-Antikörper oder einen Anti-CTLA4-Antikörper handelt.

## Revendications

1. Composé ayant la structure : 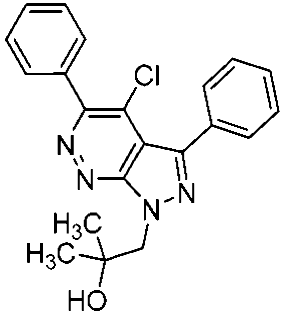
ou sel pharmaceutiquement acceptable de celui-ci, pour utilisation dans un procédé de traitement d'un cancer, de prévention d'un cancer ou de réduction du risque de développer un cancer,
dans lequel le cancer est un cancer colorectal, un glioblastome, un cancer du poumon, un cancer de l'ovaire, un cancer du pancréas, un cancer du col de l'utérus, un cancer de la prostate, un cancer du sein, un cancer gastrique, un cancer de la tête et du cou, un cancer du foie, un mélanome, un cancer lymphopoïétique, un cancer hématopoïétique, un sarcome des tissus mous ou un ostéosarcome.

2. Composé pour utilisation selon la revendication 1, dans lequel le cancer a une ou plusieurs mutations associées à une maladie dans BRCA1 ou BRCA2.

3. Composé pour utilisation selon la revendication 1, dans lequel le cancer a une ou plusieurs mutations associées à une maladie dans BRCA1 mais ne présente aucune mutation associée à une maladie dans BRCA2.

4. Composé pour utilisation selon l'une quelconque des revendications 1 à 3, dans lequel le procédé comprend en outre l'administration d'une thérapie anticancéreuse au sujet.

5. Composé pour utilisation selon la revendication 4, dans lequel la thérapie anticancéreuse est une thérapie néoadjuvante ou une thérapie adjuvante.

6. Composé pour utilisation selon la revendication 4 ou 5, dans lequel la thérapie anticancéreuse est une chimiothérapie, une thérapie ciblée, une hormonothérapie, une immunothérapie, une thérapie par cellules T ou une thérapie par cellules souches.

7. Composé pour utilisation selon la revendication 6, dans lequel la chimiothérapie est un agent alkylant, un antimétabolite, un antibiotique de type anthracycline, un antibiotique de type non-anthracycline, un inhibiteur de topoisomérase, un inhibiteur mitotique ou un corticostéroïde.

8. Composé pour utilisation selon la revendication 6, dans lequel l'immunothérapie est un anticorps anti-PD-L1, un anticorps anti-PD-1 ou un anticorps anti-CTLA4.
